# EUROPEAN PATENT SPECIFICATION

(11) **EP 2 441 493 B1**
(45) Date of publication and mention of the grant of the patent: **07.05.2014**
(21) Application number: 11192874.3
(22) Date of filing: 14.03.2007
(51) Int. Cl.: A61P 31/06, A61K 39/04

(54) **Methods for producing an immune response to tuberculosis**
Verfahren zur Erzeugung einer Immunantwort auf Tuberculosis
Méthode pour produire une reponse contre la tuberculose

(30) Priority: 14.03.2006 US 782364 P
(43) Date of publication of application: 18.04.2012
(62) Divisional of application: 10171127.3
(73) Proprietor: Oregon Health and Science University, Portland, OR 97201 (US); THE GOVERNMENT OF THE UNITED STATES OF AMERICA D.B.A THE DEPARTMENT OF VETERANS AFFAIRS, Washington, DC 20420 (US)
(72) Inventor: Lewinsohn, David, Portland, OR Oregon 97201 (US); Lewinsohn, Deborah, Portland, OR Oregon 97201 (US)
(74) Representative: Gowshall, Jonathan Vallance

(56) References cited:
- LEWINSOHN DEBORAH A ET AL: "Human dendritic cells presenting adenovirally expressed antigen elicit Mycobacterium tuberculosis-specific CD8+ T cells", AMERICAN JOURNAL OF RESPIRATORY AND CRITICAL CARE MEDICINE, vol. 166, no. 6, 15 September 2002 (2002-09-15), pages 843-848, XP002443626, ISSN: 1073-449X
- LEWINSOHN DAVID M ET AL: "Characterization of human CD8+ T cells reactive with Mycobacterium tuberculosis-infected antigen-presenting cells", JOURNAL OF EXPERIMENTAL MEDICINE, vol. 187, no. 10, 18 May 1998 (1998-05-18) , pages 1633-1640, XP002443627, ISSN: 0022-1007
- GROTZKE J E ET AL: "Role of CD8<+> T lymphocytes in control of Mycobacterium tuberculosis infection", MICROBES AND INFECTION, ELSEVIER, PARIS, FR, vol. 7, no. 4, April 2005 (2005-04), pages 776-788, XP004904672, ISSN: 1286-4579
- GARNIER T ET AL: "THE COMPLETE GENOME SEQUENCE OF MYCOBACTERIUM BOVIS", PROCEEDINGS OF THE NATIONAL ACADEMY OF SCIENCES OF USA, NATIONAL ACADEMY OF SCIENCE, WASHINGTON, DC; US, vol. 100, no. 13, 24 June 2003 (2003-06-24), pages 7877-7882, XP001205229, ISSN: 0027-8424, DOI: 10.1073/PNAS.1130426100 & DATABASE UniProt 5 July 2004 (2004-07-05), XP002667263, Database accession no. Q79FS8 & DATABASE UniProt [Online] 1 October 2003 (2003-10-01), Database accession no. Q7U0P5

## Description

### FIELD

This application relates to the field of immunology, more specifically to methods for the production of an immune response to tuberculosis antigens.

### BACKGROUND

Mycobacteria are a genus of aerobic intracellular bacterial organisms that, upon infection of a host, survive within endosomal compartments of monocytes and macrophages. Human mycobacterial diseases include tuberculosis (caused *by M tuberculosis*), leprosy (caused by *M leprae),* Bairnsdale ulcers (caused *by M*. *ulcerans*), and various infections caused by *M marinum, M. kansasii, M scrofulaceum, M szulgai, M xenopi, M fortuitum, M chelonei, M haemophilum and M. intracellulare* (see Wolinsky, E., Chapter 37 in Microbiology: Including Immunology and Molecular Genetics, 3rd Ed., Harper & Row, Philadelphia, 1980).

One third of the world's population harbors *M. tuberculosis* and is at risk for developing tuberculosis (TB). In immunocompromised patients, tuberculosis is increasing at a nearly logarithmic rate, and multidrug resistant strains are appearing. In addition, Mycobacterial strains which were previously considered to be nonpathogenic strains (e.g., *M. avium*) have now become major killers of immunosuppressed AIDS patients. Moreover, current Mycobacterial vaccines are either inadequate (such as the BCG vaccine for *M. tuberculosis*) or unavailable (such as for *M. leprae*) (Kaufmann, S., Microbiol. Sci 4:324-328, 1987; U.S. Congress, Office of Technology Assessment, The Continuing Challenge of Tuberculosis, pp. 62-67, OTA-H-574, U.S. Government Printing Office, Washington, D.C., 1993).

*Mycobacterium tuberculosis* (Mtb)-specific CD4⁺ and CD8⁺ T cells are critical for the effective control of Mtb infection. In the mouse model, passive transfer of CD4⁺ T cells to sublethally irradiated animals renders them less susceptible to Mtb infection (Orme, J Immunol. 140:3589-3593, 1988). Mice in which the gene(s) for CD4 (CD4^{-/-}) or for MHC Class II molecules are disrupted as well as wild-type mice depleted of CD4⁺ T cells demonstrate increased susceptibility to Mtb infection (Flory et al., J Leukoc Biol. 51:225-229, 1992). In humans, human immunodeficiency virus-infected individuals are exquisitely susceptible to developing tuberculosis (TB) after exposure to Mtb, supporting an essential role for CD4⁺ T cells (Hirsch et al., J Infect Dis. 180:2069-2073, 1999). CD8⁺ T cells are also important for effective T cell immunity (see Lazarevic and Flynn, Am J Respir Crit Care Med. 166:1116-1121, 2002). In humans, Mtb-specific CD8⁺ T cells have been identified in Mtb-infected individuals and include CD8⁺ T cells that are both classically HLA-Ia restricted (see, for example, Lewinsohn et al., J Immunol. 165:925-930, 2000) and nonclassically restricted by the HLA-Ib molecules HLA-E (Lewinsohn et al., J Exp Med 187:1633-1640, 1998). However, there are no vaccines or therapeutic strategies that effectively induce an immune response, such as a CD8 response, to Mtb. Thus, a need remains for agents that can produce an immune response to Mtb that can be used for treatment and/or protection from an Mtb infection.

### SUMMARY

The invention is defined by the claims.
Methods for producing an immune response to *Mycobacterium tuberculosis* (Mtb) are disclosed herein. Methods for treating an Mtb infection, or preventing an Mtb infection in a subject, are also disclosed herein. The Mtb infection can be latent or active.

In several embodiments, the methods include administering to the subject a therapeutically effective amount of a polypeptide for use according to the invention, or a polynucleotide encoding the polypeptide, wherein the polypeptide comprises the amino acid sequence SEQ ID NO: 7. In additional embodiments, the methods include administering to the subject a therapeutically effective amount of a polypeptide for use according to the invention comprising at least nine to twenty consecutive amino acids of the amino acid sequence , SEQ ID NO: 7, wherein the nine to twenty consecutive amino acids specifically bind major histocompatibility complex (MHC) class I. In several examples, the immune response is a protective immune response. In additional embodiments, methods are disclosed for preventing an infection with Mtb, or treating an infection with Mtb.

Isolated polypeptides are described herein that include nine to twenty consecutive amino acids of the amino acid sequence SEQ ID NO: 7, wherein the nine to twenty consecutive amino acids specifically bind major histocompatibility complex (MHC) class I, wherein the isolated polypeptide does not include the full length amino acid sequence SEQ ID NO: 7. Nucleic acids encoding these polypeptides, vectors including these nucleic acids, host cells including these nucleic acids, and immunogenic compositions including these polypeptides, nucleic acids and/or host cells are also disclosed. Pharmaceutical compositions including a therapeutically effective amount of these polypeptides, nucleic acids, and/or host cells are also described.

The foregoing and other features and advantages will become more apparent from the following detailed description of several embodiments, which proceeds with reference to the accompanying figures.

### BRIEF DESCRIPTION OF THE FIGURES

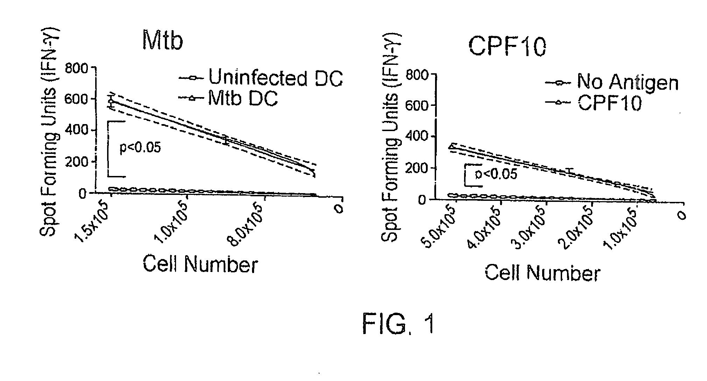

**Figure 1** is two graphs showing the determination of human effector cell frequencies *ex vivo* using the IFN-γ ELISPOT assay. Magnetic bead-purified CD8⁺ T cells were cultured with DC (20,000/well) either infected with Mtb (H37Rv, MOI = 50) or pulsed with peptide pool representing CFP10 (5 µg/ml each peptide; 15-mers overlap 11 aa) in an IFN- γ ELISPOT assay" Each responding T cell population was tested in duplicate at four different cell concentrations. To determine the effector cell frequency of antigen-specific T cells, the average number of spots per well for each duplicate was plotted against the number of respondei cells per well. Linear regression analysis was used to determine the slope of the line, which represents the frequency of antigen-specific T cells. The assay was considered positive (reflecting the presence of a primed T cell response), if the binomial probability for the number of spots was significantly different by experimental and control assays.

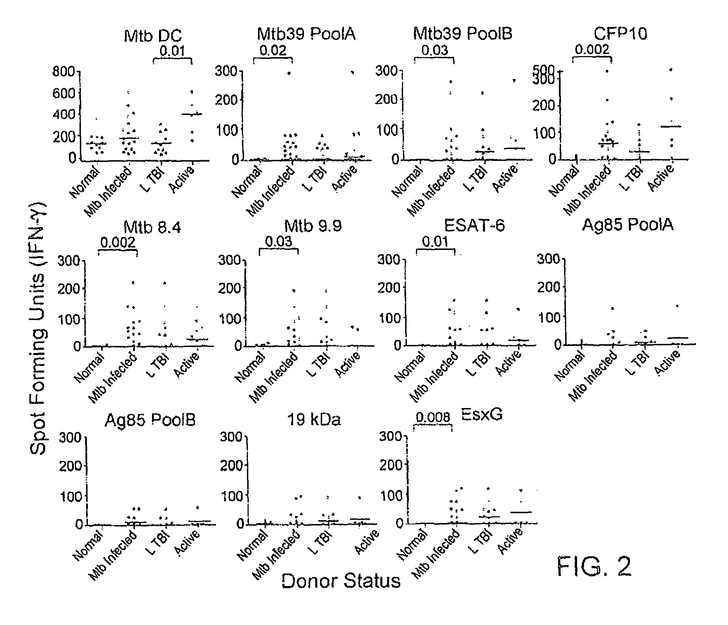

**Figure 2** is a set of graphs showing *ex vivo* CD8⁺ T cell frequencies to Mtb antigens are associated with Mtb infection. As described above (see Fig. 1), to determine *ex vivo* CD8⁺ T cell frequencies, autologous DC either infected with Mtb or pulsed with cognate peptide pools were incubated with CD8⁺ T cells in an IFN-γ ELISPOT assay. Subjects without evidence for Mtb infection, those with LTBI, and those with active TB (culture confirmed pulmonary tuberculosis) were evaluated. "Mtb Infected" includes those with latent tuberculosis (TB) infection and active tuberculosis. P values are noted where P = <0.05 (Wilcoxon/Kruskal-Wallis).

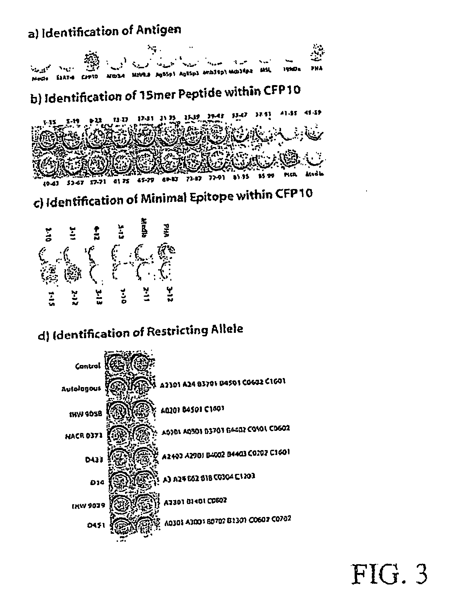

**Figures 3a to 3d** are a set of digital images showing the definition of' Antigenic Specificity and HLA-Restriction (the characterization of T cell clone D466 D6). For the results shown in Figures 3a-3c, to identify the antigen and minimal epitope recognized by T cell clone, D466 D6, T-cells (5000 cells/well) were incubated with autologous LCL (20,000/well) and 5 µg/ml of antigen. IFN- γ was assessed by ELISPOT after eighteen hours of co-culture. For the results presented in Figure 3a, antigens consisted of peptide pools representing known CD4⁺ antigens, made up of 15 amino acid (aa) peptides overlapping by 11 aa. For the results presented in Figure 3b, antigens consisted of individual 15 aa CFP10 peptides that together constitute the peptide pool For the results presented in Figure 3c, antigens consisted of individual nested CFP10₁₋₁₅ peptides (10 aa, 9 aa or 8 aa), used to further map the epitope. For the results presented in Figure 3d, the restricting allele was identified using LCL (20,000/well) expressing HLA alleles matching D466 at one or two alleles, pulsed with CFP10₂₋₁₀ (5µg/ml) as APC. After 2 hours, cells were washed and incubated with T-cells (500 cells/well) in an IFN- γ ELISPOT assay.

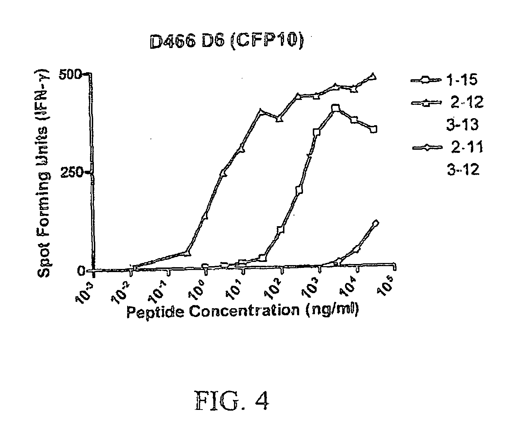

**Figure 4** is a line graph showing the conformation of minimal epitope mapping of D466 D6. To confirm the minimal epitope, autologous LCL (20,000/well) was pulsed with peptide at the concentration indicated and co-cultured with T-cells (1000 cells/well). IFN-γ was assessed by ELISPOT after eighteen hours co-culture Each point represents the mean of duplicate determinations

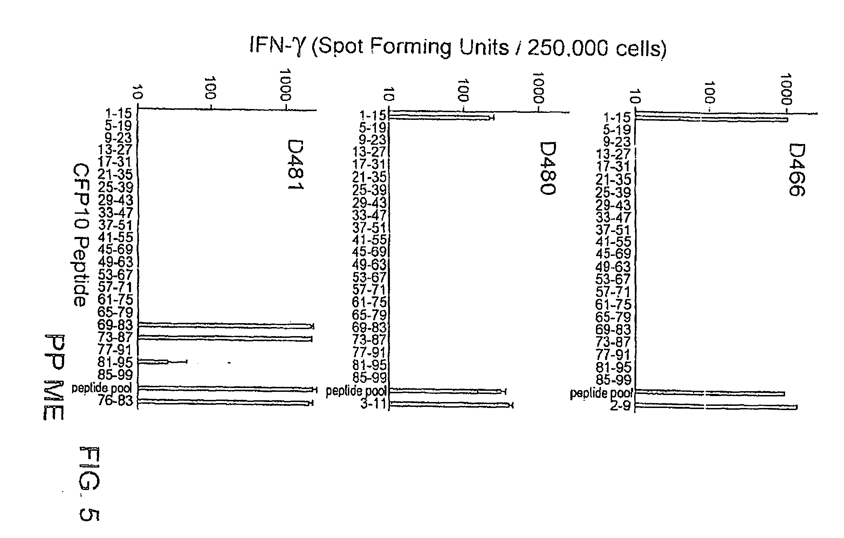

**Figure 5** is a set of bar graphs showing the profiling of the immunodominance pattern for CFP10. To determine the effector cell frequencies, autologous DC (20,000 / well) were pulsed either with each individual 15-mer peptide (5 µg/ml), the peptide pool (PP; 5 µg/each peptide) or the minimal epitope (ME) determined from T cell clones derived from each donor (D466:CFP10₂₋₁₁; D480:CFP10₃₋₁₁; D481:CFP10₇₅₋₈₃; 5 µg/ml), and tested against 250,000 magnetic bead purified CD8⁺ T cells. IFN-γ release was assessed by ELISPOT after eighteen hours of co-culture. Each point represents the mean of duplicate determinations.

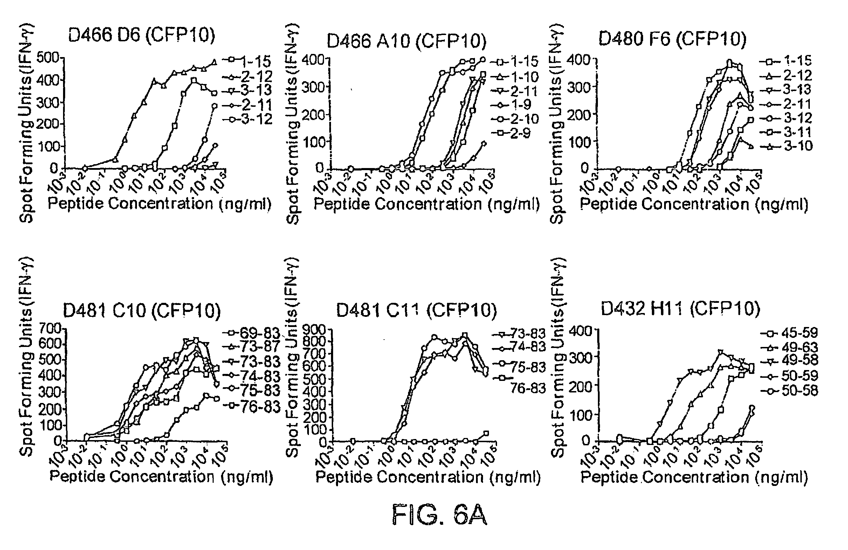

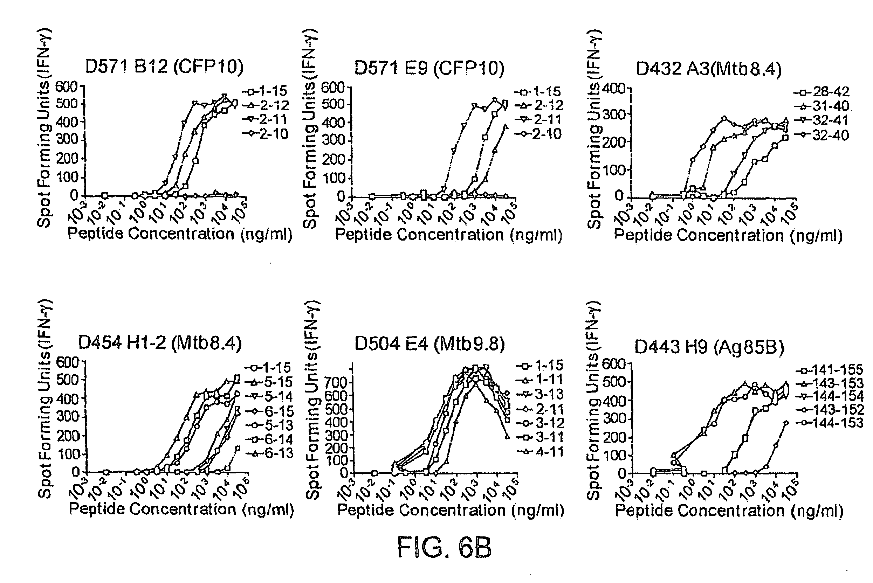

**Fig. 6** is a set of graphs summarizing the minimal epitope mapping data. To determine the minimal epitope, autologous LCL (20,000/well) was pulsed with peptide at the concentration indicated and co-cultured with T-cells (1000 cells/well). IFN-γ) was assessed by ELISPOT after eighteen hours co-culture. Each point represents the mean of duplicate determinations.

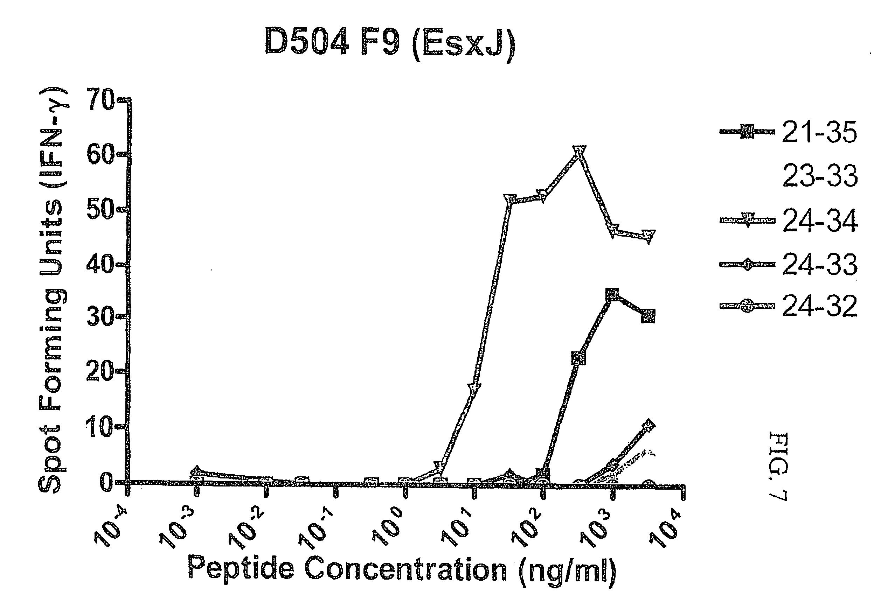

**Figure 7** is a line graph showing the mapping of Minimal Epitope for D504 Clones. To determine the minimal epitope, autologous LCL (20,000/well) was co-cultured with T-cell clones (1,000cells/well) and the peptide at the concentration indicated IFN-γ was assessed by ELISPOT after eighteen hours co-culture. Each point represents the mean of duplicate determinations.

### SEQUENCE LISTING

The nucleic and amino acid sequences listed in the accompanying sequence listing are shown using standard letter abbreviations for nucleotide bases, and three letter code for amino acids, as defined in 37 C.F.R. 1.822. Only one strand of each nucleic acid sequence is shown, but the complementary strand is understood as included by any reference to the displayed strand. In the accompanying sequence listing:
SEQ ID NOs: 1-12 are the amino acid sequence of Mtb polypeptides.
SEQ ID NOs: 13-14 are amino acids of Mtb peptides.
SEQ ID NOs: 15-25 are the nucleic acid sequences of polynucleotides encoding the Mtb polypeptides.
SEQ ID NOs: 26-38 are the amino acid sequences of specific Mtb epitopes.

### DETAILED DESCRIPTION

Methods for producing an immune response to *Mycobacterium tuberculosis* (Mtb) are disclosed herein. In several examples, the immune response is a protective immune response. In additional embodiments, methods are disclosed for preventing an infection with Mtb, or treating an infection with Mtb. Pharmaceutical compositions for the prevention and/or treatment of tuberculosis are also disclosed.

### Terms

Unless otherwise noted, technical terms are used according to conventional usage. Definitions of common terms in molecular biology may be found in Benjamin Lewin, Genes V, published by Oxford University Press, 1994 (ISBN 0-19 854287-9); Kendrew et al. (eds), The Encyclopedia of Molecular Biology, published by Blackwell Science Ltd.., 1994 (ISBN 0-632-02182-9); and Robert A. Meyers (ed.), Molecular Biology and Biotechnology: a Comprehensive Desk Reference, published by VCH Publishers, Inc., 1995 (ISBN 1-56081-569-8).

In order to facilitate review of the various embodiments of this disclosure, the following explanations of specific terms are provided:
**Adjuvant**: A vehicle used to enhance antigenicity. Adjuvants include a suspension of minerals (alum, aluminum hydroxide, or phosphate) on which antigen is adsorbed; or water-in-oil emulsion in which antigen solution is emulsified in mineral oil (Freund incomplete adjuvant), sometimes with the inclusion of killed mycobacteria (Freund's complete adjuvant) to further enhance antigenicity (inhibits degradation of antigen and/or causes influx of macrophages). Immunostimulatory oligonucleotides (such as those including a CpG motif) can also be used as adjuvants (for example see U.S. Patent No. 6,194,388; U.S. Patent No. 6,207,646; U.S. Patent No. 6,214,806; U.S. Patent No. 6,218,371; U.S. Patent No. 6,239,116; U.S. Patent No. 6,339,068; U.S. Patent No 6,406,705; and U.S. Patent No. 6,429,199). Adjuvants include biological molecules (a "biological adjuvant"), such as costimulatory molecules Exemplary adjuvants include IL-2, RANTES, GM-CSF, TNF-α, IFN-γ, G-CSF, LFA-3, CD72, B7-1, B7-2, OX-40L and 41 BBL.
**Antigen**: A compound, composition, or substance that can stimulate the production of antibodies or a T cell response in an animal, including compositions that are injected or absorbed into an animal. An antigen reacts with the products of specific humoral or cellular immunity, including those induced by heterologous immunogens. The term "antigen" includes all related antigenic epitopes. "Epitope" or antigenic determinant" refers to a site on an antigen to which B and/or T cells respond. In one embodiment, T cells respond to the epitope, when the epitope is presented in conjunction with an MHC molecule. Epitopes can be formed both from contiguous amino acids or noncontiguous amino acids juxtaposed by tertiary folding of a protein. Genterally, T cells recognize epitopes of continuous amino acids. Epitopes formed from contiguous amino acids are typically retained on exposure to denaturing solvents whereas epitopes formed by tertiary folding are typically lost on treatment with denaturing solvents. An epitope typically includes at least 3, and more usually, at least 5, about 9, or about 8-10 amino acids in a unique spatial conformation. Methods of determining spatial conformation of epitopes include, for example, x-ray crystallography and 2-dimensional nuclear magnetic resonance

An antigen can be a tissue-specific antigen, or a disease specific antigen These terms are not exclusive, as a tissue-specific antigen can also be a disease specific antigen. A tissue-specific antigen is expressed in a limited number of tissues, such as a single tissue. A tissue specific antigen may be expressed by more than one related type of tissue, such as alveolar and bronchial tissue. A disease-specific antigen is expressed coincidentally with a disease process. Specific non-limiting examples of a disease-specific antigen are an antigen whose expression correlate with, or is predictive of, tuberculosis. A disease-specific antigen can be an antigen recognized by T cells or B cells.

**Amplification:** Of a nucleic acid molecule (e.g.., a DNA or RNA molecule) refers to use of a technique that increases the number of copies of a nucleic acid molecule in a specimen An example of amplification is the polymerase chain reaction, in which a biological sample collected from a subject is contacted with a pair of oligonucleotide primers, under conditions that allow for the hybridization of the primers to a nucleic acid template in the sample. The primers are extended under suitable conditions, dissociated from the template, and then re-annealed, extended, and dissociated to amplify the number of copies of the nucleic acid The product of amplification can be characterized by electrophoresis, restriction endonuclease cleavage patterns, oligonucleotide hybridization or ligation, and/or nucleic acid sequencing using standard techniques. Other examples of amplification include strand displacement amplification, as disclosed in U.S. Patent No. 5,744,311; transcription-free isothermal amplification, as disclosed in U.S. Patent No. 6,033,881; repair chain reaction amplification, as disclosed in WO 90/01069; ligase chain reaction amplification, as disclosed in EP-A-320 308; gap filling ligase chain reaction amplification, as disclosed in U.S. Patent No. 5,427,930; and NASBA™ RNA transcription-free amplification, as disclosed in U.S. Patent No. 6,425,134.

Antibody: Immunoglobulin molecules and immunologically active portions of immunoglobulin molecules, i.e., molecules that contain an antigen binding site that specifically binds (immunoreacts with) an antigen.

A naturally occurring antibody (e.g., IgG, IgM, IgD) includes four polypeptide chains, two heavy (H) chains and two light (L) chains interconnected by disulfide bonds However, it has been shown that the antigen-binding function of an antibody can be performed by fragments of a naturally occurring antibody Thus, these antigen-binding fragments are also intended to be designated by the term "antibody." Specific, non-limiting examples of binding fragments encompassed within the term antibody include (i) a Fab fragment consisting of the V_{L}, V_{H}, C_{L} and C_{H1} domains; (ii) an F_{d} fragment consisting of the V_{H} and C_{H1} domains; (iii) an Fv fragment consisting of the V_{L} and V_{H} domains of a single arm of an antibody, (iv) a dAb fragment (Ward et al., Nature 341:544-546, 1989) which consists of a V_{H} domain; (v) an isolated complementarity determining region (CDR); and (vi) a F(ab')₂ fragment, a bivalent fragment comprising two Fab fragments linked by a disulfide bridge at the hinge region.

Immunoglobulins and certain variants thereof are known and many have been prepared in recombinant cell culture (e.g., see U.S. Patent No. 4,745,055; U.S. Patent No. 4,444,487; WO 88/03565; EP 256,654; EP 120,694; EP 125,023; Faoulkner et al., Nature 298:286, 1982; Morrison, J. Immunol. 123:793, 1979; Morrison et al., Ann Rev. Immunol 2:239, 1984).

**Animal:** Living multi-cellular vertebrate organisms, a category that includes, for example, mammals and birds. The term mammal includes both human and non-human mammals. Similarly, the term "subject" includes both human and veterinary subjects.

**Antigen presenting cell (APC):** A cell that can present an antigen to a T cell, such that the T cells are activated. Dendritic cells are the principle antigen presenting cells (APCs) involved in primary immune responses. Their major function is to obtain antigen in tissues, migrate to lymphoid organs and present the antigen in order to activate T cells.

When an appropriate maturational cue is received, dendritic cells are signaled to undergo rapid morphological and physiological changes that facilitate the initiation and development of immune responses. Among these are the up-regulation of molecules involved in antigen presentation; production of pro-inflammatory cytokines, including IL-12, key to the generation of Th1 responses; and secretion of chemokines that help to drive differentiation, expansion, and migration of surrounding naive Th cells. Collectively, these up-regulated molecules facilitate the ability of dendritic cells to coordinate the activation and effector function of other surrounding lymphocytes that ultimately provide protection for the host.

**cDNA (complementary DNA):** A piece of DNA lacking internal, non-coding segments (introns) and regulatory sequences that determine transcription. cDNA is synthesized in the laboratory by reverse transcription from messenger RNA extracted from cells.

**CD4:** Cluster of differentiation factor 4, a T cell surface protein that mediates interaction with the MHC Class II molecule. CD4 also serves as the primary receptor site for HIV on T cells during HIV infection. Cells that express CD4 are often helper T cells.

**CD8:** Cluster of differentiation factor 8, a T cell surface protein that mediates interaction with the MHC Class I molecule. Cells that express CD8 are often cytotoxic T cells. "CD8+ T cell mediated immunity" is an immune response implemented by presentation of antigens to CD8+T cells.

**cDNA (complementary DNA):** A piece of DNA lacking internal, non-coding segments (introns) and regulatory sequences that determine transcription cDNA is synthesized in the laboratory by reverse transcription from messenger RNA extracted from cells.

**Conservative variants:** "Conservative" amino acid substitutions are those substitutions that do not substantially affect or decrease an activity or antigenicity of the *Mycobacterium* polypeptide. Specific, non-limiting examples of a conservative substitution include the following examples:

| Original Residue | Conservative Substitutions |
|---|---|
| Ala | Ser |
| Arg | Lys |
| Asn | Gln, His |
| Asp | Glu |
| Cys | Ser |
| Gln | Asn |
| Glu | Asp |
| His | Asn; Gln |
| Ile | Leu, Val |
| Leu | Ile; Val |
| Lys | Arg; Gln; Glu |
| Met | Leu; Ile |
| Phe | Met; Leu; Tyr |
| Ser | Thr |
| Thr | Ser |
| Trp | Tyr |
| Tyr | Trp; Phe |
| Val | Ile; Leu |

The term conservative variation also includes the use of a substituted amino acid in place of an unsubstituted parent amino acid, provided that antibodies raised to the substituted polypeptide also immunoreact with the unsubstituted polypeptide, or that an immune response can be generated against the substituted polypeptide that is similar to the immune response against and unsubstituted polypeptide, such a *Mycobacterium* antigen, Thus, in one embodiment, non-conservative substitutions are those that reduce an activity or antigenicity.

**Consists Essentially Of/Consists Of:** With regard to a polypeptide, a polypeptide that consists essentially of a specified amino acid sequence if it does not include any additional amino acid residues. However, the polypeptide can include additional non-peptide components, such as labels (for example, fluorescent, radioactive, or solid particle labels), sugars or lipids. With regard to a polypeptide, a polypeptide that consists of a specified amino acid sequence does not include any additional amino acid residues, nor does it include additional non-peptide, components, such as lipids, sugars or labels

**Contacting:** The process of incubating one agent in the presence of another. Thus, when a cell is contacted with an agent, the cell is incubated with the agent for a sufficient period of time for the agent and the cell to interact.

**Costimulatory molecule:** Although engagement of the TCR with peptide-MHC delivers one signal to the T cell, this signal alone can be insufficient to activate the T cell. Costimulatory molecules are molecules that, when bound to their ligand, deliver a second signal required for the T cell to become activated. The most well-known costimulatory molecule on the T cell is CD28, which binds to either B7-1 (also called CD80) or B7-2 (also known as CD86). An additional costimulatory molecule is B7-3. Accessory molecules that also provide a second signal for the activation of T cells include intracellular adhesion molecule (ICAM-1 and ICAM-2), and leukocyte function associated antigen (LFA-1, LFA-2 and LFA-3). Integrins and tumor necrosis factor (TNF) superfamily members can also serve as costimulatory molecules.

**Cytokine:** Proteins made by cells that affect the behavior of other cells, such as lymphocytes. In one embodiment, a cytokine is a chemokine, a molecule that affects cellular trafficking. Specific, non-limiting examples of cytokines include the interleukins (IL-2, IL-4, IL-6, IL-10, IL-21, etc.); and IFN-γ.

**Degenerate variant:** A polynucleotide encoding an epitope of an Mtb polypeptide that includes a sequence that is degenerate as a result of the genetic code. There are 20 natural amino acids, most of which are specified by more than one codon. Therefore, all degenerate nucleotide sequences are included in this disclosure as long as the amino acid sequence of the Mtb polypeptide encoded by the nucleotide sequence is unchanged.

**Dendritic cell (DC):** Dendritic cells are the principle antigen presenting cells (APCs) involved in primary immune responses. Dendritic cells include plasmacytoid dendritic cells and myeloid dendritic cells. Their major function is to obtain antigen in tissues, migrate to lymphoid organs and present the antigen in order to activate T cells. Immature dendritic cells originate in the bone marrow and reside in the periphery as immature cells.

**Diagnostic:** Identifying the presence or nature of a pathologic condition, such as, but not limited to, tuberculosis. Diagnostic methods differ in their sensitivity and specificity. The "sensitivity" of a diagnostic assay is the percentage of diseased individuals who test positive (percent of true positives), The "specificity" of a diagnostic assay is 1 minus the false positive rate, where the false positive rate is defined as the proportion of those without the disease who test positive. While a particular diagnostic method may not provide a definitive diagnosis of a condition, it suffices if the method provides a positive indication that aids in diagnosis: "Prognostic" means predicting the probability of development (for example, severity) of a pathologic condition, such as tuberculosis.

**Displaying:** The process of localizing a peptide:antigen complex, or a peptide, on the outer surface of a cell where the peptide:antigen complex or peptide is accessible to a second cell, molecules displayed by a second cell, or soluble factors A peptide, or a peptide:antigen complex, is "displayed" by a cell when it is present on the outer surface of the cell and is accessible to a second cell, to molecules displayed by the second cell, or to soluble factors.

**Epitope:** An antigenic determinant. These are particular chemical groups or peptide sequences on a molecule that are antigenic, i.e. that elicit a specific immune response An antibody specifically binds a particular antigenic epitope on a polypeptide, such a *Mycobacterium* polypeptide.

**Expression Control Sequences:** Nucleic acid sequences that regulate the expression of a heterologous nucleic acid sequence to which it is operatively linked. Expression control sequences are operatively linked to a nucleic acid sequence when the expression control sequences control and regulate the transcription and, as appropriate, translation of the nucleic acid sequence. Thus expression control sequences can include appropriate promoters, enhancers, transcription terminators, a start codon (*i.e*., ATG) in front of a protein-encoding gene, splicing signal for introns, maintenance of the correct reading frame of that gene to permit proper translation of mRNA, and stop codons. The term "control sequences" is intended to include, at a minimum, components whose presence can influence expression, and can also include additional components whose presence is advantageous, for example, leader sequences and fusion partner sequences Expression control sequences can include a promoter.

A promoter is a minimal sequence sufficient to direct transcription: Also included are those promoter elements which are sufficient to render promoter-dependent gene expression controllable for cell-type specific, tissue-specific, or inducible by external signals or agents; such elements may be located in the 5' or 3' regions of the gene. Both constitutive and inducible promoters, are included (see *e*.*g*., Bitter et al., Methods in Enzymology 153:516-544, 1987). For example, when cloning in bacterial systems, inducible promoters such as pL of bacteriophage lambda, plac, ptrp, ptac (ptrp-lac hybrid promoter) and the like may be used. In one embodiment, when cloning in mammalian cell systems, promoters derived from the genome of mammalian cells (*e*.*g*., metallothionein promoter) or from mammalian viruses (*e*.*g*, the retrovirus long terminal repeat; the adenovirus late promoter; the vaccinia virus 7.5K promoter) can be used. Promoters produced by recombinant DNA or synthetic techniques may also be used to provide for transcription of the nucleic acid sequences. In one embodiment, the promoter is a cytomegalovirus promoter.

**Fractionating:** Subjecting a sample to conditions or procedures which separate the components of the sample based on physical or chemical properties such as, but not limited to, size, charge, solubility, or composition. Example of fractionation procedures include, but are not limited to, selective precipitation, organic extraction, size exclusion dialysis or chromatography, such as ion exchange chromatography, In one embodiment, a fraction is a soluble extract or an organic extract of an organism, such as a *Mycobacterium.*

**Functionally Equivalent:** Sequence alterations, such as in an epitope of an antigen, that yield the same results as described herein. Such sequence alterations can include, but are not limited to, conservative substitutions, deletions, mutations, frameshifts, and insertions.:

**Heterologous:** Onginating from separate genetic sources or species. A polypeptide that is heterologous to an Mtb polypeptide originates from a nucleic acid that does not encode the Mtb polypeptide. In one specific, non-limiting example, a polypeptide comprising nine consecutive amino acids from an Mtb polypeptide, or at most 20 consecutive amino acids from the Mtb polypeptide, and a heterologous amino acid sequence includes a β-galactosidase, a maltose binding protein, albumin, hepatitis B surface antigen, or an immunoglobulin amino acid sequence. Generally, an antibody that specifically binds to a protein of interest will not specifically bind to a heterologous protein.

**Host cells:** Cells in which a vector can be propagated and its DNA expressed. The cell may be prokaryotic or eukaryotic. The cell can be mammalian, such as a human cell. The term also includes any progeny of the subject host cell. It is understood that all progeny may not be identical to the parental cell since there may be mutations that occur during replication. However, such progeny are included when the term "host cell" is used.

**Human Leukocyte Antigen (HLA):** A genetic designation of the human major histocompatibility complex (MHC). Individual loci are designated by uppercase letters, as in HLA-E, and alleles are designated by numbers, as in HLA-A*0201. The three main MHC class I genes are called HLA-A, HLA-B, and HLA-C. However, there are many genes that encode β2 microglobulin-associated cell surface molecules that are linked to the MHC class I genes. The expression of these genes is variable, both in the tissue distribution and the amount expressed on cells; these genes have been termed the MHC class IB genes.

**Immune response:** A response of a cell of the immune system, such as a B cell, natural killer cell, or a T cell, to a stimulus In one embodiment, the response is specific for a particular antigen (an "antigen-specific response"). In one embodiment, an immune response is a T cell response, such as a Th1, Th2, or Th3 response. In another embodiment, an immune response is a response of a suppressor T cell.

**Immunogenic peptide:** A peptide which comprises an allele-specific motif or other sequence such that the peptide will bind an MHC molecule and induce a cytotoxic T lymphocyte ("CTL") response, or a B cell response (e.g. antibody production) against the antigen from which the immunogenic peptide is derived.

In one embodiment, immunogenic peptides are identified using sequence motifs or other methods, such as neural net or polynomial determinations, known in the art. Typically, algorithms are used to determine the "binding threshold" of peptides to select those with scores that give them a high probability of binding at a certain affinity and will be immunogenic. The algorithms are based either on the effects on MHC binding of a particular amino acid at a particular position, the effects on antibody binding of a particular amino acid at a particular position, or the effects on binding of a particular substitution in a motif containing peptide. Within the context of an immunogenic peptide, a "conserved residue" is one which appears in a significantly higher frequency than would be expected by random distribution at a particular position in a peptide. In one embodiment, a conserved residue is one where the MHC structure may provide a contact point with the immunogenic peptide.

Immunogenic peptides can also be identified by measuring their binding to a specific MHC protein and by their ability to stimulate CD4 and/or CD8 when presented in the context of the MHC protein.

In one example, an immunogenic "Mtb peptide" is a series of contiguous amino acid residues from the Mtb protein generally between 9 and 20 amino acids in length, such as about 9 to 12 residues in length. Specific immunogenic polypeptides are disclosed herein that are 9 or 10 amino acid residues in length, or at most 12 amino acids in length. Generally, immunogenic Mtb polypeptides can be used to induce an immune response in a subject, such as a B cell response or a T cell response. In one example, an immunogenic Mtb polypeptide, when bound to a MHC Class I molecule, activates cytotoxic T lymphocytes (CTLs) against Mtb. Induction of CTLs using synthetic peptides and CTL cytotoxicity assays are known in the art, see U.S. Patent 5,662,907. In one example, an immunogenic peptide includes an allele-specific motif or other sequence such that the peptide will bind an MHC molecule and induce a cytotoxic T lymphocyte ("CTL") response against the antigen from which the immunogenic peptide is derived.

**Immunogenic composition:** A composition comprising an immunogenic Mtb polypeptide or a nucleic acid encoding the immunogenic Mtb polypeptide that induces a measurable CTL response against Mtb, or induces a measurable B cell response (such as production of antibodies that specifically bind an Mtb polypeptide). For *in vitro* use, the immunogenic composition can consist of the isolated nucleic acid, vector including the nucleic acid/or immunogenic peptide. For *in vivo* use, the immunogenic composition will typically comprise the nucleic acid vector including the nucleic acid, and or immunogenic polypeptide, in pharmaceutically acceptable carriers, and/or other agents. An immunogenic composition can optionally include an adjuvants, a costimulatory molecule, or a nucleic acid encoding a costimulatory mlolecule, An Mtb polypeptide, or nucleic acid encoding the polypeptide, can be readily tested for its ability to induce a CTL by art-recognized assays.

**Inhibiting or treating a disease**: Inhibiting a disease, such as tuberculosis, refers to inhibiting the full development of a disease. In several examples, inhibiting a disease refers to lessening symptoms of a tuberculosis. "Treatment" refers to a therapeutic intervention that ameliorates a sign or symptom of a disease or pathological condition related to the disease, such as tuberculosis.

**Interferon gamma (γ):** IFN-γ is a dimeric protein with subunits of 146 amino acids. The protein is glycosylated at two sites, and the pI is 8.3-8.5. IFN-γ is synthesized as a precursor protein of 166 amino acids including a secretory signal sequence of 23 amino acids. Two molecular forms of the biologically active protein of 20 and 25 kDa have been described. Both of them are glycosylated at position 25 The 25 kDa form is also glycosylated at position 97. The observed differences of natural IFN-γ with respect to molecular mass and charge are due to variable glycosylation patterns. 40-60 kDa forms observed under non-denaturing conditions are dimers and tetramers IFNf-γ. The human gene has a length of approximately 6 kb. It contains four exons and maps to chromosome 12q24.1.

IFN-γ can be detected by sensitive immunoassays, such as an ELISPOT test that allows detection of individual cells producing IFN-γ. Minute amounts of IFN-γ can be detected indirectly by measuring IFN-induced proteins such as Mx protein. The induction of the synthesis of IP-10 has been used also to measure IFN-γ concentrations In addition, bioassays can be used to detect IFN-γ, such as an assay that employs induction of indoleamine 2,3-dioxygenase activity in 2D9 cells. The production of IFN-γ can be used to assess T cell activation, such as activation of a I cell by an HLA-E presented *Mycobacterium* antigen.

**Isolated**. An "isolated" nucleic acid has been substantially separated or purified away from other nucleic acid sequences in the cell of the organism in which the nucleic acid naturally occurs, i.e., other chromosomal and extrachromosomal DNA and RNA. The term "isolated" thus encompasses nucleic acids purified by standard nucleic acid purification methods. The term also embraces nucleic acids prepared by recombinant expression in a host cell as well as chemically synthesized nucleic acids.

**Label:** A detectable compound or composition that is conjugated directly or indirectly to another molecule to facilitate detection of that molecule Specific, non-limiting examples of labels include fluorescent tags, enzymatic linkages, and radioactive isotopes.

**Linker sequence**: A linker sequence is an amino acid sequence that covalently links two polypeptide domains. Linker sequences can be included in between the Mtb epitopes disclosed herein to provide rotational freedom to the linked polypeptide domains and thereby to promote proper domain folding and presentation to the MHC By way of example, in a recombinant polypeptide comprising two Mtb domains, linker sequences can be provided between them, such as a polypeptide comprising Mtb polypeptide-linker Mtb polypeptide. Linker sequences, which are generally between 2 and 25 amino acids in length, are well known in the art and include, but are not limited to, the glycine(4)-serine spacer (GGGGS x3) described by Chaudhary et al., Nature 339:394-397, 1989.

**Lymphocytes**: A type of white blood cell that is involved in the immune defenses of the body. There are two main types of lymphocytes: B cells and T cells.

**Mammal**: This term includes both human and non-human mammals. Similarly, the term "patient" or "subject" includes both human and veterinary subjects.

**Mycobacteria**: A genus of aerobic intracellular bacterial organisms. Upon invasion of a host, these organisms survive within endosomal compartments of' monocytes and macrophages. Human mycobacterial diseases include tuberculosis (cause by *M. tuberculosis*); Leprosy (caused by *M leprae*), Bairnsdale ulcers (caused by *M. ulcerans*), and other infections that can be caused by *M. marinum, M. kansasii, M. scrofulaceum, M. szulgai, M. xenopi, M. fortuitum, M*. *haemophilum, M. chelonei,* and *M. intracelluare. Mycobacterlum* strains that were previously considered to be nonpathogenic (such as *M. avium)* are also now known to be major killers of immunosuppressed AIDS patients.

An immune response to mycobacteria can involve cell mediated hypersensitivity (DTH) reactions with T cells and macrophages playing major roles in the intracellular killing and walling off (or containing) of the organism (granuloma formation). A T cell response can involve CD4⁺ lymphocytes that recognize myocbacterial heat shock proteins and immunodominant antigens as well as CD8⁺ cells.

**Operably linked:** A first nucleic acid sequence is operably linked with a second nucleic acid sequence when the first nucleic acid sequence is placed in a functional relationship with the second nucleic acid sequence. For instance, a promoter is operably linked to a coding sequence if the promoter effects the transcription or expression of the coding sequence. Generally, operably linked DNA sequences are contiguous and, where necessary to join two protein coding regions, the open reading frames are aligned.

**ORF (open reading frame):** A series of nucleotide triplets (codons) coding for amino acids without any termination codons. These sequences are usually translatable into a polypeptide.

**Peptide Modifications:** *Mycobacterium* polypeptides include synthetic embodiments of peptides described herein. In addition, analogues (non-peptide organic molecules), derivatives (chemically functionalized peptide molecules obtained starting with the disclosed peptide sequences) and variants (homologs) of these proteins can be utilized in the methods described herein. Each polypeptide of the invention is comprised of a sequence of amino acids, which may be either Land/or D- amino acids, naturally occurring and otherwise.

Peptides may be modified by a variety of chemical techniques to produce derivatives having essentially the same activity as the unmodified peptides, and optionally having other desirable properties. For example, carboxylic acid groups of the protein, whether carboxyl-terminal or side chain, may be provided in the form of a salt of a pharmaceutically-acceptable cation or esterified to form a C₁-C₁₆ ester, or converted to an amide of formula NR₁R₂ wherein R₁ and R₂ are each independently H or C₁-C₁₆ alkyl, or combined to form a heterocyclic ring, such as a 5- or 6-membered ring. Amino groups of the peptide, whether amino-terminal or side chain, may be in the form of a pharmaceutically-acceptable acid addition salt, such as the HCl, HBr, acetic, benzoic, toluene sulfonic, maleic, tartaric and other organic salts, or may be modified to C₁-C₁₆ alkyl or dialkyl amino or further converted to an amide.

Hydroxy groups of the peptide side chains may be converted to C₁-C₁₆ alkoxy or to a C₁-C₁₆ ester using well-recognized techniques. Phenyl and phenolic rings of the peptide side chains may be substituted with one or more halogen atoms, such as fluorine, chlorine, bromine or iodine, or with C₁-C₁₆ alkyl, C₁-C₁₆ alkoxy, carboxylic acids and esters thereof, or amides of such carboxylic acids Methylene groups of the peptide side chains can be extended to homologous C₂-C₄ alkylenes. Thiols can be protected with any one of a number of well-recognized protecting groups, such as acetamide groups, Those skilled in the art will also recognize methods for introducing cyclic structures into the peptides of this invention to select and provide conformational constraints to the structure that result in enhanced stability.

Peptidomimetic and organomimetic embodiments are envisioned, whereby the three-dimensional arrangement of the chemical constituents of'such peptido- and organomimetics mimic the three-dimensional arrangement of the peptide backbone and component amino acid side chains, resulting in such peptido and organomimetics of a *Mycobacterium* polypeptide having measurable or enhanced ability to generate an immune response. For computer modeling applications, a pharmacophore is an idealized, three-dimensional definition of the structural requirements for biological activity. Peptido- and organomimetics can be designed to fit each pharmacophore with current computer modeling software (using computer assisted drug design or CADD). See Walters, "Computer-Assisted Modeling of Drugs", in Klegerman & Groves, eds., 1993, Pharmaceutical Biotechnology, Interpharm Press: Buffalo Grove, IL, pp. 165-174 and Principles of Pharmacology Munson (ed.) 1995, Ch. 102, for descriptions of techniques used in CADD. Also included are mimetics prepared using such techniques.

**Pharmaceutical agent or drug:** A chemical compound or composition capable of inducing a desired therapeutic or prophylactic effect when properly administered to a subject.

**Pharmaceutically acceptable carriers:** The pharmaceutically acceptable carriers useful with the polypeptides and nucleic acids described herein are conventional Remington's Pharmaceutical Sciences, by E. W. Martin, Mack Publishing Co., Easton, PA, 15th Edition (1975), describes compositions and formulations suitable for pharmaceutical delivery of the fusion proteins herein disclosed.

In general, the nature of the carrier will depend on the particular mode of administration being employed. For instance, parenteral formulations usually comprise injectable fluids that include pharmaceutically and physiologically acceptable fluids such as water, physiological saline, balanced salt solutions, aqueous dextrose, glycerol or the like as a vehicle. For solid compositions (*e.g*., powder, pill, tablet, or capsule forms), conventional non-toxic solid carriers can include, for example, pharmaceutical grades of mannitol, lactose, starch, or magnesium stearate. In addition to biologically-neutral carriers, pharmaceutical compositions to be administered can contain minor amounts of non-toxic auxiliary substances, such as wetting or emulsifying agents, preservatives, and pH buffering agents and the like, for example sodium acetate or sorbitan monolaurate.

**Polynucleotide**: A linear nucleotide sequence, including sequences of greater than 100 nucleotide bases in length.

**Polypeptide**: Any chain of amino acids, regardless of length or post-translational modification (e.g., glycosylation or phosphorylation). A "peptide" is a chain of amino acids that is less than 100 amino acids in length. In one embodiment, a "peptide" is a portion of a polypeptide, such as at about 10, 20, 30, 40*,* 50*,* or 100 contiguous amino acids of a polypeptide that is greater than 100 amino acids in length.

**Portion of a nucleic acid sequence:** At least 10, 20, 30 or 40 contiguous nucleotides of the relevant sequence, such as a sequence encoding an antigen. In some instances it would be advantageous to use a portion consisting of 50 or more nucleotides. For instance, when describing a portion of an antigen (such as an antigenic epitope), it may be advantageous to remove a portion of the relevant sequence comprising at least 10, 20, 30, 40 or 50 nucleotides up to a length.

**Probes and primers:** Nucleic acid probes and primers may readily be prepared based on the nucleic acids provided by this invention. A probe comprises an isolated nucleic acid attached to a detectable label or reporter molecule. Typical labels include radioactive isotopes, ligands, chemiluminescent agents, and enzymes. Methods for labeling and guidance in the choice of labels appropriate for various purposes are discussed, e.g., in Sambrook et al. (1989) and Ausubel et al. (1987)

Primers are short nucleic acids, preferably DNA oligonucleotides 15 nucleotides or more in length. Primers may be annealed to a complementary target DNA strand by nucleic acid hybridization to form a hybrid between the primer and the target DNA strand, and then extended along the target DNA stand by a DNA polymerase enzyme. Primer pairs can be used for amplification of a nucleic acid sequence; e.g., by the polymerase chain reaction (PCR) or other nucleic-acid amplification methods known in the art.

Methods for preparing and using probes and primers are described, for example, in Molecular Cloning. A Laboratory Manual, 2nd ed.., vol. 1-3, ed.. Sambrook et al., Cold Spring Harbor Laboratory Press, Cold Spring Harbor, NY, 1989, and Current Protocols in Molecular Biology, ed. Ausubel et al., Greene Publishing and Wiley-Interscience, New York, 1987 (with periodic updates). PCR primer pairs can be derived from a known sequence, for example, by using computer programs intended for that purpose such as Primer (Version 0.5, © 1991, Whitehead Institute for Biomedical Research, Cambridge, MA).

**Preventing or treating a disease:** "Preventing" a disease refers to inhibiting the full development of a disease, for example in a person who is known to be at risk of infection with *M. tuberculosis,* or M. leprae. An example of'a person with a known predisposition is someone living with a person diagnosed with tuberculosis, health care professionals, or someone otherwise known to have been exposed to *M. tuberculosis"* "Treatment" refers to a therapeutic intervention that ameliorates a sign or symptom of a disease or pathological condition, such as tuberculosis, after it has begun to develop.

**Promoter:** A promoter is an array of nucleic acid control sequences which direct transcription of a nucleic acid. A promoter includes necessary nucleic acid sequences near the start site of transcription, such as, in the case of a polymerase II type promoter, a TATA element. A promoter also optionally includes distal enhancer or repressor elements which can be located as much as several thousand base pairs from the start site of transcription. The promoter can be a constitutive or an inducible promoter. A specific, non-limiting example of a promoter is the HCMV IE promoter.

**Purified:** The term purified does not require absolute purity; rather, it is intended as a relative term. Thus, for example, a purified antigen preparation is one in which the antigen is more pure than the protein in its originating environment within a cell. A preparation of an antigen is typically purified such that the antigen represents at least 50% of the total protein content of the preparation However, more highly purified preparations may be required for certain applications. For example, for such applications, preparations in which the antigen comprises at least 75% or at least 90% of the total protein content may be employed.

**Recombinant:** A recombinant nucleic acid or polypeptide is one that has a sequence that is not naturally occuring or has a sequence that is made by an artificial combination of two or more otherwise separated segments of sequence This artificial combination is often accomplished by chemical synthesis or, more commonly, by the artificial manipulation of isolated segments of nucleic acids, e.g., by genetic engineering techniques.

**Sequence identity:** The similarity between amino acid sequences is expressed in terms of the similar ity between the sequences, otherwise referred to as sequence identity. Sequence identity is frequently measured in terms of percentage identity (or similarity or homology); the higher the percentage, the more similar the two sequences are. Variants of antigen polypeptides will possess a relatively high degree of sequence identity when aligned using standard methods.

Methods of alignment of sequences for comparison are well known in the art Altschul et al. (1994) presents a detailed consideration of sequence alignment methods and homology calculations. The NCBI Basic Local Alignment Search Tool (BLAST) (Altschul et al., 1990) is available from several sources, including the National Center for Biotechnology Information (NCBI, Bethesda, MD) and on the Internet, for use in connection with the sequence analysis programs blastp, blastn, blastx, tblastn and tblastx. It can be accessed at the NCBI website. A description of how to determine sequence identity using this program is available at the NCBI website, as are the default parameters.

Variants of antigenic polypeptides, such as a *Mycobacterium* polypeptide, are typically characterized by possession of at least 50% sequence identity counted over the full length alignment with the amino acid sequence of' a native antigen sequence using the NCBI Blast 2.0, gapped blastp set to default parameters. Proteins with even greater similarity to the reference sequences will show increasing percentage identities when assessed by this method, such as at least 60%, at least 65%, at least 70%, at least 75%, at least 80%, at least 90% or at least 95% sequence identity. When less than the entire sequence is being compared for sequence identity, variants will typically possess at least 75% sequence identity over short windows of 10-20 amino acids, and may possess sequence identities of at least 85% or at least 90% or 95% depending on their similarity to the reference sequence. Methods for determining sequence identity over such short windows are described at the NCBI website. Variants of MHC domain polypeptides also retain the biological activity of the native polypeptide.

**Therapeutically active polypeptide:** An agent, such as an epitope of Mtb that causes induction of an immune response, as measured by clinical response (such as an increase in a population of immune cells, increased cytolytic activity against Mtb, or measurable reduction of a symptom of an infection). Therapeutically active molecules can also be made from nucleic acids, Examples of a nucleic acid based therapeutically active molecule is a nucleic acid sequence that encodes a Mtb epitope, wherein the nucleic acid sequence is operably linked to a control element such as a promoter.

**Therapeutically effective dose:** A dose sufficient to prevent advancement, or to cause regression of the disease, or which is capable of relieving symptoms caused by the disease. In one embodiment, a therapeutically effective dose is a dose sufficient to prevent advancement or relieve symptoms of tuberculosis

**Transduced and Transformed:** A virus or vector "transduces" a ceil when it transfers nucleic acid into the cell. A cell is "transformed" by a nucleic acid transduced into the cell when the DNA becomes stably replicated by the cell, either by incorporation of the nucleic acid into the cellular genome, or by episomal replication. As used herein, the term transformation encompasses all techniques by which a nucleic acid molecule might be introduced into such a cell, including transfection with viral vectors, transformation with plasmid vectors, and introduction of naked DNA by electroporation, lipofection, and particle gun acceleration.

**Tuberculosis (TB):** A disease that is generally caused by *Mycobacterium tuberculosis* that usually infects the lungs. However, other "atypical" mycobacteria such as *M. kansasii* may produce a similar clinical and pathologic appearance of disease.

Transmission of *M*. *tuberculosis* occurs by the airborne route in confined areas with poor ventilation. In more than 90% of cases, following infection with *M. tuberculosis;* the immune system prevents development of disease from *M. tuberculosis,* often called, active tuberculosis. However, not all of the *M. tuberculosis* is killed, and thus tiny, hard capsules are formed. "Primary tuberculosis" is seen disease that develops following an initial infection, usually in children. The initial focus of infection is a small subpleural granuloma accompanied by granulomatous hilar lymph node infection. Together, these make up the Ghon complex. In nearly all cases, these granulomas resolve and there is no further spread of the infection. "Secondary tuberculosis" is seen mostly in adults as a reactivation of previous infection (or reinfection), particularly when health status declines. The granulomatous inflammation is much more florid and widespread Typically, the upper lung lobes are most affected, and cavitation can occur. Dissemination of tuberculosis outside of lungs can lead to the appearance of a number of uncommon findings with characteristic patterns that include skeletal tuberculosis, genital tract tuberculosis, urinary tract tuberculosis, central nervous system (CNS) tuberculosis, gastrointestinal tuberculosis, adrenal tuberculosis, scrofula, and cardiac tuberculosis. "Latent" tuberculosis is an Mtb infection in an individual that can be detected by a diagnostic assay, such as, but not limited to a tuberculin skin test (TST) wherein the infection does not produce symptoms in that individual. "Active" tuberculosis is a symptomatic Mtb infection in a subject.

Microscopically, the inflammation produced with TB infection is granulomatous, with epithelioid macrophages and Langhans giant cells along with lymphocytes, plasma cells, maybe a few polymorphonuclear cells, fibroblasts with collagen, and characteristic caseous necrosis in the center. The inflammatory response is mediated by a type IV hypersensitivity reaction, and skin testing is based on this reaction. In some examples, tuberculosis can be diagnosed by a skin test, an acid fast stain, an auramine stain, or a combination thereof. The most common specimen screened is sputum, but the histologic stains can also be performed on tissues or other body fluids.

TB is a frequent complication of HIV infection. TB infection in subjects infected with a human immunodeficiency virus (HIV) can spread readily and progress rapidly to active disease. Specific symptoms of lung disease due to Mtb infection include chronic cough and spitting blood, Other symptoms of TB disease include fatigue, loss of appetite, weight loss, fever and drenching night sweats. :

**Vector:** A nucleic acid molecule as introduced into a host cell, thereby producing a transduced or transformed host cell. A vector may include nucleic acid sequences that permit it to replicate in a host cell, such as an origin of replication. A vector may also include one or more selectable marker gene and other genetic elements known in the art. Vectors include plasmid vectors, including plasmids for expression in gram negative and gram positive bacterial cells. Exemplary vectors include those for expression in E. coli and Salmonella. Vectors also include viral vectors, such as, but are not limited to, retrovirus, orthopox, avipox, fowlpox, capripox, suipox, adenoviral, herpes virus, alpha virus, baculovirus, Sindbis virus, vaccinia virus and poliovirus vectors. Vectors also include vectors for expression in yeast cells

Unless otherwise explained, all technical and scientific terms used herein have the same meaning as commonly understood by one of ordinary skill in the art to which this disclosure belongs. The singular terms "a," "an," and "the" include plural referents unless context clearly indicates otherwise Similarly, the word "or" is intended to include "and" unless the context clearly indicates otherwise. It is further to be understood that all base sizes or amino acid sizes, and all molecular weight or molecular mass values, given for nucleic acids or polypeptides are approximate, and are provided for description. Although methods and materials similar or equivalent to those described herein can be used in the practice or testing of this disclosure, suitable methods and materials are described below. The term "comprises" means "includes." In case of conflict, the present specification, including explanations of terms, will control. In addition, the materials, methods, and examples are illustrative only and not intended to be limiting.

### Mycobacterium Polypeptides

It is disclosed herein that several Mycobacterium polypeptides can be used to induce an immune response to Mtb, such as a T cell response. In several embodiments, the polypeptide comprises or consists of the amino acid sequence set forth as:
1.MX1 SRFMTDPHAMRDMAGRFEVHAQTVEDEARRMWASAQNISG AGWSGMAEATSLDTMX₂X₃MNQAFRNIVNMLHGVRDGLVRDANNY EQQEQASQQILS, (SEQ ID NO: 1, wherein X1 is A or T, X₂ is T or A and X₃ is any amino acid, such as Q or no amino acid)
In several examples, the polypeptide comprises or consists of the amino acid sequence set forth as:
a. MASRFMTDPHAMRDMAGRFEVHAQTVEDEARRMWASAQNISGA GWSGMAEATSLDTMTQMNQAFRNIVNMLHGVRDGLVRDANNYEQ QEQASQQILS (SEQ ID NO: 2) (See also TUBERCULIST No. Rv1038c, as available on March 1, 2007 known as EsxJ, *ES6_2, TB11.0, QILSS)*
*b*. MASRFMTDPHAMRDMAGRFEVHAQTVEDEARRMWASAQNISG AGWSGMAEATSLDTMAQMNQAFRNIVNMLHGVRDGLVRDANNYE QQEQASQQILSS (SEQ ID NO: 3, TUBERCULIST No. Rv1197, as available on March 1, 2007 also known as EsxK, *ES6_3, TB11.0, QILSS)*
*c*. MASRFMTDPHAMRDMAGRFEVHAQTVEDEARRMWASAQNISG AGWSGMAEATSLDTMT+MNQAFRNIVNMLHGVRDGLVRDANNYE QQEQASQQILSS (SEQ ID NO: 4, TUBERCULIST No. Rv 1992, as available on March 1, 2007 also known as EsxM, *TB11.0, QILSS.*
*d*. MATRFMTDPHAMRDMAGRFEVHAQTVEDEARRMWASAQNISG AGWSGMAEATSLDTMAQMNQAFRNIVNMLHGVRDGLVRDANNYE QQEQASQQILSS (SEQ ID NO: 5, TUBERCULIST No. Rv 2347c, as available on March 1, 2007, also known as EsxP, *ES6_7, QILSS)*
*e.* MTSRFMTDPHAMRDMAGRFEVHAQTVEDEARRMWASAQNISG AGWSGMAEATSLDTMTQMNQAFRNIVNMLHGVRDGLVRDANNYE QQEQASQQILSS (SEQ ID NO: 6, TUBERCULIST No. Rv3620c, as available on March 1, 2007 also known as EsxW, *ES6_10, QILSS).*
In additional embodiments, the polypeptide comprises or consists of the amino acid sequence set forth as:
2. MSYMIATPAALTAAATDIDGIGSAVSVANAAAVAATTGVLAAGG DEVLAAIARLFNANAEEYHALSAQVAAFQTLFVRTLTGGCGVFRRR RGRQCVTAAEHRAAGAGRRQRRRRSGDGQW RLRQQRHFGCGGQPEFRQHSEHRR (SEQ ID NO: 7, TUBERCULIST NO. Rv1088, as available on March 1, 2007, also known as PE9 the polypeptide for use according to the claimed invention).
3. VSLVIATPQLLATAALDLASIGSQVSAANAAAAMPTTEVVAAAA DEVSAAIAGLFGAHARQYQALSVQVAAFHEQFVQALTAAAGRYAST EAAVERSLLGAVNAPTEALLGRPLIGNGADGTAPGQPGAAGGLLFG NGGNGAAGGFGQTGGSGGAAGLIGNGGNGGAGGTGAAGGAGGNG GWLWGNGGNGGVGGTSVAAGIGGAGGNGGNAGLFGHGGAGGTG GAGLAGANGVNPTPGPAASTGDSPADVSGIGDQTGGDGGTGGHGTA GTPTGGTGGDGATATAGSGKATGGAGGDGGTAAAGGGGGNGGDG GVAQGDIASAFGGDGGNGSDGVAAGSGGGSGGAGGGAFVHIATAT STGGSGGFGGNGAASAASGADGGAGGAGGNGGAGGLLFGDGGNG GAGGAGGIGGDGATGGPGGSGGNAGIARFDSPDPEAEPDVVGGKGG DGGKGGSGLGVGGAGGTGGAGGNGGAGGLLFGNGGNGGNAGAGG DGGAGVAGGVGGNGGGGGTATFHEDPVAGVWAVGGVGGDGGSG GSSLGVGGVGGAGGVGGKGGASGMLIGNGGNGGSGGVGGAGGVG GAGGDGGNGGSGGNASTFGDENSIGGAGGTGGNGGNGANGGNGG AGGIAGGAGGSGGFLSGAAGVSGADGIGGAGGAGGAG GAGGSGGEAGAGGLTNGPGSPGVSGTEGMAGAPG (SEQ ID NO: 8, TUBERCULIST NO. Rv2487, as available on March 1, 2007, also known as PE_PGRS42.)
4. MHQVDPNLTRRKGRLAALAIAAMASASLVTVAVPATANADPEPA PPVPTTAASPPSTAAAPPAPATPVAPPPPAAANTPNAQPGDPNAAPPP ADPNAPPPPVIAPNAPQPVRIDNPVGGFSFALPAGWVESDAAHFDYG SALLSKTTGDPPFPGQPPPVANDTRIVLGRLDQKLYASAEATDSKAA ARLGSDMGEFYMPYPGTRINQETVSLDANGVSGSASYYEVKFSDPSK PNGQIWTGVIGSPAANAPDAGPPQRWFVVWLGTANNPVDKGAAKA LAESIRPLVAPPPAPAPAPAEP APAPAPAGEVAPTPTTPTPQRTLPA (SEQ ID NO: 9, TUBERCULIST No. Rv1860, as available on March 1, 2007, also known as *Apa, modD. mpt32)*
5. MLLALLRQHIRPYRRLVAMLMMLQLVSTLASLYLPTVNAAIVDD GVAKGDTATIVRLGAVMLGVTGLQVLCAIGAVYLGSRTGAGFGRDL RSAMFEHIITFSERETARFGAPTLLTRSTNDVRQILFLVQMTATVLVT APIMCVGGIIMAIHQEAALTWLLLVSVPILAVANYWIISHMLPLFRRM QSLIDGINRVMRDQLSGVRVVRAFTREGYERDKFAQANTALSNAAL SAGNWQALMLPVTTLTINASSVALIWFGGLRIDSGQMQVGSLIAFLS YFAQILMAVLMATMTLAVLPRASVCAERITEVLSTPAALGNPDNPKF PTDGVTGVVRLAGATFTYPGADCPVLQDISLTARPGTTTAIVGSTGS GKSTLVSLICRLYDVTAGAVLVDGIDVREYHTERLWSAIGLVPQRSY LFSGTVADNLRYGGGPDQVVTEQEMWEALRVAAADGFVQTDGLQT RVAQGGVNFSGGQRQRLAIARAVIRRPAIYVFDDAFSALDVHTDAK VHASLRQVSGDATIIVVTQRISNAAQADQVIVVDNGKIVGTGTHETL LADCPTYAEFAASQSLSATVGGVG (SEQ ID NO: 10, TUBERCULIST NO. Rv1273c, as available March 1, 2007).
6. MSYVIAAPEMLATTAADVDGIGSAIRAASASAAGPTTGLLAAAA DEVSSAAAALFSEYARECQEVLKQAAAFHGEFTRALAAAGAAYAQ AEASNTAAMSGTAGSSGALGSVGMLSGNPLTALMMGGTGEPILSDR VLAIIDSAYIRPIFGPNNPVAQYTPEQWWPFIGNLSLDQSIAQGVTLLN NGINAELQNGHDVVVFGYSQSAAVATNEIRALMALPPGQAPDPSRL AFTLIGNINNPNGGVLERYVGLYLPFLDMSFNGATPPDSPYQTYMYT GQYDGYAHNPQYPLNILSDLNAFMGIRWVHNAYPFTAAEVANAVPL PTSPGYTGNTHYYMFLTQDLPLLQPIRAIPFVGTPIAELIQPDLRVLVD LGYGYGYADVPTPASLFAPINPIAVASALATGTVQGPQAALVSIGLLP QSALPNTYPYLPSANPGLMFNFGQSSVTELSVLSGALGSVARLIPPIA (SEQ ID NO: 11, TUBERCULIST NO. Rv0159c, as available March 1, 2007 also known as PE3 or PE).
7. MEFPVLPPEINSVLMYSGAGSSPLLAAAAAWDGLAEELGSAAVSF GQVTSGLTAGVWQGAAAAAMAAAAAPYAGWLGSVAAAAEAVAG QARVVVGVFEAALAATVDPALVAANRARLVALAVSNLLGQNTPAIA AAEAEYELMWAADVAAMAGYHSGASAAAAALPAFSPPAQALGGG VGAFLTALFASPAKALSLNAGLGNVGNYNVGLGNVGVFNLGAGNV GGQNLGFGNAGGTNVGFGNLGNGNVGFGNSGLGAGLAGLGNIGLG NAGSSNYGFANLGVGNIGFGNTGTNNVGVGLTGNHLTGIGGLNSGT QNIGLFNSGTGNVGFFNSGIGNFGVFNSGNYNTGVGNAGTASTGLF NAGNFNTGVVNVGSYNTGSFNAGDTNTGGFNPGGVNTGWLNTGNT NTGIANSGNVNTGAFISGNFNNGVLWVGDYQGLFGVSAGSSIPAIPIG LVLNGDIGPITIQPIPILPTIPLSIHQTVNLGPLVVPDIVIPAFGGGIGIPIN IGPLTITPITLFAQQTFVNQLPFPTFSLGKITIPQIQTFDSNGQLVSFIGPI VIDTTIPGPTNPQIDLTIRWDTPPITLFPNGISAPDNPLGLLVSVSISNPG FTIPGFSVPAQPLPLSIDIEGQIDGFSTPPITIDRIPLTVGGGVTIGPITIQG LHIPAAPGVGNTTTAPSSGFFNSGAGGVSGFGNVGAGSSGWWNQAP SALLGAGSGVGNVGTLGSGVLNLGSGISGFYNTSVLPFGTPAAVSGI GNLGQQLSGVSAAGTTLRSMLAGNLGLANVGNFNTGFGNVGDVNL GAANIGGHNLGLGNVGDGNLGLGNIGHGNLGFANLGLTAGAAGVG NVGFGNAGINNYGLANMGVGNIGFANTGTGNIGIGLVGDHRTGIGG LNSGIGNIGLFNSGTGNVGFFNSGTGNFGIGNSGRFNTGIGNSGTAST GLFNAGSFSTGIANTGDYNTGSFNAGDTNTGGFNPGGINTGWFNTGH ANTGLANAGTFGTGAFMTGDYSNGLLWRGGYEGLVGVRVGPTISQF PVTVHAIGGVGPLHVAPVPVPAVHVEITDATVGLGPFTVPPISIPSLP
IASITGSVDLAANTISPIRALDPLAGSIGLFLEPFRLSDPFITIDAFQVVA GVLFLENIIVPGLTVSGQILVTPTPIPILTLNLDTTPWTLFPNGFIIPAQT PVTVGMEVANDGFTFFPGGLTFPRASAGVTGLSVGLDAFTLLPDGFT LDTVPATFDGTILIGDIPIPIIDVPAVPGFGNTTTAPSSGFFNTGGGGGS GFANVGAGTSGWWNQGHDVLAGAGSGVANAGTLSSGVLNVGS GISGWYNTSTLGAGTPAVVSGIGNLGQQLSGFLANGTVLNRSPIVNIG WADVGAFNIGLGNVGDLNWGAANIGAQNLGLGNLGSGNVGFGNIG AGNVGFANSGPAVGLAGLGNVGLSNAGSNNWGLANLGVGNIGLAN TGTGNIGIGLVGDYQTGIGGLNSGSGNIGLFNSGTGNVGFFNTGTGNF GLFNSGSFNTGIGNSGTGSTGLFNAGNFNTGIANPGSYNTGSFNVGDT NTGGFNPGDINTGWFNTGIMNIGTRNTGALMSGIDSNGMLWRGDH EGLFGLSYGITIPQFPIRITTIGGIGPIVIPDTTILPPLHLQITGDADYSFT VPDIPIPAIHIGINGVVTVGFTAPEATLLSALKNNGSFISFGPITLSNIDIP PMDFTLGLPVLGPITGQLGPIHLEPIVVAGIGVPLEIEPIPLDAISLSESIP IRIPVDIPASVIDGISMSEVVPIDASVDIPAVTITGTTISAIPLGFDIRTSA GPLNIPIIDIPAAPGFGNSTQMPSSGFFNTGAGGGSGIGNLGAGVSGLL NQAGAGSLVGTLSGLGNAGTLASGVLNSGTAISGLFNVSTLDATTPA VISGFSNLGDHMSGVSIDGLIAILTFPPAESVFDQIIDAAIAELQHLDIG NALALGNVGGVNLGLANVGEFNLGAGNVGNINVGAGNLGGSNLGL GNVGTGNLGFGNIGAGNFGFGNAGLTAGAGGLGNVGLGNAGS GSWGLANVGVGNIGLANTGTGNIGIGLIGDYRTGIGGLNSGTGNLGL FNSGTGNIGFFNTGTGNFGLFNSGSYSTGVGNAGTASTGLFNAGNFN TGLANAGSYNTGSLNVGSFNTGGVNPGTVNTGVIFNTGHTNTGLFNT GNVNTGAFNSGSFNNGALWTGDYHGLVGFSFSIDIAGSTLLDLNETL NLGPIHIEQIDIPGMSLFDVHEIVEIGPFTIPQVDVPAIPLEIHESIHMDPI VLVPATTIPAQTRTIPLDIPASPGSTMTLPLISMRFEGEDWILGSTAAIP NFGDPFPAPTQGITIHTGPGPGTTGELKISIPGFEIPQIATTRFLLDVNIS GGLPAFTLFAGGLTIPTNAIPLTIDASGALDPITIFPGGYTIDPLPLHLAL NLTVPDSSIPIIDVPPTPGFGNTTATPSSGFFNSGAGGVSGFGNVGSNL SGWWNQAASALAGSGSGVLNVGTLGSGVLNVGSGVSGIYN TSVLPLGIPAVLSGLGNTVGHQLSGVSAAGTALNQIPILNIGLADVGNF NVGFGNVGDVNLGAANLGAQNLGLGNVGTGNLGFANVGHGNIGFG NSGLTAGAAGLGNTGFGNAGSANYGFANQGVRNIGLANTGTGNIGI GLVGDNLTGIGGLNSGAGNIGLFNSGTGNIGFFNSGTGNFGIGNSGSF NTGIGNSGTGSTGLFNAGSFNTGVANAGSYNTGSFNAGDTNTGGFNP CYTINTGWFNTGHTNTGIANSGNVGTGAFMSGNFSNGLLWRGDHEGL FSLFYSLDVPRITIVDAHLDGGFGPVVLPPIPVPAVNAHLTGNVAMGA FTIPQIDIPALTPNITGSAAFRIVVGSVRIPPVSVIVEQIINASVGAEMRI DPFEMWTQGYNGLGITFYSFGSADGSPYATGPLVFGAGTSD GSHLTISASSGAFTTPQLETGPITLGFQVPGSVNAITLFPGGLTFPATSL LNLDVTAGAGGVDIPAITWPEIAASADGSVYVLASSIPLINIPPTPGIG NSTITPSSGFFNAGAGGGSGFGNFGAGTSGWWNQAHTALAGAGSGF ANVGTLHSGVLNLGSGVSGIYNTSTLGVGTPALVSGLGNVGHQLSG LLSGGSAVNPVTVLNIGLANVGSHNAGFGNVGEVNLGAANLGAHNL GFGNIGAGNLGFGNIGHGNVGVGNSGLTAGVPGLGNVGLGNAGGN NWGLANVGVGNIGLANTGTGNIGIGLTGDYQTGIGGLNSGAGNLGL FNSGAGNVGFFNTGTGNFGLFNSGSFNTGVGNSGTGSTGLFNAGSFN TGVANAGSYNTGSFNVGDTNTGGFNPGSINTGWLNAGNANTGVAN AGNVNTGAFVTGNFSNGILWRGDYQGLAGFAVGYTLPLFPAVGAD VSGGIGPITVLPPIHIPPIPVGFAAVGGIGPIAIPDISVPSIHLGLDPAVHV GSITVNPITVRTPPVLVSYSQGAVTSTSGPTSEIWVKPSFFPGIRIAPSS GGGATSTQGAYFVGPISIPSGTVTFPGFTIPLDPIDIGLPVSLTIPGFTIP GGTLIPTLPLGLALSNGIPPVDIPAIVLDRILLDLHADTTIGPINVPIAGF GGAPGFGNSTTLPSSGFFNTGAGGGSGFSNTGAGMSGLLNAMSDPLL GSASGFANFGTQLSGILNRGAGISGVYNTGALGVVTAAVVSGFGNV GQQLSGLLFTGVGP (SEQ ID NO: 12, TUBERCULIST No. 3350c, as available March 1, 2007 also known as PPE56 or PPE).

An Mtb polypeptide as described herein can have a sequence at least 75%, 85%, 90%, 95%, 96%, 97%, 98% or 99% homologous to the amino acid sequence set forth in one of SEQ ID NOs: 1-12. For example, the polypeptide can have an amino acid sequence, at least 85%, 90%, 95%, 96%, 97%, 98% or 99% homologous to one of the amino acid sequences set forth in SEQ ID NOs: 1-12. Exemplary sequences can be obtained using computer programs that are readily available on the internet and the amino acid sequences set forth herein In one example, the polypeptide retains a function of the Mtb protein, such as binding to an antibody that specifically binds the Mtb epitope.

Minor modifications of an Mtb polypeptide primary amino acid sequences may result in peptides which have substantially equivalent activity as compared to the unmodified counterpart polypeptide described herein. Such modifications may be deliberate, as by site-directed mutagenesis, or may be spontaneous. All of the polypeptides produced by these modifications are included herein. Thus, a specific, non-limiting example of a Mtb polypeptide is a conservative variant of the Mtb polypeptide. A table of conservative substitutions is provided herein. Substitutions of the amino acids sequence shown in SEQ ID NOs: 1-12 can be made based on this table.

Mtb polypeptides are disclosed herein that can be used to induce an immune response (immunogenic). These peptides include or consist of at least nine amino acids, such as nine to twenty amino acids consecutive amino acids of an Mtb polypeptide set forth above. Specific, non-limiting examples are twelve, eleven, ten amino acids, or nine consecutive amino acids of one of the Mtb polypeptides set forth above. In these examples, the Mtb polypeptide does not include the full-length amino acid sequences set forth as SEQ ID NOs: 1-12.

An isolated polypeptide is disclosed that includes nine to twelve consecutive amino acids from an Mtb polypeptide, wherein the isolated polypeptide comprises the amino acid sequence set forth as QTVEDEARRMW (SEQ ID NO: 13). In some embodiments, the polypeptide is nine, ten or eleven amino acids in length. In additional embodiments, the polypeptide consists of the amino acid sequence set forth as SEQ ID NO: 13. An isolated polypeptide is disclosed that includes nine to twelve consecutive amino acids from an Mtb polypeptide, wherein the isolated polypeptide comprises the amino acid sequence set forth as VSAAIAGLF (SEQ ID NO: 14). In some embodiments, the polypeptide is nine, ten or eleven amino acids in length. In additional embodiments, the polypeptide consists of the amino acid sequence set forth as SEQ ID NO: 14.

In several embodiments, the isolated Mtb polypeptide is included in a fusion protein. Thus, the fusion protein can include the Mtb polypeptide (see above) and a second heterologous moiety, such as a myc protein; an enzyme or a carrier (such as a hepatitis carrier protein or bovine serum albumin) covalently linked to the Mtb polypeptide. In several examples, a polypeptide consisting of nine to twelve amino acids of one of the amino acid sequences set forth as SEQ ID NOs: 1.14 that bind MHC class I is covalently linked to a carrier. In additional example, a polypeptide consisting of one of the amino acid sequences set forth as one of SEQ ID NOs: 1-14 is covalently linked to a carrier.

In additional examples, the polypeptide can be a fusion protein and can also include heterologous sequences to Mtb (such as amino acid sequences of at least nine amino acids in length that are not included in SEQ ID NO: 1). Thus, in several specific non-limiting examples, the immunogenic peptide is a fusion polypeptide, for example the polypeptide includes six sequential histidine residues, a β-galactosidase amino acid sequence, or an immunoglobulin amino acid sequence. The polypeptide can also be covalently linked to a carrier. Suitable carriers include, but are not limited to, a hepatitis B small envelope protein HBsAg. This protein has the capacity to self assemble into aggregates and can form viral-like particles. The preparation of HBsAg is well known, see for example European Patent Application Publication No. EP-A-0 226 846, European Patent Application Publication No. EP-A-0 299 108 and PCT Publication No. WO 01/117554, and the amino acid sequence disclosed, for example, in Tiollais et al., Nature, 317: 489, 1985, and European Patent Publication No. EP-A-0 278 940, and PCT Publication No. WO 91/14703.

In additional embodiments, the protein consists of the Mtb polypeptide A second heterologous moiety can be non-covalently linked to the Mtb polypeptide. For example, a polypeptide consisting of nine to twelve consecutive amino acids of one of the proteins set forth as one of SEQ ID NO: 1-14 can be non-covalently linked to a carrier.

The polypeptide can optionally include repetitions of one or more of the Mtb polypeptides disclosed herein. In one specific, non-limiting example, the polypeptide includes two, three, four, five, or up to ten repetitions of one of the Mtb polypeptides described above. Alternatively, more than one polypeptide can be included in a fusion polypeptide. Thus, in several examples, the polypeptide can include at least two, at least three, at least four, at least five or at least six of the amino acid sequences set forth as SEQ ID NOs: 1-14. A linker sequence can optionally be included between the Mtb polypeptides.

The Mtb polypeptides disclosed herein can be chemically synthesized by standard methods, or can be produced recombinantly. An exemplary process for polypeptide production is described in Lu et al., Federation of European Biochemical Societies Letters. 429:31-35, 1998. They can also be isolated by methods including preparative chromatography and immunological separations.

If desired, polypeptides can also be chemically synthesized by emerging technologies. One such process is described in W. Lu et al., Federation of European Biochemical Societies Letters. 429:31-35, 1998. Polypeptides can also be produced using molecular genetic techniques, such as by inserting a nucleic acid encoding Mtb or an epitope thereof into an expression vector, introducing the expression vector into a host cell, and isolating the polypeptide (see below).

An Mtb polypeptide can be covalently linked to a carrier, which is an immunogenic macromolecule to which an antigenic molecule can be bound. Carriers are chosen to increase the immunogenicity of the bound molecule and/or to elicit higher titers of antibodies against the carrier which are diagnostically, analytically, and/or therapeutically beneficial. Covalent linking of a molecule to a carrier can confer enhanced immunogenicity and T cell dependence (see Pozsgay et al., PNAS 96:5194-97, 1999; Lee et al., J. Immunol. 116:1711-18, 1976; Dintzis et al., PNAS 73:3671-75, 1976). Useful carriers include polymeric carriers, which can be natural (for example, polysaccharides, polypeptides or proteins from bacteria or viruses), semi-synthetic or synthetic materials containing one or more functional groups to which a reactant moiety can be attached Bacterial products and viral proteins (such as hepatitis B surface antigen and core antigen) can also be used as carriers, as well as proteins from higher organisms such as keyhole limpet hemocyanin, horseshoe crab hemocyanin, edestin, mammalian serum albumins, and mammalian immunoglobulins. Additional bacterial products for use as carriers include bacterial wall proteins and other products (for example, streptococcal or staphylococcal cell walls and lipopolysaccharide (LPS)).

Polynucleotides encoding the Mtb polypeptides disclosed herein are also provided. Exemplary nucleic acid sequences are set forth below:
ESXJ (ESAT-6 LIKE PROTEIN 2)
ESXK (ESAT 6 LIKE PROTEIN 3)
ESXM (ESAT 6 LIKE PROTEIN ESXM)
ESXP (ESAT-6 LIKE PROTEIN 7)
ESXW (ESAT-6 LIKE PROTEIN 10)
PE9 (PE FAMILY PROTEIN)
PE_PGRS42 (PE-PGRS FAMILY PROTEIN)
Rv1860 (FIBRONECTIN ATTACHMENT PROTEIN)
RV1273C (PROBABLE DRUGS-TRANSPORT TRANSMEMBRANE ATP-BINDING PROTEIN ABC TRANSPORTER)
Rv0159c (PE FAMILY PROTEIN)
RV3350C (PPE FAMILY PROTEIN)

These polynucleotides include DNA, cDNA and RNA sequences which encode the polypeptide of interest. Silent mutations in the coding sequence result from the degeneracy (i.e., redundancy) of the genetic code, whereby more than one codon can encode the same amino acid residue. Thus; for example, leucine can be encoded by CTT, CTC, CTA; CTG, TTA, or TTG; serine can be encoded by TCT, TCC, TCA, TCG, AGT, or AGC; asparagine can be encoded by AAT or AAC; aspartic acid can be encoded by GAT or GAC; cysteine can be encoded by TGT or TGC; alanine can be encoded by GCT, GCC, GCA, or GCG; glutamine can be encoded by CAA or CAG; tyrosine can be encoded by TAT or TAC; and isoleucine can be encoded by ATT, ATC, or ATA. Tables showing the standard genetic code can be found in various sources (e.g., L. Stryer, 1988, Biochemistry, 3 sup rd Edition, W.H. 5 Freeman and Co., NY).

A nucleic acid encoding a Mtb polypeptide can be cloned or amplified by *in vitro* methods, such as the polymerase chain reaction (PCR), the ligase chain reaction (LCR), the transcription-based amplification system (TAS), the self-sustained sequence replication system (3SR) and the Qβ replicase amplification system (QB). For example, a polynucleotide encoding the protein can be isolated by polymerase chain reaction of cDNA using primers based on the DNA sequence of the molecule. A wide variety of cloning and *in vitro* amplification methodologies are well known to persons skilled in the art. PCR methods are described in, for example, US Patent No. 4,683,195; Mullis et al., Cold Spring Harbor Symp. Quant Biol. 51:263, 1987; and Erlich, ed., PCR Technology, (Stockton Press, NY, 1989). Polynucleotides also can be isolated by screening genomic or cDNA libraries with probes selected from the sequences of the desired polynucleotide under stringent hybridization conditions.

The polynucleotides encoding a Mtb polypeptide include a recombinant DNA which is incorporated into a vector, into an autonomously replicating plasmid or virus; or into the genomic DNA of a prokaryote or eukaryote, or which exists as a separate molecule (such as a cDNA) independent of other sequences. The nucleotides of the invention can be ribonucleotides, deoxyribonucleotides, or modified forms of either nucleotide. The term includes single and double forms of DNA.

In one embodiment, vectors are used for expression in yeast such as S. *cerevisiae* or *Kluyveromyces lactis.* Several promoters are known to be of use in yeast expression systems such as the constitutive promoters plasma membrane H⁺. ATPase (*PMA1*), glyceraldehyde-3-phosphate dehydrogenase (*GPD*), phosphoglycerate kinase-1 (*PGK1*), alcohol dehydrogenase-1 (*ADH1*), and pleiotropic drug resistant pump (*PDR5*). In addition, may inducible promoters are of use, such as *GAL1-10* (induced by galactose), *PHO5* (induced by low extracellular inorganic phosphate), and tandem heat shock *HSE* elements (induced by temperature elevation to 37°C). Promoters that direct variable expression in response to a titratable inducer include the methionine-responsive *MET3* and *MET25* promoters and copper dependent *CUP1* promoters. Any of these promoters may be cloned into multicopy (2µ) or single copy (*CEN*) plasmids to give an additional level of control in expression level. The plasmids can include nutritional markers (such as *URA3, ADE3, HIS1,* and others) for selection in yeast and antibiotic resistance (*AMP*) for propagation in bacteria Plasmids for expression on *K lactis* are known, such as pKLAC1. Thus, in one example, after amplification in bacteria, plasmids can be introduced into the corresponding yeast auxotrophs by methods similar to bacterial transformation.

The Mtb polypeptides can be expressed in a variety of yeast strains. For example, seven pleiotropic drug-resistant transporters, *YOR1, SNQ2, PDR5, YCF1, PDR10, PDR11,* and *PDR15,* together with their activating transcription factors, *PDR1* and *PDR3,* have been simultaneously deleted in yeast host cells, rendering the resultant strain sensitive to drugs. Yeast strains with altered lipid composition of the plasma membrane, such as the *erg6* mutant defective in ergosterol biosynthesis, can also be utilized. Proteins that are highly sensitive to proteolysis can be expressed in a yeast lacking the master vacuolar endopeptidase Pep4, which controls the activation of other vacuolar hydrolases. Heterologous expression in strains carrying temperature-sensitive (*ts*) alleles of genes can be employed if the corresponding null mutant is inviable.

Viral vectors can also be prepared encoding the Mtb polypeptides disclosed herein. A number of viral vectors have been constructed, including polyoma, SV40 (Madzak et al., 1992, J. Gen. Virol., 73:15331536), adenovirus (Berkner, 1992, Cur Top. Microbiol. Immunol., 158:39-6; Berliner et al., 1988, Bio Techniques, 6:616-629; Gorziglia et al., 1992, J. Virol., 66:4407-4412; Quantin et al., 1992, Proc. Nad Acad. Sci. USA, 89:2581-2584; Rosenfeld et al., 1992, Cell, 68:143-155; Wilkinson et al., 1992, Nucl. Acids Res., 20:2233-2239; Stratford-Perricaudet et al., 1990, Hum. Gene Ther., 1:241-256), vaccinia virus (Mackett et al., 1992, Biotechnology, 24:495-499), adeno-associated virus (Muzyczka, 1992, Curr. Top. Microbiol. Immunol., 158:91-123; On et al., 1990, Gene, 89:279-282), herpes viruses including HSV and EBV (Margolskee, 1992, Curr. Top. Microbiol. Immunol., 158:67-90; Johnson et al., 1992, J. Virol., 66:29522965; Fink et al., 1992, Hum. Gene Ther. 3:11-19; Breakfield et al., 1987, Mol. Neurobiol., 1:337-371; Fresse et al., 1990, Biochem. Pharmacol., 40:2189-2199), Sindbis viruses (H. Herweijer et al., 1995, Human Gene Therapy 6:1161-1167; U.S. Patent No. 5,091,309 and U.S. Patent No. 5,2217,879), alphaviruses (S. Schlesinger, 1993, Trends Biotechnol. 11:18-22; I. Frolov et al., 1996, Proc. Natl. Acad. Sci. USA 93:11371-11377) and retroviruses of avian (Brandyopadhyay et al., 1984, Mol. Cell Biol., 4:749-754; Petropouplos et al., 1992, J. Virol., 66:3391-3397), murine (Miller, 1992, Curr. Top. Microbiol. Immunol., 158:1-24; Miller et al., 1985, Mol. Cell Biol., 5:431-437; Sorge et al., 1984, Mol. Cell Biol., 4:1730-1737; Mann et al., 1985, J. Virol., 54:401-407), and human origin (Page et al., 1990, J. Virol., 64:5370-5276; Buchschalcher et al., 1992, J. Virol., 66:2731-2739), Baculovirus (Autographa californica multinuclear polyhedrosis virus; AcMNPV) vectors are also known in the art, and may be obtained from commercial sources (such as PharMingen, San Diego, Calif.; Protein Sciences Corp., Meriden, Conn.; Stratagene, La Jolla, Calif.).

Thus, in one embodiment, the polynucleotide encoding an Mtb polypeptide is included in a viral vector. Suitable vectors include retrovirus vectors, orthopox vectors, avipox vectors, fowlpox vectors, capripox vectors, suipox vectors, adenoviral vectors, herpes virus vectors, alpha virus vectors, baculovirus vectors, Sindbis virus vectors, vaccinia virus vectors and poliovirus vectors. Specific exemplary vectors are poxvirus vectors such as vaccinia virus, fowlpox virus and a highly attenuated vaccinia virus (MVA), adenovirus, baculovirus and the like.

Pox viruses useful in practicing the present methods include orthopox, suipox, avipox, and capripox virus. Orthopox include vaccinia, ectromelia, and raccoon pox. One example of an orthopox of use is vaccinia. Avipox includes fowlpox, canary pox and pigeon pox. Capripox include goatpox and sheeppox. In one example, the suipox is swinepox. Examples of pox viral vectors for expression as described for example, in U.S. Patent No. 6,165,460. Other viral vectors that can be used include other DNA viruses such as herpes virus and adenoviruses, and RNA viruses such as retroviruses and polio.

In some cases, vaccinia viral vectors may elicit a strong antibody response. Thus, while numerous boosts with vaccinia vectors are possible, its repeated use may not be useful in certain instances. However, this sensitivity problem can be minimized by using pox from different genera for boosts. In one example, when the first or initial pox virus vector is vaccinia, the second and subsequent pox virus vectors are selected from the pox viruses from a different genus such as suipox, avipox, capripox or an orthopox immunogenically distinct from vaccinia.

The vaccinia virus genome is known in the art. It is composed of a HIND F13L region, TK region, and an HA region. Recombinant vaccinia virus has been used to incorporate an exogenous gene for expression of the exogenous gene product (see, for example, Perkus et al. Science 229:981-984, 1985; Kaufman et al. Int. J. Cancer 48:900-907, 1991; Moss Science 252:1662, 1991). A gene encoding an antigen of interest, such as an immunogenic Mtb polypeptide, can be incorporated into the HIND F13L region or alternatively incorporated into the TK region of recombinant vaccinia virus vector (or other nonessential regions of the vaccinia virus genome). Baxby and Paoletti (Vaccine 10:8-9, 1992) disclose the construction and use as a vector, of the non-replicating poxvirus, including canarypox virus, fowlpox virus and other avian species. Sutter and Moss (Proc. Nat'l. Acad. Sci U.S.A. 89:10847-10851, 1992) and Sutter et al. (Virology 1994) disclose the construction and use as a vector, the non-replicating recombinant Ankara virus (MVA, modified vaccinia Ankara) in the construction and use of a vector.

Suitable vectors are disclosed, for example, in U.S. Patent No. 6,998,252. In one example, a recombinant poxvirus, such as a recombinant vaccinia virus is synthetically modified by insertion of a chimeric gene containing vaccinia regulatory sequences or DNA sequences functionally equivalent thereto flanking DNA sequences which in nature are not contiguous with the flanking vaccinia regulatory DNA sequences that encode a Mtb polypeptide. The recombinant virus containing such a chimeric gene is effective at expressing the Mtb polypeptide. In one example, the vaccine viral vector comprises (A) a segment comprised of (i) a first DNA sequence encoding a Mtb polypeptide and (ii) a poxvirus promoter, wherein the poxvirus promoter is adjacent to and exerts transcriptional control over the DNA sequence encoding an Mtb polypeptide; and, flanking said segment, (B) DNA from a nonessential region of a poxvirus genome. The viral vector can encode a selectable marker. In one example, the poxvirus includes, for example, a thymidine kinase gene (see U.S. Patent No. 6,998,252).

Viral vectors, such as poxviral vectors, that encode an Mtb polypeptide include at least one expression control element operationally linked to the nucleic acid sequence encoding the Mtb polypeptide. The expression control elements are inserted in the viral vector to control and regulate the expression of the nucleic acid sequence. Examples of expression control elements of use in these vectors includes, but is not limited to, lac system, operator and promoter regions of phage lambda, yeast promoters and promoters derived from polyoma, adenovirus, retrovirus or SV40. Additional operational elements include, but are not limited to, leader sequence, termination codons, polyadenylation signals and any other sequences necessary for the appropriate transcription and subsequent translation of the nucleic acid sequence encoding the Mtb polypeptide in the host system. The expression vector can contain additional elements necessary for the transfer and subsequent replication of the expression vector containing the nucleic acid sequence in the host system. Examples of such elements include, but are not limited to, origins of replication and selectable markers. It will further be understood by one skilled in the art that such vectors are easily constructed using conventional methods (Ausubel et al., (1987) in "Current Protocols in Molecular Biology," John Wiley and Sons, New York, N.Y.) and are commercially available.

Basic techniques for preparing recombinant DNA viruses containing a heterologous DNA sequence encoding the one or more Mtb polypeptides are known in the art. Such techniques involve, for example, homologous recombination between the viral DNA sequences flanking the DNA sequence in a donor plasmid and homologous sequences present in the parental virus (Mackett et al., 1982, Proc. Natl. Acad. Sci. USA 79:7415-7419). In particular, recombinant viral vectors such as a poxyviral vector can be used in delivering the gene. The vector can be constructed for example by steps known in the art, such as steps analogous to the methods for creating synthetic recombinants of the fowlpox virus described in U.S. Pat. No. 5,093,258. Other techniques include using a unique restriction endonuclease site that is naturally present or artificially inserted in the parental viral vector to insert the heterologous DNA.

Generally, a DNA donor vector contains the following elements: (i) a prokaryotic origin of replication, so that the vector may be amplified in a prokaryotic host; (ii) a gene encoding a marker which allows selection of prokaryotic host cells that contain the vector (e.g., a gene encoding antibiotic resistance); (iii) at least one DNA sequence encoding the one or more Mtb polypeptide located adjacent to a transcriptional promoter capable of directing the expression of the sequence; and (iv) DNA sequences homologous to the region of the parent virus genome where the foreign gene(s) will be inserted, flanking the construct of element (iii). Methods for constructing donor plasmids for the introduction of multiple foreign genes into pox virus are described in PCT Publication No. WO 91/19803.

Generally, DNA fragments for construction of the donor vector, including fragments containing transcriptional promoters and fragments containing sequences homologous to the region of the parent virus genome into which foreign DNA sequences are to be inserted, can be obtained from genomic DNA or cloned DNA fragments. The donor plasmids can be mono, di-, or multivalent (i.e., can contain one or more inserted foreign DNA sequences). The donor vector can contain an additional gene that encodes a marker that will allow identification of recombinant viruses containing inserted foreign DNA. Several types of marker genes can be used to permit the identification and isolation of recombinant viruses. These include genes that encode antibiotic or chemical resistance (e.g., see Spyropoulos et al., 1988, J. Virol. 62:1046; Falkner and Moss, 1988, J. Virol. 62:1849; Franke et al., 1985, Mol. Cull. Biol. 5:1918), as well as genes such as the E. coli lacZ gene, that permit identification of recombinant viral plaques by colorimetric assay (Panicali et al., 1986, Gene 47:193-199).

The DNA gene sequence to be inserted into the virus can be placed into a donor plasmid, such as an E. coli or a Salmonella plasmid construct, into which DNA homologous to a section of DNA such as that of the insertion site of the poxvirus where the DNA is to be inserted has been inserted. Separately the DNA gene sequence to be inserted is ligated to a promoter. The promoter-gene linkage is positioned in the plasmid construct so that the promoter-gene linkage is flanked on both ends by DNA homologous to a DNA sequence flanking a region of pox DNA that is the desired insertion region. With a parental pox viral vector, a pox promoter is used. The resulting plasmid construct is then amplified by growth within E. coli bacteria and isolated. Next, the isolated plasmid containing the DNA gene sequence to be inserted is transfected into a cell culture, for example chick embryo fibroblasts, along with the parental virus, for example poxvirus. Recombination between homologous pox DNA in the plasmid and the viral genome respectively results in a recombinant poxvirus modified by the presence of the promoter-gene construct in its genome, at a site that does not affect virus viability.

As noted above, the DNA sequence is inserted into a region (insertion region) in the virus that does not affect virus viability of the resultant recombinant virus. One of skill in the art can readily identify such regions in a virus by, for example, randomly testing segments of virus DNA for regions that allow recombinant formation without seriously affecting virus viability of the recombinant. One region that can readily be used and is present in many viruses is the thymidine kinase (TK) gene. The TK gene has been found in all pox virus genomes examined, including leporipoxvirus (Upton et al., 1986, J. Virology 60:920); shope fibromavirus; capripoxvirus (Gershon et al., 1989, J. Gen, Virol. 70:525) Kenya sheep 1; orthopoxvirus (Weir et al., 1983, J. Virol 46:530) vaccinia (Esposito et al., 1984, Virology 135:561); monkeypox and variola virus (Hruby et al., 1983, PNAS 80:3411) vaccinia (Kilpatrick et al., 1985, Virology 143:399); Yaba monkey tumor virus; avipoxvirus (Binns et al., 1988, J. Gen. Virol. 69:1275); fowipox; (Boyle et al., 1987, Virology 156:355); fowlpox (Schnitzlein et al., 1988, J. Virological Methods 20:341); fowlpox, quailpox; entomopox (Lytvyn et al., 1992, J. Gen. Virol, 73:3235-3240). In vaccinia, in addition to the TK region, other insertion regions include, for example, the HindIII M fragment. In fowlpox, in addition to the TK region, other insertion regions include, for example, the BamHI J fragment (Jenkins et al., 1991, AIDS Research and Human Retroviruses 7:991-998) the EcoRI-HindIII fragment, EcoRV-HindIII fragment, BamHI fragment and the HindIII fragment set forth in EPO Application No. 0 308220 A1 (see also Calvert et al., 1993, J. Virol. 67:3069-3076; Taylor et al., 1988, Vaccine 6:497-503; Spehner et al., 1990; Boursnell et al., 1990, J. Gen. Virol. 71:621-628).

In swinepox, insertion sites include the thymidine kinase gene region. In addition to the requirement that the gene be inserted into an insertion region, successful expression of the inserted gene by the modified poxvirus requires the presence of a promoter operably linked to the desired gene. Generally, the promoter is placed so that it is located upstream from the gene to be expressed. Promoters are well known in the art and can readily be selected depending on the host and the cell type you wish to target. In one example, in poxviruses, pox viral promoters are used; such as the vaccinia 7.5K, 40K or fowlpox promoters such as FPV C1A. Enhancer elements can also be used in combination to increase the level of expression. Furthermore, inducible promoters can be utilized.

Homologous recombination between donor plasmid DNA and viral DNA in an infected cell can result in the formation of recombinant viruses that incorporate the desired elements. Appropriate host cells for *in vivo* recombination are generally eukaryotic cells that can be infected by the virus and transfected by the plasmid vector. Examples of such cells suitable for use with a pox virus are chick embryo fibroblasts, HuTK143 (human) cells, and CV-1 and BSC-40 (both monkey kidney) cells. Infection of cells with pox virus and transfection of these cells with plasmid vectors is accomplished by techniques standard in the art (see U.S. Pat. No. 4,603,112 and PCT Publication No. WO 89/03429).

Following *in vivo* recombination, recombinant viral progeny can be identified by one of several techniques. For example, if the DNA donor vector is designed to insert foreign genes into the parent virus thymidine kinase (TK) gene, viruses containing integrated DNA will be TK- and can be selected on this basis (Mackett et al., 1982, Proc. Natl. Acad. Sci. USA 79:7415). Alternatively, co-integration of a gene encoding a marker or indicator gene with the foreign gene(s) of interest, as described above, can be used to identify recombinant progeny. One specific non-limiting example of an indicator gene is the E. coli lacZ gene. Recombinant viruses expressing beta-galactosidase can be selected using a chromogenic substrate for the enzyme (Panicali et al., 1986, Gene 47:193). Once a recombinant virus has been identified, a variety of well-known methods can be used to assay the expression of the Mtb sequence encoded by the inserted DNA fragment. These methods include black plaque assay (an in situ enzyme immunoassay performed on viral plaques), Western blot analysis, radioimmunoprecipitation (RIPA), and enzyme immunoassay (EIA).

This disclosure encompasses a recombinant virus comprising more than one antigen of interest for the purpose of having a multivalent vaccine. For example, the recombinant virus may comprise the virus genome or portions thereof, the nucleic acid sequence encoding the Mtb polypeptide and a nucleic acid sequence encoding a hepatitis B surface antigen or any other carrier protein.

In one embodiment, a composition is provided that includes a recombinant virus comprising a vaccinia virus genome or portions thereof, the nucleic acid sequence encoding an Mtb polypeptide and a recombinant virus comprising the nucleic acid sequence encoding the immunostimulatory molecule, B 7.1 alone or in combination with the nucleic acid sequence encoding the immunostimulatory molecule, B7-2, or a recombinant virus containing both the genes for an Mtb polypeptide and an immunostimulatory molecule. This disclosure also encompasses a recombinant virus comprising the Mtb polypeptide that is administered with a second recombinant virus comprising the virus genome or portion thereof, and one or more nucleic acid sequences encoding one or more B7 molecules, such as a recombinant vaccinia virus expressing B7-1 and/or B7-2.

Thus, in one example, recombinant virus is disclosed that is a recombinant vaccinia virus containing B7-1 and a recombinant vaccinia virus containing B7-2 (designated rV-B7-1 and rV-B7-2, respectively); the composition can include rV-B7-1 and/or rV-B7-2 in combination with an immunogenic Mtb polypeptide

The B7 molecule includes but is not limited to B7-1, B7-2 and analogs thereby. The B7 gene may be cloned from mammalian sources, including but not limited to mammalian tissues, genomic libraries or cDNA libraries, such as from murine or human sources. Without being bound by theory, co-stimulatory molecules of the B7 family (namely B7-1, B7-2, and possibly B7-3) are believed to be members of the immunoglobulin gene superfamily. These molecules are present on macrophages, dendritic cells, monocytes (antigen presenting cells (APCs)). Significant amplification of the immune response against a given antigen generally does not occur without co-stimulation (June et al. (Immunology Today 15:321-331, 1994); Chen et al., (Immunology Today 14:483-486); Townsend et al. (Science 259:368-370)). Freeman et al. (J. Immunol. 143:2714-2722, 1989) report cloning and sequencing of B7-1 gene. Azuma et al. (Nature 366:76-79, 1993) report cloning and sequencing B7-2 gene. Thus, in one embodiment the B7-1 gene or the B7-2 genes are administered in conjunction with the Mtb polypeptide The insertion of nucleic acids encoding B7-1 and B7-2 into vaccinia virus has been disclosed (see for example, U.S. Patent No. 6,893,869, this U.S. Patent also discloses the use of a nucleic acid encoding IL-2 in a vaccinia virus). Several vectors including IL-2, B7-1 and B7-2 have been deposited with the American Type Culture Collection (ATCC) on Oct. 3,1994 under the terms of the Budapest Treaty (for example, rV-CEA/ₙIL-2 (ATCC Designation VR 2480), rV-ₘB7-2 (ATCC Designation VR 2482); and rV-ₘB7-1 (ATCC Designation VR 2483)).

DNA sequences encoding a Mtb polypeptide can be expressed *in vitro* by DNA transfer into a suitable host cell. The cell may be prokaryotic or eukaryotic. The term also includes any progeny of the subject host cell. It is understood that all progeny may not be identical to the parental cell since there may be mutations that occur during replication. Methods of stable transfer, meaning that the foreign DNA is continuously maintained in the host, are known in the art

As noted above, a polynucleotide sequence encoding a Mtb polypeptide can be operatively linked to expression control sequences, An expression control sequence operatively linked to a coding sequence is ligated such that expression of the coding sequence is achieved under conditions compatible with the expression control sequences. The expression control sequences include, but are not limited to, appropriate promoters, enhancers, transcription terminators, a start codon (i.e., ATG) in front of a protein-encoding gene, splicing signal for introns, maintenance of the correct reading frame of that gene to permit proper translation of mRNA, and stop codons.

Hosts cells can include microbial, yeast, insect and mammalian host cells Methods of expressing DNA sequences having eukaryotic or vital sequences in prokaryotes are well known in the art. Non limiting examples of suitable host cells include bacteria, archea, insect, fungi (for example, yeast), mycobacterium (such as *M. smegmatis*), plant, and animal cells (for example, mammalian cells, such as human). Exemplary cells of use include *Escherichia coli, Bacillus subtilis, Saccharomyces cerevisiae, Salmonella typhimurium,* SF9 cells, C129 cells, 293 cells, *Neurospora,* and immortalized mammalian myeloid and lymphoid cell lines. Techniques for the propagation of mammalian cells in culture are well-known (see, Jakoby and Pastan (eds), 1979, Cell Culture. Methods in Enzymology, volume 58, Academic Press, Inc., Harcourt Brace Jovanovich, N.Y.). Examples of commonly used mammalian host cell lines are VERO and HeLa cells, CHO cells, and WI38. BHK, and COS cell lines, although cell lines may be used, such as cells designed to provide higher expression desirable glycosylation patterns, or other features. As discussed above, techniques for the transformation of yeast cells, such as polyethylene glycol transformation, protoplast transformation and gene guns are also known in the art (see Gietz and Woods Methods in Enzymology 350: 87-96,2002).

Transformation of a host cell with recombinant DNA can be carried out by conventional techniques as are well known to those skilled in the art. Where the host is prokaryotic, such as, but not limited to, *E. coli,* competent cells which are capable of DNA uptake can be prepared from cells harvested after exponential growth phase and subsequently treated by the CaCl₂ method using procedures well known in the art. Alternatively, MgCl₂ or RbCl can be used. Transformation can also be performed after forming a protoplast of the host cell if desired, or by electroporation.

When the host is a eukaryote, such methods of transfection of DNA as calcium phosphate coprecipitates, conventional mechanical procedures such as microinjection, electroporation, insertion of a plasmid encased in liposomes, or virus vectors can be used. Eukaryotic cells can also be co-transformed with polynucleotide sequences encoding a Mtb polypeptide, and a second foreign DNA molecule encoding a selectable phenotype, such as the herpes simplex thymidine kinase gene. Another method is to use a eukaryotic viral vector, such as simian virus 40 (SV40) or bovine papilloma virus, to transiently infect or transform eukaryotic cells and express the protein (see for example, Eukaryotic Viral Vectors, Cold Spring Harbor Laboratory, Gluzman ed., 1982).

### Therapeutic Methods and Pharmaceutical Compositions

The Mtb polypeptides disclosed herein, or nucleic acids encoding the Mtb polypeptides, can be used to generate an immune response in a subject. In several examples, the subject is infected with Mtb or is at risk of being infected with Mtb. Thus, in several embodiments, the methods include administering to a subject a therapeutically effective amount of one or more of the Mtb polypeptides disclosed herein (or polynucleotides encoding these polypeptides), in order to generate an immune response, such as, but not limited to, a protective immune response.

In exemplary applications, compositions are administered to a subject having in an amount sufficient to produce an immune response to Mtb. These Mt polypeptides, or polynucleotides encoding these polypeptides are of use to prevent an infection with Mtb, prevent progression to disease in a subject having a latent Mtb infection, or to treat tuberculosis in a subject infected with Mtb. In several examples, administration of a therapeutically effective amount of a composition including one or move of the Mtb polypeptides disclosed herein (or polynucleotides encoding these polypeptides) induces a sufficient immune response to decrease a symptom of a disease due to Mtb infection, to prevent the development of one or more symptoms of tuberculosis, or to prevent infection with Mtb.

In some examples, the compositions are of use in preventing a future infection with Mtb. Thus, a therapeutically effective amount of the composition is administered to a subject at risk of becoming infective with Mtb. The composition prevents the development of tuberculosis, such as latent or active tuberculosis in the subject upon subsequent exposure to Mtb.

In additional examples, the compositions are administered to a subject with a latent Mtb infection, and prevent the development of symptoms of tuberculosis. Thus the compositions are of use in treating a subject with latent tuberculosis, such that the subject does not develop active tuberculosis.

Amounts effective for these uses will depend upon the severity of the disease, the general state of the patient's health, and the robustness of the patient's immune system. In one example, a therapeutically effective amount of the compound is that which provides either subjective relief of a symptom(s) or an objectively identifiable improvement as noted by the clinician or other qualified observer. In other examples, a therapeutically effective amount is an amount sufficient to prevent an infection with Mtb in a subject upon subsequent exposure of the subject to Mtb. In additional examples, a therapeutically effective amount is an amount sufficient to prevent development of symptom in a subject infected with Mtb.

A Mtb polypeptide can be administered by any means known to one of skill in the art (see Banga, A., "Parenteral Controlled Delivery of Therapeutic Peptides and Proteins," in Therapeutic Peptides and Proteins, Technomic Publishing Co., Inc., Lancaster, PA, 1995) either locally or systemically, such as by intramuscular injection, subcutaneous injection, intraperitoneal infection, intravenous injection, oral administration, nasal administration, transdermal administration or even anal administration. In one embodiment, administration is by oral, subcutaneous injection or intramuscular injection. To extend the time during which the peptide or protein is available to stimulate a response, the peptide or protein can be provided as an implant, an oily injection, or as a particulate system. The particulate system can be a microparticle, a microcapsule, a microsphere, a nanocapsule, or similar particle. (*see*, e.g., Banga, *supra*). A particulate carrier based on a synthetic polymer has been shown to act as an adjuvant to enhance the immune response, in addition to providing a controlled release Aluminum salts can also be used as adjuvants to produce an immune response.

In one specific, non-limiting example, the Mtb polypeptide is administered in a manner to direct the immune response to a cellular response (that is, a cytotoxic T lymphocyte (CTL) response), rather than a humoral (antibody) response.

Optionally, one or more cytokines, such as IL-2, IL-6, IL-12; RANTES, GM-CSF, TNF-α, or IFN-γ, one or more growth factors, such as GM-CSF or G-CSF; one or more molecules such as OX-40L or 41 BBL, or combinations of these molecules, can be used as biological adjuvants (see, for example, Salgaller et al., 1998, J. Surg Oncol. 68(2):122-38; Lotze et al 2000, Cancer J Sci. Am. 6(Suppl 1):S61-6, Cao et al., 1998, Stem Cells 16(Suppl 1):251-60; Kuiper et al., 2000, Adv. Exp. Med. Biol, 465:381-90). These molecules can be administered systemically (or locally) to the host. In several examples, IL-2, RANTES, GM-CSF, INF-α, IFN-γ, G-CSF, LFA-3, CD72, B7-1, B7-2, B7-1 B7-2, OX-40L, 41 BBL and ICAM-1 are administered

A number of means for inducing cellular responses, both *in vitro* and *in vivo*, are known. Lipids have been identified as agents capable of assisting in priming CTL *in vivo* against various antigens. For example, as described in U.S. Patent No. 5,662,907, palmitic acid residues can be attached to the alpha and epsilon amino groups of a lysine residue and then linked (for example, via one or more linking residues, such as glycine, glycine-glycine, serine, serine-serine, or the like) to an immunogenic peptide. The lipidated peptide can then be injected directly in a micellar form, incorporated in a liposome, or emulsified in an adjuvant As another example, *E. coli* lipoproteins, such as tripalmitoyl-S-glycerylcysteinlyseryl-serine can be used to prime tumor specific CTL when covalently attached to an appropriate peptide (*see*, Deres et al., Nature 342:561, 1989). Further, as the induction of neutralizing antibodies can also be primed with the same molecule conjugated to a peptide which displays an appropriate epitope, two compositions can be combined to elicit both humoral and cell-mediated responses where that is deemed desirable.

A pharmaceutical composition including a Mtb polypeptide is thus provided These compositions are of use to promote an immune response to Mtb. In one embodiment, the Mtb polypeptide is mixed with an adjuvant containing two or more of a stabilizing detergent, a micelle-forming agent, and an oil. Suitable stabilizing detergents, micelle-forming agents, and oils are detailed in U.S. Patent No. 5,585,103; U.S. Patent No. 5,709,860; U.S Patent No. 5,270,202; and U.S. Patent No. 5,695,770. A stabilizing detergent is any detergent that allows the components of the emulsion to remain as a stable emulsion. Such detergents include polysorbate, 80 (TWEEN) (Sorbitan-mono-9-octadecenoate-poly(oxy-1,2-ethanediyl; manufactured by ICI Americas, Wilmington, DE), TWEEN 40™, TWEEN 20^{™}, TWEEN 60^{™}, ZWITTERGENT^{™} 3-12, TEEPOL HB7^{™}, and SPAN 85^{™}. These detergents are usually provided in an amount of approximately 0.05 to 0.5%, such as at about 0.2%. A micelle forming agent is an agent which is able to stabilize the emulsion formed with the other components such that a micelle-like structure is formed. Such agents generally cause some irritation at the site of injection in order to recruit macrophages to enhance the cellular response. Examples of such agents include polymer surfactants described by BASF Wyandotte publications, e.g., Schmolka, J. Am. Oil. Chem. Soc. 54:110, 1977, and Hunter et al., J. Immunol 129:1244, 1981, PLURONIC^{™} L62LF, L101, and L64, PEG1000, and TETRONIC^{™} 1501, 150R1, 701, 901, 1301, and 130R1, The chemical structures of such agents are well known in the art. In one embodiment, the agent is chosen to have a hydrophile-lipophile balance (HLB) of between 0 and 2, as defined by Hunter and Bennett, J. Immun. 133:3167, 1984. The agent can be provided in an effective amount, for example between 0.5 and 10%, or in an amount between 1.25 and 5%.

The oil included in the composition is chosen to promote the retention of the antigen in oil-in water emulsion, such as to provide a vehicle for the desired antigen, and preferably has a melting temperature of less than 65°C such that emulsion is formed either at room temperature (about 20°C to 25°C), or once the temperature of the emulsion is brought down to room temperature. Examples of such oils include squalene, Squalane, EICOSANE^{™}, tetratetracontane, glycerol, and peanut oil or other vegetable oils. In one specific, non-limiting example, the oil is provided in an amount between 1 and 10%, or between 2.5 and 5%. The oil should be both biodegradable and biocompatible so that the body can break down the oil over time, and so that no adverse affects, such as granulomas, are evident upon use of the oil.

In one embodiment, the adjuvant is a mixture of stabilizing detergents, micelle-forming agent, and oil available under the name PROVAX® (IDEC Pharmaceuticals, San Diego, CA). An adjuvant can also be an immunostimulatory nucleic acid, such as a nucleic acid including, a CpG motif, or a biological adjuvant (see above)

Controlled release parenteral formulations can be made as implants, oily injections, or as particulate systems. For a broad overview of protein delivery systems, see Banga, Therapeutic Peptides and Proteins: Formulation, Processing, and Delivery Systems, Technomic Publishing Company, Inc., Lancaster, PA, 1995. Particulate systems include microspheres, microparticles, microcapsules, nanocapsules, nanospheres, and nanoparticles. Microcapsules contain the therapeutic protein as a central core. In microspheres, the therapeutic agent is dispersed throughout the particle. Particles, microspheres, and microcapsules smaller than about 1 µm are generally referred to as nanoparticles, nanospheres, and nanocapsules, respectively. Capillaries have a diameter of approximately 5 µm so that only nanoparticles are administered intravenously. Microparticles are typically around 100 µm in diameter and are administered subcutaneously or intramuscularly (see Kreuter, Colloidal Drug Delivery Systems, J. Kreuter, ed., Marcel Dekker, Inc., New York, NY, pp. 219-342, 1994; Tice & Tabibi, Treatise on Controlled Drug Delivery, A. Kydonieus, ed., Marcel Dekker, Inc. New York, NY, pp. 315-339, 1992).

Polymers can be used for ion-controlled release.. Various degradable and nondegradable polymeric matrices for use in controlled drug delivery are known in the art (Langer, Accounts Chem, Res. 26:537, 1993). For example, the block copolymer, polaxamer 407 exists as a viscous yet mobile liquid at low temperatures but forms a semisolid gel at body temperature. It has shown to be an effective vehicle for formulation and sustained delivery of recombinant interleukin-2 and urease (Johnston et al., Pharm. Res. 9:425, 1992; and Pec, J. Parent. Sci. Tech 44(2):58, 1990). Alternatively, hydroxyapatite has been used as a microcarrier for controlled release of proteins (Ijntema et al Int. J. Pharm. 112:215, 1994). In yet another aspect, liposomes are used for controlled release as well as drug targeting of the lipid-capsulated drug (Betageri et al., Liposome Drug Delivery Systems, Technomic Publishing Co., Inc., Lancaster, PA, 1993). Numerous additional systems for controlled delivery of therapeutic proteins are known (e.g., U.S. Patent No. 5,055,303; U.S. Patent No. 5,188,837; U.S. Patent No. 4,235,871; U.S. Patent No. 4,501,728; U.S. Patent No. 4,837,028; U.S. Patent No. 4,957,735; and U.S Patent No. 5,019,369; U.S. Patent No. 5,055,303; U.S. Patent No. 5,514,670; U.S. Patent No. 5,413,797; U.S. Patent No. 5,268,164; U.S. Patent No. 5,004,697; U.S. Patent No. 4,902,505; U.S. Patent No. 5,506,206; U.S. Patent No. 5,271,961; U.S. Patent No. 5,254,342; and U.S. Patent No. 5,534,496).

In another embodiment, a pharmaceutical composition includes a nucleic acid encoding an Mtb polypeptide. A therapeutically effective amount of the Mtb polynucleotide can be administered to a subject in order to generate an immune response.

Optionally, one or more cytokines, such as IL-2, IL-6, IL-12, RANTES, GM-CSF, TNF-α, or IFN-γ, one or more growth factors, such as GM-CSF or G-CSF, one or more costimulatory molecules, such as ICAM-1, LFA-3, CD72, B7-1, B7-2, or other B7 related molecules; one or more molecules such as OX-40L or 41 BBL, or combinations of these molecules, can be used as biological adjuvants (see, for example, Salgaller et al., 1998, J. Surg. Oncol. 68(2):1.22-38; Lotze et al., 2000; Cancer J Sci. Am. 6(Suppl 1):S61-6; Cao et al., 1998, Stem Cells 16(Suppl 1):251-60; Kuiper et al., 2000, Adv. Exp. Med.. Biol. 465:381-90). These molecules can be administered systemically to the host. It should be noted that these molecules can be co-administered via insertion of a nucleic acid encoding the molecules into a vector, for example, a recombinant pox vector (see, for example, U.S. Pat. No. 6,045,802). In various embodiments, the nucleic acid encoding the biological adjuvant can be cloned into same vector as the Mtb polypeptide coding sequence, or the nucleic acid can be cloned into one or more separate vectors for co-administration. In addition, nonspecific immunomodulating factors such as Bacillus Cahnette-Guerin (BCG) and levamisole can be co-administered.

One approach to administration of nucleic acids is direct immunization with plasmid DNA, such as with a mammalian expression plasmid. As described above, the nucleotide sequence encoding an Mtb polypeptide can be placed under the control of a promoter to increase expression of the molecule.

Immunization by nucleic acid constructs is well known in the art and taught, for example, in U.S. Patent No. 5,643,578 (which describes methods of immunizing vertebrates by introducing DNA encoding a desired antigen to elicit a cell-mediated or a humoral response), and U.S. Patent No. 5,593,972 and U.S. Patent No. 5,817,637 (which describe operably linking a nucleic acid sequence encoding an antigen to regulatory sequences enabling expression). U.S. Patent No. 5,880,103 describes several methods of delivery of nucleic acids encoding immunogenic peptides or other antigens to an organism. The methods include liposomal delivery of the nucleic acids (or of the synthetic peptides themselves), and immune-stimulating constructs, or ISCOMS™, negatively charged cage-like structures of 30. 40 nm in size formed spontaneously on mixing cholesterol and Quil A™ (saponin). Protective immunity has been generated in a variety of experimental models of infection, including toxoplasmosis and Epstein-Barr virus-induced tumors, using ISCOMS™ as the delivery vehicle for antigens (Mowat and Donachie, Immunol Today 12:383, 1991). Doses of antigen as low as 1 µg encapsulated in ISCOMS™ have been found to produce Class I mediated CTL responses (Takahashi et al., Nature 344:873, 1990).

In another approach to using nucleic acids for immunization, an Mtb polypeptide can also be expressed by attenuated viral hosts or vectors or bacterial vectors. Recombinant vaccinia virus, adeno-associated virus (AAV), herpes virus, retrovirus, or other viral vectors can be used to express the peptide or protein, thereby eliciting a CTL response For example, vaccinia vectors and methods useful in immunization protocols are described in U.S. Patent No. 4,722,848. BCG (Bacillus Calmette Guerin) provides another vector for expression of the peptides (see Stover, Nature 351:456-460, 1991).

When a viral vector is utilized, it is desirable to provide the recipient with a dosage of each recombinant virus in the composition in the range of from about 10⁵ to about 10¹⁰ plaque forming units/mg mammal, although a lower or higher dose can be administered. The composition of recombinant viral vectors can be introduced into a mammal (1) prior to any evidence of an infection with Mtb; (2) to prevent development of tuberculosis in an individual infected with tuberculosis; or (3) to decrease a symptom of tuberculosis in a mammal infected with Mtb. Examples of methods for administering the composition into mammals include, but are not limited to, exposure of cells to the recombinant virus *ex vivo*, or injection of the composition into the affected tissue or intravenous, subcutaneous, intradermal or intramuscular administration of the virus. Alternatively the recombinant viral vector or combination of recombinant viral vectors may be administered locally in a pharmaceutically acceptable carrier. Generally, the quantity of recombinant viral vector, carrying the nucleic acid sequence of one or more Mtb polypeptides to be administered is based on the titer of virus particles. An exemplary range of the immunogen to be administered is 10⁵ to 10¹⁰ virus particles per mammal, such as a human.

In the embodiment where a combination of a first recombinant viral vector carrying a nucleic acid sequence of one or more Mtb polypeptide and a second recombinant viral vector carrying the nucleic acid sequence of one or more immunostimulatory molecules is used, the mammal can be immunized with different ratios of the first and second recombinant viral vector. In one embodiment the ratio of the first vector to the second vector is about 1:1, or about 1:3, or about 1:5 Optimal ratios of the first vector to the second vector may easily be titered using the methods known in the art (see, for example, U.S. Patent No. 6,893,869).

In one embodiment the recombinant viruses have been constructed to express cytokines (such as TNF-α, IL-6, GM-CSF, and IL-2), and co-stimulatory and accessory molecules (B7-1, B7-2) alone and in a variety of combinations Simultaneous production of an immunostimulatory molecule and the Mtb polypeptide enhances the generation of specific effectors. Without being bound by theory, dependent upon the specific immunostimulatory molecules, different mechanisms might be responsible for the enhanced immunogenicity: augmentation of help signal (IL-2), recruitment of professional APC (GM-CSF), increase in CTL frequency (IL-2), effect on antigen processing pathway and MHC expression (IFNγ and TNFα) and the like. For example, IL-2, IL-6, interferon, tumor necrosis factor, or a nucleic acid encoding these molecules, can be administered in conjunction with a Mtb immunogenic polypeptide, or a nucleic acid encoding an Mtb polypeptide. The co-expression of a Mtb polypeptide together with at least one immunostimulatory molecule can be effective in an animal model to show anti-tumore effects.

In one embodiment, a nucleic acid encoding an Mtb polypeptide is introduced directly into cells. For example, the nucleic acid can be loaded onto gold microspheres by standard methods and introduced into the skin by a device such an Bio-Rad's Helios™ Gene Gun. The nucleic acids can be "naked," consisting of plasmids under control of a strong promoter" Typically, the DNA is injected into muscle, although it can also be injected directly into other sites, including tissues in proximity to metastases, Dosages for injection are usually around 0.5 µg/kg to about 50 mg/kg, and typically are about 0.005 mg/kg to about 5 mg/kg (see, for example, U.S. Patent No. 5,589,466).

In one specific, non-limiting example, a pharmaceutical composition for intrayenous administration would include about 0.1 µg to 10 mg of immunogenic Mtb polypeptide per patient per day. Dosages from 0.1 up to about 100 mg per patient per day can be used; particularly if the agent is administered to a secluded site and not into the cuculatory or lymph system, such as into a body cavity or into a lumen of an organ. Actual methods for preparing administrable compositions will be known or apparent to those skilled in the art and are described in more detail in such publications as Remingtons Pharmaceutical Sciences, 19th Ed., Mack Publishing Company, Easton, Pennsylvania, 1995.

Single or multiple administrations of the compositions are administered depending on the dosage and frequency as required and tolerated by the subject. In one embodiment, the dosage is administered once as a bolus, but in another embodiment can be applied periodically until a therapeutic result is achieved In one embodiment, the dose is sufficient to treat or ameliorate symptoms or signs of' tuberculosis without producing unacceptable toxicity to the subject. In another embodiment, the dose is sufficient to prevent infection with Mtb upon subsequent exposure to Mtb In a fiuther embodiment, the dose is sufficient to prevent a symptom of tuberculosis in a subject with a latent Mtb infection Systemic or local administration can be utilized.

In another method, antigen presenting cells (APCs), such as dendritic cells, are isolated from a subject of interest and pulsed or co-incubated with peptides comprising an Mtb polypeptide *in vitro*. In one specific, non-limiting example, the antigen presenting cells are autologous cells isolated from the subject of interest. A therapeutically effective amount of the antigen presenting cells is administered (re-introduced) to the subject of interest.

The Mtb polypeptide can be delivered to the dendritic cells or to dendritic cell precursors via any method known in the art, including, but not limited to, pulsing dendritic cells directly with antigen, or utilizing a broad variety of antigen delivery vehicles, such as, for example, liposomes, or other vectors known to deliver antigen to cells. In one specific, non-limiting example an antigenic formulation includes about 0.1 µg to about 1,000 µg, or about 1 to about 100 µg of a selected Mtb polypeptide. The Mtb polypeptide can also be administered with agents that promote dendritic cell maturation. Specific, non limiting examples of agents of use are interleukin-4 (IL-4) and granulocyte/macrophage colony stimulating factor (GM-CSF), or flt-3 ligand (flt-3L). The preparation can also contain buffers, excipients, and preservatives, amongst other ingredients,

In one embodiment, mature antigen presenting cells are generated to present the immunogenic Mtb polypeptide. These dendritic cells are then administered alone to a subject infected with Mtb, or at risk for infection with Mtb. In another embodiment, the mature dendritic cells are administered in conjunction with an antiviral agent.

Alternatively, the APCs are used to sensitize CD8 cells, such as periphetal blood lymphocytes (PBLs). The PBLs can be from the same subject (autologous) that is to be treated. Alternatively, the PBLs, can be heterologous. However, they should at least be MHC Class-I restricted to the HLA types the subject possesses An effective amount of the sensitized cells are then administered to the subject.

Peripheral blood mononuclear cells (PBMCs) can be used as the responder cell source of cytotoxic T lymphocyte (CTL) precursors. The appropriate antigen-presenting cells are incubated with peptide, after which the peptide-loaded antigen-presenting cells are then incubated with the responder cell population under optimized culture conditions Positive CTL activation can be determined by assaying the culture for the presence of CTLs that kill radio-labeled target cells, both specific peptide-pulsed targets as well as target cells expressing endogenously processed forms of the antigen from which the peptide sequence was derived.

Alternatively, a CD8⁺ T cell clone that recognizes the Mtb polypeptide can be isolated from a subject of interest. This CD8⁺ T cell clone can be expanded *in vitro,* using methods known in the art. A therapeutically effective amount of the CD8⁺ T cells is then administered to the subject of interest.

Thus, cells can be administered to a subject to treat or an Mtb infection, such as to decrease a symptom of an Mtb infection. In these applications, a therapeutically effective amount of activated antigen presenting cells, or activated lymphocytes, are administered to a subject in an amount sufficient to raise an immune response to Mtb.

In supplemental methods, any therapeutic regimen is augmented by administering a cytokine, such as interleukin (IL)-2, IL-3, IL-6, IL-10, IL-12, IL-15, GM-CSF, interferons. In further methods, an additional antiviral agent is administered to the subject.

The disclosure is illustrated by the following non-limiting Examples:

### EXAMPLES

For many infections, the repertoire of the CD8 response is shaped by the entry of antigen into the MHC-I processing pathway, binding of peptides and/or non-peptide antigens to MHC-I molecules, and recognition of these structures by T cells Ultimately, a relatively limited subset of pathogen-specific T cells emerge. While a number of commonly recognized CD4 Mtb antigens have been described (Reed et al., Microbes Infect 7:922-931, 2005) (ESAT-6, CFP10, Ag85, etc.), surprisingly little is known about common Mtb antigens recognized by human CD8⁺ T cells. The majority of CD8 epitopes that have been identified were defined by testing of Mtb peptides selected for high affinity binding to MHC Class Ia molecules (HLA-A2 in most cases (see, for example, Lalvani, Microbes Infect 7:922-931; 1998)). In almost all of these, however, the *ex vivo* frequency of these T cells in Mtb-infected individuals is low or undetectable, suggesting that these specificities may not represent immunodominant responses. In contrast, in the limited cases in which T cells have been used to define epitopes contained in selected Mtb antigens, high *ex vivo* frequencies have been demonsnated (see Lewinsohn et al., Am J Respir Crit Care Med 166:843-848,2002), suggesting, that a T cell-centered approach can identify immunodominant epitopes. Moreover, CD8 T cell responses to some Mtb antigens which represent good CD4 antigens (CFP10, ESAT-6, Ag85, and Mtb39) have been detected at high frequency in persons infected with Mtb. Therefore, a limited library of overlapping synthetic peptides representing several known CD4 Mtb antigens was used to determine the magnitude of the CD8 response to these antigens in persons with active tuberculosis (TB) and latent tuberculosis infection (LTBI) as well as uninfected subjects. Furthermore, a panel of Mtb-specific CD8⁺ T cell clones was utilized to define minimal epitopes recognized within these antigens and determined the contribution of these novel epitopes to the *ex vivo* Mtb-specific CD8 response.

### Example 1 :

### Materials and Methods

*Human subjects.* Uninfected individuals were defined as healthy individuals with a negative tuberculin skin test (TST) and no know risk factors for infection with Mtb. Individuals with LTBI were defined as healthy persons with a positive TST and no symptoms and signs of active TB. In all active IB cases, pulmonary TB was diagnosed by the TB Controller of the county and confirmed by positive sputum culture for *Mycobacterium tuberculosis*. Peripheral blood mononuclear cells (PBMC) were isolated from whole blood obtained by venipuncture or apheresis.

*Media and Reagents.* Culture medium consisted of RPMI 1640 supplemented with 10% Fetal Bovine Sera (FBS; Bio Whittaker), 5 X 10⁻⁵ M 2 ME (Sigma-Aldrich), and 2 mM glutamine (GIBCO BRL). For the growth and assay of Mtb-reactive T cell clones, RPMI 1640 was supplemented with 10% human serum Mtb strain H37Rv was obtained from the American Type Culture Collection (Rockville, MD) and prepared as previously described (Lewinsohn et al, J Immunol 165:925-930, 2000). Peptides were synthesized by Genemed Synthesis, Inc, (San Francisco, CA). Synthetic peptide pools consisted of 15-mers overlapping by 11 amino acids (aa) representing Mtb proteins demonstrated to be potent CD4 antigens. Peptide pools representing CFP-10 (Berthet et al., Microbiology 144:3195-3203, 1998; Dillon et al., J Glin Microbiol 38:3285-3290, 2000), ESAT-6 (Sorenson et al., Infect Immun 63:1710-1717, 1995), Mtb39a (two pools, A &B; reference) (Dillon et al, Infect Immun 67:2941-2950, 1999), Mtb8.4 (Coler et al., J Immunol 161:2356-2364, 1998), Mtb 9.9 (Alderson et al., J Exp Med 191:551-560, 2000), (Coler et al., J Immunol 161:2356-2364, 1998), Mtb 9.9 (Alderson et al.; J Exp Med 191:551-560, 2000), EsxG (Rosenkrands et al., Electrophoresis 21:3740-3756, 2002), 19kDa antigen (Collins et al. J Gen Microbiol 136:1429-1436, 1990), antigen 85b ( Borremans et al., Infect Immun 57:3123-3130, 1989) (two pools, A & B, reference) were synthesized. Peptides were resuspended in DMSO and up to 50 peptides were combined into one pool such that each peptide in the pool was at a concentration of 1mg/ml. Peptide pools were stored at -80 °C.

*Cell Lines and T Cell Clones.* EBV-fransformed B cell lines, LCL, were either generated using supernatants from the cell line 9B5-8 (American Type Culture Collection) or obtained from the National Marrow Donor Program (NMDP; Minneapolis, MN). LCL were maintained by continuous passage as previously described (Heinzel et al., J Exp Med 196:1473-1481,2002). Mtb-specific T cell clones were isolated from individuals with LTBI or active tuberculosis, using Mtb-infected DCs as APCs and limiting dilution cloning methodology as previously described (Lewinsohn et al., J Immunol 165:925-930, 2000). Briefly, CD8⁺ T cells were isolated from PBMC using negative selection using CD4 antibody-coated beads and then positive selection using CD8 antibody-coated magnetic beads per the manufacturer's instructions (Miltenyi Biotec, Auburn CA) or via flow cytometry, In this case, CD4-PE (BD Biosciences cat # 555347) negative, CD8-APC (BD Biosciences, cat# 555369) positive cells (purity >99%) were sorted on a Becton Dickenson LSR II. T cells were seeded at various concentrations in the presence of a 1 X 10⁵ irradiated autologous Mtb-infected DC, generated as described below, and rIL-2 (5 ng/ml) in cell culture media consisting of 200 µl of RPMI 1640 supplemented with 10% human sera. Wells exhibiting growth between 10 - 14 days, were assessed for Mtb specificity using ELISPOT and Mtb-infected DC as a source of APCs. T cells retaining Mtb specificity were further phenotyped for αβ T cell receptor expression and CD8 expression by FACS and expanded as described below Vβ usage was determined using the IOTest Beta Mark Kit from Beckman Coulter.

*Expansion of T cell clones.* To expand the CD8⁺ T cell clones, a rapid expansion protocol using anti-CD3 mAb stimulation was used as described previously (Heinzel et al., J Exp Med 196:1473-1481, 2002).

*Generation and Infection of Peripheral Blood DCs*. Monocyte-derived DCs were prepared (Heinzel et al.., *supra*; Romani et al., J Exp Med 180:83 -93, 1994). To generate Mtb-infected DC, cells (1 X 10⁶) were cultured overnight in the presence of Mtb (multiplicity of infection [MOI] = 50:1). After 18 hours, the cells were harvested and resuspended in RPMI/10% human serum.

*MHC binding assays.* The MHC-peptide binding assay utilized measures the ability of peptide ligands to inhibit the binding of a radiolabeled peptide to purified NHC molecules, and has been described in detail elsewhere (Sidney et al.; 1999 UNIT 18.3 Measurement of MHC/peptide interactions by gel filtration In Current Protocols in Immunology. Coligan et al., eds., John Wiley & Sons, Inc, 1996). Briefly, purified MHC molecules, test peptides, and a radiolabeled probe peptide were incubated at room temperature in the presence of human B2-microglobulin and a cocktail of protease inhibitors. After a two-day incubation, binding of the radiolabeled peptide to the corresponding MHC class I molecule was determined by capturing MHC/peptide complexes on W6/32 antibody (anti-HLA A, B, and C) or B123.2 (anti-HLA B, C and some A) coated plates, and bound counts per minute (cpm) were measured using a microscintillation counter. For competition assays, the concentration of peptide yielding 50% inhibition of the binding of the radiolabeled peptide was calculated. Peptides were typically tested at six different concentrations covering a 100,000- fold dose range, and in three or more independent assays. Under the conditions utilized, where [label]<[MHC] and IC₅₀ ≥ [MHC], the measured IC₅₀ values are reasonable approximations of the true Kd values.

*IFN-γ ELISPOT assay*. The EFN-γ ELISPOT assay was performed as described previously (Beckman et al., J Immunol 157:2795-2803, 1996). For determination of *ex vivo* frequencies of CD4⁺ or CD8⁺ T cells responding to Mtb infection or Mtb antigens, CD4⁺ or CD8⁺ T-cells were positively selected from PBMC using magnetic beads (Miltenyi Biotec, Auburn CA) as a source of responder T cells and tested in duplicate at four different cell concentrations. Autologous DC (20,000 cells/well) were used as APC and DC were either infected with Mtb or pulsed with peptide pools (5 µg/ml, final concentration of each peptide) and then added to the assay For assays using T cell clones, T cells (1000 or 5000 cells/well) were incubated with autologous LCL (20,000 cells/well) in the presence or absence of antigen.

*Data analysis* To determine the *ex vivo* frequency of antigen-specific T cells, the average number of spots per well for each duplicate was plotted against the number of responder cells per well. Linear regression analysis was used to determine the slope of the line, which represents the frequency of antigen specific T cells. The assay is considered positive, i.e., reflecting the presence of a primed T cell response, if the binomial probability (Lewinshon et al., Microbes Infect 8:2587-2598, 2006) for the number of spots is significantly different by experimental and control assays. To determine differences in *ex vivo* T cell frequencies between groups, Wilcoxon/Kruskal-Wallis analysis was used.

### Example 2

### Defining Immunodominant Mtb-Specific CD8⁺ Antigens

To define immunodominant Mtb-specific CD8⁺ antigens, and to determine whether or not these responses result from infection with Mtb, CD8⁺ T cells were used from donors uninfected, with LTBI; or actively infected with Mtb. Responses were determined either directly *ex vivo,* or using CD8⁺ T cell clones obtained by limiting dilution cloning on Mtb-infected autologous DC (Lewinsohn et al., J Immunol 165:925-930, 2000). As much is known about dominant CD4⁺ Mtb antigens, a panel of these commonly recognized antigens was selected for further evaluation These were: Mtb39, CFP10, and Mtb8.4, Mtb9.9, ESAT-6, Ag85b, 19kDa, and EsxG. To avoid bias introduced by using peptides of predicted HLA binding specificity, we synthesized overlapping peptides (15 aa, overlap 11 aa) to represent the proteins of interest (Lewinshon et al., J Immunol 166:439-446, 2001).

To accurately determine the *ex vivo* effector cell frequencies of CD8⁺ T cells, linear regression analysis was used. As shown in Fig. 1, magnetic bead purified CD8⁺ T cells were tested against peptide pulsed DC over a range of CD8⁺ T cell numbers in an IFN-γ ELISPOT assay, A positive assay was determined as described below and if positive, the antigen specific frequency was determined using linear regression.

Subjects uninfected (*n* = 14), those with LTBI (*n* = 20) and those with active IB (*n* = 12) were evaluated for CD8⁺ responses to a panel of Mtb CD4⁺ T cell antigens, as well as to Mtb-infected DC All subjects tested had robust CD8⁺ T cell responses to Mtb-infected DC and were of greater magnitude in individuals with active TB than in those with LTBI (p = 0.01; Fig. 2, Table I). However, CD8⁺ T cell responses to the panel of Mtb antigens were found almost exclusively in those infected with Mtb in that statistically significant differences between uninfected and Mtb-infected individuals were noted for seven often antigens for both the magnitude of the response (Fig 2) and the proportion of positive assays (Table I)

**Table I. CD8⁺ T cell responses to known TB antigens.**

| Antigen | Mtb Infected # positive^{a} / # tested (%) | Mtb Uninfected # positive^{a} / # tested (%) | P value (2 tail fishers) |
|---|---|---|---|
| Mtb DC | 17/17 (100) | 11/11 (100) | |
| Mtb39 Pool A | 13/30 (43) | 0/14 (0) | 0.003 |
| Mtb 39 Pool B | 10130 (33) | 0/14 (0) | 0.01 |
| CFP10 | 14/30 (47) | 1/14 (7) | 0.02 |
| Mtb 8.4 | 13/30 (43) | 0/4 (0) | 0.003 |
| Mtb | 9.9 10/25 (40) | 1//4 (7) | 0.06 |
| ESAT 6 | 12/25 (48) | 0/14 (0) | 0.003 |
| Ag85b Pool A | 5/22 (23) | 1/14 (7) | 0.37 |
| Ag85b.Pool B | 4/22 (18) | 0/14 (0) | 0.14 |
| 19 kd | 6/22 (27) | 1/12 (8) | 0.38 |
| EsxG | 9/22(41) | 0/14 (0) | 0.006 |

| | | | |
|---|---|---|---|
| ^{a}Positive assay defined in text. | | | |

However differences in CD8⁺ T cell responses between individuals with active TB and LTBI were not statistically different, While strong CD8⁺ T cell responses were observed against many of the antigens tested, it is equally notable that several subjects with strong Mtb directed CD8⁺ T cell responses did not have demonstrable responses to many of the antigens tested.

These *ex vivo* frequency data demonstrate the presence of high-frequency responses to a number of known Mtb antigens, but do not shed light on the restricting allele, minimal epitope, or dominance Hierarchy within the gene of interest To address this question, limiting dilution cloning of human CD8⁺ T cells using Mtb-infected DC was performed (see Lewinsohn et aL, J Immunol 166:439-446, 2001), and panels of both classically and non-classically HLA-restricted CD8⁺ T cell clones were generated. Using peptide pools representing known CD4⁺ antigens, the antigenic specificity of the HLA-Ia restricted clones can be defined in more than half of the clones (TableII).

**Table II. Many CD8⁺ T cell clones recognize known CD4⁺ T cell antigens**

| **Donor** | **Tb Status** | **HLA-Ia Clones (#)^{a}** | **Antigen Identifled (#)^{b}** | **# Distinct Antigens (#)**^{c} | **# Distinct Epitopes (#)^{d}** |
|---|---|---|---|---|---|
| D431 | Active TB | 1 | 0 | 0 | 0 |
| D432 | Active TB | 14 | 4 | 2 | 2 |
| D466 | Active TB | 11 | 10 | 1 | 2 |
| D571 | Active TB | 7 | 7 | 1 | 1 |
| D480 | Active TB | 6 | 6 | 1 | 1 |
| D481 | Active TB | 11 | 11 | 1 | 1 |
| D426 | LTBI | 1 | 0 | 0 | 0 |
| D443 | LTBI | 1 | 1 | 1 | 1 |
| D454 | LTBI | 2 | 2 | 2 | 2 |
| D504 | LTBI | 7 | 1 | 1 | 1 |
| Totals | | 61 | 42 | 10 | 11 |

| | | | | | |
|---|---|---|---|---|---|
| ^{a} Number of clones derived from donor ^{b} Number of clones for which cognate antigen was identified. ^{c} Total number of distinct antigens idenfifed from the clone set. ^{d} Total number of distinct epitopes identified from the clone set. | | | | | |

This approach is demonstrated in detail for a single representative clone, D466 D6, derived from a subject with active TB. As shown in Fig 3A, testing the clone against autologous DC pulsed with a panel of peptide pools unambiguously defined the antigenic specificity as CFP10, The clone was then tested against each of the 15-mer peptides that comprise the CFP10 pool, revealing that the epitope was contained within CFP10₁₋₁₅ (Fig. 3B). Each possible 8 aa, 9 aa, 10 aa, and 11 aa peptide was then synthesized and tested for reactivity, revealing antigenic activity between aa 2-11 (Fig. 3C). Similarly, each clone was tested against lymphoblastoid cell lines (LCL) sharing at least one HLA-type with the donor (Fig. 3D). Autologous LCL and IHW 9058 LCL, which share B4501 and C1601, present the epitope to the clone, identifying both B4501 and C1601 as possible restricting alleles. However, C1601⁺ D433 LCL do not present the epitope, eliminating C1601 as a candidate restricting allele. Therefore D466 D6 is restricted by HLA-B4501. As demonstrated in Fig. 4, by testing each plausible epitope over a broad range of concentrations, the minimal epitope was defined as CFP10₂₋₁₀ for D466 D6. Experimental data supporting the assignment of the minimal epitope is provided for each clone in the supplemental Figure. A summary of the antigenic specificity, minimal epitope, and HLA-restricting allele is presented in Table III. Unexpectedly, all but one of the T cell clones were restricted by HLA-B alleles. Furthermore, a minority of those observed were 9 aa in length

**Table III. Summary of Epitopes Identified**

| **Clone^{a}** | **Gene** | **Accession Number** | **HLA-Restrict Allele** | **Epitope Locat'n** | **Epitope Sequence (SEQ ID NO: 26-38)** | **#SFU^{b}** | **MHC Bind. Aff.^{c}** | **V beta region** |
|---|---|---|---|---|---|---|---|---|
| D160 1-1B^{d} (0) | CFP10 | Rv3874 | B44 | 2-11 | **AEMKTDAATL** | **360** | 38 | |
| D160 1-6F^{d} (0) | CFP10 | Rv3874 | B14 | 85-94 | **RADEEQQQAL** | **120** | NA | |
| D432 H12 (2) | CFP10 | Rv3874 | B3514 | -49-58 | **TAAQAAVVRF** | **258** | 2011^{c} | 53 |
| D466 A10 (10) | CFP10 | Rv3874 | B4501 | 2-9 | **AEMKTDAA** | **2458** | 48 | IND |
| D466 D6 (1) | CFP10 | Rv3874 | B4501 | 2-12 | **AEMKTDAATLA** | **1993** | 62 | 22 |
| D481 C10 (10) | CFP10 | Rv3874 | B1502 | 75-83 | **NIRQAGVQY** | **1715** | 14^{f} | 9 |
| D481 C11 (1) | CFP10 | Rv3874 | B1502 | 75-83 | **NIRQAGVQY** | **1715** | 14^{f} | 13.6 |
| D480 F6 (6) | CFP10 | Rv3874 | B0801 | 3-11 | **EMKTDAATL** | **387** | 79 | 13.1 |
| D571 B12 (3) | CFP10 | Rv3874 | B4402 | 2-11 | **AEMKIDAATL** | **31** | 38 | IND |
| D571 E9 (4) | CFP10 | Rv3874 | B4402 | 2-11 | **AEMKIDAATL** | **31** | 38 | 14 |
| D504 E4 (1) | Mtb9 8 | Rv0287 | A0201 | 3-11 | **LLDAHIPQL** | **<10** | 0.39 | 8 |
| D454 B10 (1) | Mtb9 8 | Rv0287 | B0801 | 53-61 | **AAHARFVAA** | **88** | 0.22 | IND |
| D454 H1-2 (1) | Mtb8 4 | Rv1174c | B1501 | 5-15 | **AVINTTCNVGQ** | **2d** | 10 | 7.1 |
| D432 A3 (2) | Mtb 8 4 | Rv1174c | B3514 | 32.40 | **ASPVAQSYL** | **210** | 121^{c} | 14 |
| D443 H9 (1) | Ag85 B | Rv1886c | ID | 144-153 | **ELPQWLSANR** | **<10** | NA | 22 |

| | | | | | | | | |
|---|---|---|---|---|---|---|---|---|
| ^{a}Number of sister clones is in parenthesis. ^{b}# of SFU /250,000 CD8⁺ T cells is shown. ^{c}IC50 In nm is shown. ^{d}Publisbed previously J Immunol. 2001 Jan 1;166(1),439-46 ^{d}Measured binding affinity to B3501 is shown. ^{f}Measured binding affinity to B1501 is shown. NA = Not Availnble. IND = Indeterminate TBD = To be done | | | | | | | | |

Because each of the individual CD8⁺ T cell clones were derived based on growth of Mtb- infected DC, it was determined Whether or not the antigen and epitopes identified reflected immunodominant epitopes *ex vivo*. Two independent approaches were pursued, the first to determine if the response was present at high frequency, and the second to determine what proportion of the total response to the antigen is constituted by the epitope. To determine the ex-vivo effector cell frequency, as described in Fig. 1, each epitope was tested using autologous DC and magnetic bead purified CD8^{*} T cells derived from the donor from whom the T cell clones was isolated. A summary of the effector cell frequencies is presented in Table III. For the majority, the epitopes reflect high frequency responses, and thus could be considered a response that has been primed by exposure to Mtb Notably, T cell clones isolated from four donors recognized CFP10. To determine if the epitopes defined reflected a substantial proportion of the total response to the aritigen of interest, magnetic bead purified CD8⁺ T cells from three donors with sufficient available peripheral blood mononuclear dells (PBMC) were tested for reactivity to each individual 15-mer peptide, the peptide pool, and peptide representing the minimal epitope. As is demonstrated in Fig. 5, the *ex vivo* frequencies to the minimal epitope, 15-mer peptide(s) containing the minimal epitope, and peptide pool were remarkably concordant. These data suggested that for each donor a dominance hierarchy has been clearly established, and is reflected in the original clones. Finally, as is noted in Table III, daughter clones of identical specificity were frequently identified, a result that would be predicted based on an immundominance hierarchy. TCR V beta staining was used to confirm the clonal relationship between daughter clones. Interestingly, in two cases, the identical minimal epitope and HLA-restriction was represented by two distinct clones (Table III).

Because much work on human CD8⁺ T cell responses to Mtb has relied upon the use of HLA-prediction algorithms, as each epitope was defined we asked whether or not the epitopes would have been predicted by these approaches. Many of these epitopes were not ranked strongly. This might highlight the limitations of those algorithms at the time they were used. To address this question experimentally, the IC₅₀ for each peptide that had been synthesized in the course of definition of the minimal epitope was determined against a panel of human HLA molecules Shown in Table III is the IC₅₀ for the minimal epitope with the cognate restricting allele. The data demonstrated that the T cell epitopes bound avidly to HLA, and show a high degree of concordance between the T cell epitope data and HLA-binding data.

The data demonstrated that CD8⁺ T cell responses are present in persons infected with Mtb at frequencies that are comparable to that seen following many common viral infections such as vaccinia, influenza, and CMV. All but one of the epitopes that were mapped were restricted by HLA-B molecules. The data suggest that by using a T cell driven approach to epitope identification, dominant epitopes can be defined in humans infected with Mtb.

### Example 3

### Screening of T cell clones against a genomic peptide library

The classically-restricted and non-classically-restricted T cell clones (see Table II above) that did not recognize one of the known Mtb antigen peptide pools (Rv3875, Rv3874, Rv1886c, Rv0287, Rv3763, Rv1174c, Rv1196, Rv1793, Rv2346c, Rv1037c, Rv3619c and Rv1198) were screened against a genomic peptide library. This peptide library represents 389 genes, representing roughly 10% of the Mtb genome. The peptides are 15mers overlapping by 11 for each gene product. 50nmol of each peptide was synthesized individually and then pooled into 777 pools of 50 peptides in a 96 well format (nine plates): Five blank wells and one well of' an irrelevant peptide pool, SIV gag, were included on each of the nine plates. To screen the clones against the genomic peptide library, the clones are first expanded and tested against Mtb-infected DCs to ensure that each clone from this particular expansion yields a robust Mtb-specific signal in the ELISPOT assay. Then up to six T' cell clones are pooled. For the screen, T cell clones (5,000 cells/well of each clone), autologous DCs (20,000 cells/well), IL-2 (0.5ng/ml) and the peptide pools (5ug/ml, individual peptides) were incubated overnight at 37C in the ELISPOT assay. Only one technical replicate is done per pool because 5000 T cell clones per well with a peptide antigen produced an overwhelmingly positive response, resulting in a definitive result Six classical clones from D504 were screened against the genomic peptide library, leading to the discovery of a new epitope. This epitope was from a family of four proteins that includes EsxJ, EsxW, EsxK and EsxP., These proteins share 98% homology and differ at only 3 amino acids. There is a fifth member of this family, EsxM (Rv1792), that was not included in the genomic peptide library.

The clones were screened against the individual fifteen-mers for these peptide pools. All six classical clones recognized EsxJ 21-3 5. This is a region of EsxJ that is identical to the other four members of this family. Next, 9, 10 and 11mer peptides were made from this 15mer and screened against each clone. The minimal epitope was determined to be EsxJ 24-34. In addition, the HLA restriction was found to be B5701.

### Example 4

### Additional Screening of T cell clones against a genomic peptide library

Eleven classical clones from D432B were screened against the genomic peptide library described above, The antigen was determined for two clones, which led to the identification of two novel epitopes, PE_PGRS42₄₇₋₅₅ and PE9₅₃₋₆₇. The minimal epitope for one clone was determined to be PE_PGRS42₄₇₋₅₅ and the HLA restriction was found to be B3514. The minimal epitope for the other clone is not yet determined, but is contained in the 15mer PE9₅₃₋₆₇. The HLA restriction for this clone was found to be B3905.

**Table IV. Detail of Novel Epitopes from Genomic Peptide Library Screens.**

| **Clone** | **Gene** | **Accession Number** | **Epitope Location** | **Epitope** | **#SFU/ 250,000 CD8+ T-cells** | **MHC-Restriction** | **MHC Binding Afflnity (IC50 nm)** | **TCR V beta region** |
|---|---|---|---|---|---|---|---|---|
| D504 F9 (6) | EsxJ* | Rv1038c | 24-34 SEQ ID NO: 2 | **QTVEDEARRMW** | **84** | B5701 | TBD | Indeterminate |
| D432 D8(1) | PE9 | Rv1088 | 53-67 SEQ ID NO: 7 | **RLFNANAEEYHA-LSA** | **TBD** | B3905 | TBD | 8 |
| D432 H8 (1) | PE_PGR S42 | Rv2487c | 47-55 SEQ ID NO: 8 | **VSAAIAG -LF** | **TBD** | B3514 | TBD | 71 |

| | | | | | | | | |
|---|---|---|---|---|---|---|---|---|
| Number of clones recognizing epitope from each donor in parentheses. *This is a family of proteins that have almost identical sequences The family consists of Rv1038c, Rv1197, Rv2347, Rv3620c. | | | | | | | | |

**. Table V. Summary of Completed Clone Screens.**

| Donor | TB Status | #.Classical available (screened) | # Non-Classical available (screened) | # positive wells in screen | # of confirmed hits | # novel epitopes | # classical clones epitope Identified | # classical clones epitope NOT identified |
|---|---|---|---|---|---|---|---|---|
| 426 | PPD+ | 1 (1) | 4 (4) | 1 | 0 | 0 | 0 | 1 |
| 431 | Active | 1 (1) | 1 (1) | 1** | 0 | 0 | 0 | 1 |
| 432 | Active | 11 (11) | 14 (7) | 11 | 3 | 2 | 3 | 8 |
| 454 | PPD+ | 1* (0) | 6 (4) | 0 | 0 | 0 | 0 | 0 |
| 466 | Active | 1 (1) | 4 (4) | 1 | 0 | 0 | 0 | 1 |
| 504 | PPD+ | 6 (6) | 9 (9) | 5 | 4 | 1 | 6 | 0 |
| | | 21 (20) | 38 (29) | 18 | 7 | 3 | 9 | 11 |

| | | | | | | | | |
|---|---|---|---|---|---|---|---|---|
| * The classical clone from D454 did not recognize Mtb upon re-expansion and was not screened against library ** The classical clones from 426 and 431 were screened together, so there was one positive well between both clones, | | | | | | | | |

### Example 5

### Screening of ex vivo CD8⁺ T-ce/ls against a genomic peptide library

CD8+ T-cells from a LTBI donor, D610 (SE Asian) were screened against the genomic peptide library described above. Each plate of the genomic peptide library was screened in duplicate, for a total of 18 ELISPOT plates per screen. CD8⁺ T-cells were prepared from cryopreserved PBMC by CD8⁺ selection using magnetic bead separations. Resulting cell populations contained ≥96% CD8⁺ T cells. CD8⁺ T cells (250,000 cells/well), autologous DCs (20,000 cells/well), and IL-2 (0.5ng/ml) were added to peptide (final 5ug/ml, individual peptides) in the ELISPOT plates. Five media control wells are included on each plate For each plate, the mean of these five wells was subtracted from each well of that plate to normalize between plates. Each technical replicate on each plate was then scored. A well was scored positive if the spot forming units (SFU), less the mean of the media wells, was greater than or equal to ten and the SFU was greater than or equal to twice the mean of the media. (Hudgens et al., J. Immunol Methods 288: 19-34, 2004). This donor responded to the four peptide, wells containing EsxJ, EsxW, EsxK and EsxP. CD8⁺ T-cells were then screened against each 15mer from these peptide pools and found to respond only to EsxJ 21-35, the same region of EsxJ, EsxW, EsxK and EsxP that is described in example 3 above.

Seven additional donors were screened against the genomic peptide library. The top 10 responses are detailed in Table 7. The four peptide pools highlighted in yellow contain peptides from only one gene. These four genes contain four novel epitopes.

**Table 7. Top 10 Responses from Peptide Pool Screens of Seven Donors. Spot Forming Units are for 250,000 CD8+ T-cells.**

| Peptide Pool | Donor | **Average SFU** | RvNumbers Represented in Wells | Functional Category |
|---|---|---|---|---|
| C09_1 | D560 | **208.2** | Rv1860(50): | cell wall and cell processes |
| C12_4 | D545 | **156.4** | Rv0468(27): Rv0456c(23): | lipid metabolism |
| A04_3 | D454 | **136** | Rv0284(I7):Rv0288(11):Rv0287(22) | cell wall and cell processes |
| B10_3 | D560 | **112.3** | Rv1273c(50): | cell wall and cell processes |
| E04_4 | D560 | **78.2** | Rv0152c(40):Rv0151c(10): | PE/PPE |
| G12_8 | D560 | **77.4** | Rv3478(18):Rv3507(32): | PE/PPE |
| E07_4 | D525 | **76.8** | Rv0159c(50): | PE/PPE |
| A10_8 | D560 | **70.4** | Rv3136(47):Rv3144c(3): | PE/PPE |
| E11_8 | D560 | **66.4** | Rv3350c(50): | PE/PPE |
| E08_9 | D545 | **60.2** | Rv1404(13):Rv2711(31): | regulatory proteins |

### Example 6

### Animal models

In tuberculosis research, the mouse model has been used extensively to model various aspects of the disease. Mice can be infected by a variety of routes, including intravenous, intraperitoneal and tracheal. One route is aerosolization of the organism for respiratory infection. The mice are exposed to the aerosol in a chamber (wither whole body or nose only infection) The dose of invention can be varied by manipulating the concentration of Mtb in the nebulizer or time of exposure. A low dose infection, such as about 50 colony forming units (CFU) via aerosol reulsts in a slow and steady increase in bacterial numbers in the lungs, generally reaching a peak in four weeks, which coincides with the peak number off cells in the lungs. The initial period is considered the acute stage of infection. Following infection, there is a dissemination of bacteria to the mediastinal lymph nodes. T cell priming is generally detectable between two and three weeks. After about four weeks the bacterial numbers stabilize, and there is a slow progressive pathologic response. This system is of use for modeling active infection. Thus, the above described polypeptides, or polynucleotides encoding these polypeptides, can be administered prior to infection.. The ability of the Mtb polypeptides (or polynucleotides encoding these polypeptides) to prevent infection is then assessed. Alternatively, the mice are administered Mtb, and the ability of the Mtb polypeptide (or polynucleotide encoding these polypeptides) to treat the Mtb infection is monitored. The effectiveness of the Mtb polypeptides (or polynucleotides) can be monitored by measuring the T cell response, such as the number of CD8⁺ or CD4⁺ T cells, and/or measuring the bacterial numbers, and/or evaluating the pathology.

Exemplary protocols are provided below (see also Repique et al.; Infec. Immun. 70: 3318-3323, 2002 for an additional protocol):

### A. Short Term Mouse Model:

C57BL/6 mice are vaccinated with a composition including one or more Mtb polypeptide, or a polynucleotide encoding these one or more polypeptides according to the appropriate protocol and then rested for 4 to 6 weeks. Immunized mice are infected with a low dose aerosol 50-100 CFU) of virulent M. tuberculosis and protection is evaluated by assessing the number of viable bacilli 30 days post challenge.

Viable counts are performed on the lung and spleen of mice by homogenizing the organs and plating serial 10-fold dilutions on 7H11 agar plates. Plates are incubated for up to 21 days and the number of colony forming units per organ determined.

BCG vaccinated mice have approximately 1 Logl 0 protection in their lung and spleen when compared to PBS-treated mice

### B. Short term guinea pig model

Out-bred Hartley guinea pigs are vaccinated with a composition including one or more Mtb polypeptide, or a polynucleotide encoding these one or more polypeptides and then rested for 8 to 10 weeks. Immunized guinea pigs are infected with a low dose aerosol (10-30 CFU) of virulent *M. tuberculosis* and protection is evaluated by assessing the number of viable bacilli 30 days post challenge.

Viable counts are performed on the lung and spleen of guinea pigs by homogenizing the organs and plating serial 10-fold dilutions on 7H11 agar plates. Plates are incubated for up to 21 days and the number of colony forming units per organ determined Lung and spleen segments are also taken for histological analyses.

BCG vaccinated guinea pigs have approximately 2-3Log₁₀ protection in their lung and spleen when compared to PBS-treated guinea pigs. In addition, BCG vaccinated guinea pigs have well defined granulomas when compared to unvaccinated animals.

### C. Long term guinea pig model

The guinea pig model is similar to the mouse model, but the experiments are open-ended survival type and can last for as long as 2 years Guinea pigs develop 'classical' granulomas similar to humans with active tuberculosis (TB), and as lung tissue necrosis progresses, they begin to lose weight and die of TB similar to humans The number of colony forming units in the lungs and spleen can be assessed. Histological examination can also be performed to determine the degree of lung involvement and tissue destruction. After low-dose aerosol exposure in the guinea pig the number of organisms increases progressively during the first three weeks and then plateaus into a chronic state. During the later stages of infection there is increased bacterial load in the lung and this is associated with a worsening pathological condition. Without treatment, there is a concomitant rise in both CD4 and CD8 T cells in the lungs of infected guinea pigs.

Out-bred Hartley guinea pigs are vaccinated with the experimental vaccine according to the appropriate protocol and then rested for 8 to 10 weeks. Immunized guinea pigs are then infected with a low dose aerosol (10-30 CFU) of virulent *M. tuberculosis"* Guinea pigs are weighedweekly and monitored daily for signs of disease (such as increased respiration and failure to thrive). Unvaccinated guinea pigs succumb to infection from 20 to 25 weeks post challenge, while BCG vaccinated guinea pigs survive for 50 to 55 weeks post challenge.

At necropsy, the lung and spleen are assessed for the number of CFU and the extent of pathology. The relative protection of the experimental composition is compared to BCG vaccinated animals.

It will be apparent that the precise details of the methods or compositions described may be varied or modified without departing from the described invention. We claim all such modifications and variations that fall within the scope of the claims below.

## Claims

1. A polypeptide, wherein the polypeptide comprises:
(a) the amino acid sequence set forth as SEQ ID NO: 7; or
(b) at least nine to twenty consecutive amino acids of the amino acid sequence set forth as SEQ ID NO: 7, wherein the nine to twenty consecutive amino acids specifically bind major histocompatibility complex (MHC) class I,
for use in a method for producing an immune response to *Mycobacterium tuberculosis* in a subject, comprising
administering to the subject a therapeutically effective amount of the polypeptide or polynucleotide;
thereby inducing an immune response to *Mycobacteriurn tuberculosis.*

2. The polypeptide for use according to claim 1, wherein the polypeptide is covalently linked to a carrier.

3. The polypeptide for use according to claim 1, wherein the polypeptide consists of:
(a) the amino acid sequence set forth as SEQ ID NO: 7; or
(b) at least nine consecutive amino acids of the amino acid sequence set forth as SEQ ID NO: 7, wherein the nine consecutive amino acids specifically bind MHC class I.

4. A polynucleotide encoding a polypeptide, wherein the polypeptide comprises:
(a) the amino acid sequence set forth as SEQ ID NO: 7; or
(b) at least nine to twenty consecutive amino acids of the amino acid sequence set forth as SEQ ID NO: 7, wherein the nine to twenty consecutive amino acids specifically bind major histocompatibility complex (MHC) class I,
for use in a method for producing an immune response to *Mycobacterium tuberculosis* in a subject, comprising
administering to the subject a therapeutically effective amount of the polypeptide or polynucleotide;
thereby inducing an immune response to *Mycobacterium tuberculosis.*

5. The polynucleotide for use according to claim 4, wherein the polypeptide consists of:
(a) the amino acid sequence set forth as SEQ ID NO: 7; or
(b) at least nine consecutive amino acids of the amino acid sequence set forth as SEQ ID NO: 7, wherein the nine consecutive amino acids specifically bind MHC class I.

6. The polypeptide or a polynucleotide for use according to any one of claims 1-5, wherein the method comprises further administering a therapeutically effective amount of an adjuvant.

7. The polypeptide or a polynucleotide for use according to any one of claims 1-6, wherein the immune response is a protective immune response.

8. The polypeptide or a polynucleotide for use according to any of claims 1-7, wherein the subject is infected with Mtb, or the subject is at risk for infection with Mtb, or the subject has a latent infection with Mtb.

9. The isolated polypeptide for use according to claim 1,
wherein the polypeptide comprises nine to twenty consecutive amino acids of the amino acid sequences set forth as SEQ ID NO: 7, wherein the nine to twenty consecutive amino acids specifically bind major histocompatibility complex (MHC) class I, wherein the isolated polypeptide does not comprise the amino acid sequence set forth as SEQ ID NO: 7.

10. The isolated polypeptide for use according to claim 9,
wherein the polypeptide consists of nine to twenty consecutive amino acids of the amino acid sequences set forth as SEQ ID NO: 7, wherein the nine to twenty consecutive amino acids specifically bind major histocompatibility complex (MHC) class I.

11. An isolated polynucleotide for use in a method for producing an immune response to *Mycobacterium tuberculosis* in a subject, comprising
administering to the subject a therapeutically effective amount of the polynucleotide,
which polynucleotide encodes the polypeptide as defined in claim 9 or 10.

12. An isolated polynucleotide for use according to claim 11, wherein the polynucleotide is operably linked to a promoter.

13. A vector for use in a method for producing an immune response to *Mycobacterium tuberculosis* in a subject, comprising
administering to the subject a therapeutically effective amount of the polynucleotide in the vector,
which vector comprises the polynucleotide as defined in claim 11 or 12.

14. A vector for use according to claim 13, wherein the vector is a plasmid vector or a viral vector.

15. A vector for use according to claim 13, wherein the plasmid vector is expressed in yeast or wherein the viral vector is a retrovirus, orthopox, avipox, fowlpox, capripox, suipox, adenoviral, herpes virus, alpha virus, baculovirus, Sindbis virus, vaccinia virus and poliovirus vectors.

16. An isolated host cell comprising the vector as defined in any one of claims 13 to 15,
wherein the host cell is a eukaryotic cell, a yeast cell, or an *E*. *coli* cell.

17. A pharmaceutical composition for use in a method for producing an immune response to *Mycobacterium tuberculosis* in a subject, comprising
administering to the subject a therapeutically effective amount of a composition comprising the polypeptide as defined in claims 9 or 10, and a pharmaceutically acceptable carrier, or comprising a therapeutically effective amount of the polynucleotide as defined in claim 11 or 12 in a pharmaceutically acceptable carrier

18. A polypeptide or a polynucleotide as defined in any one of claims 1 to 5 for use in a method for treating a subject infected with *Mycobacterium tuberculosis,* comprising administering to the subject a therapeutically effective amount of the polypeptide or a polynucleotide encoding the polypeptide,
thereby treating the subject infected with *Mycobacterium tuberculosis.*

19. The polypeptide or a polynucleotide for use according to claim 18, wherein the subject infected with Mycobacterium tuberculosis does not have a symptom of tuberculosis.

20. The polypeptide or a polynucleotide for use according to claim 19 wherein treating the subject comprises preventing the development of tuberculosis in the subject.

21. An isolated polypeptide or a polynucleotide as defined in any one of claims 1 to 5, for use in a method of inhibiting an infection with *Mycobacterium tuberculosis,* in a subject comprising administering to the subject a therapeutically effective amount of the polypeptide or polynucleotide;
thereby inhibiting the infection with *Mycobacterium tuberculosis* in the subject.

22. The isolated polypeptide or a polynucleotide for use according to claim 21, wherein administering to the subject the therapeutically effective amount of the polypeptide or the polynucleotide encoding the polypeptide prevents an infection with *Mycobacterium tuberculosis.*

23. The pharmaceutical composition for use according to claim 17, further comprising an adjuvant.

## Patentansprüche

1. Polypeptid, wobei das Polypeptid Folgendes umfasst:
(a) die als SEQ ID Nr. 7 dargestellte Aminosäuresequenz oder
(b) mindestens neun bis zwanzig aufeinander folgende Aminosäuren der als SEQ ID Nr. 7 dargestellten Aminosäuresequenz, wobei die neun bis zwanzig aufeinander folgenden Aminosäuren die Klasse I des Haupthistokompatibilitätskomplexes (major histocompatibility complex, MHC) spezifisch binden,
zur Verwendung in einem Verfahren zur Erzeugung einer Immunantwort auf *Mycobacterium tuberculosis* in einem Subjekt, wobei das Verfahren Folgendes umfasst:
Verabreichen einer therapeutisch wirksamen Menge des Polypeptids oder des Polynukleotids an das Subjekt;
wodurch eine Immunantwort auf *Mycobacterium tuberculosis* induziert wird.

2. Polypeptid zur Verwendung nach Anspruch 1, wobei das Polypeptid mit einem Träger kovalent verknüpft ist.

3. Polypeptid zur Verwendung nach Anspruch 1, wobei das Polypeptid aus folgendem besteht:
(a) der als SEQ ID Nr. 7 dargestellten Aminosäuresequenz oder
(b) mindestens neun aufeinander folgenden Aminosäuren der als SEQ ID Nr. 7 dargestellten Aminosäuresequenz, wobei die neun aufeinander folgenden Aminosäuren die MHC-Klasse I spezifisch binden.

4. Polynukleotid, das ein Polypeptid codiert, wobei das Polypeptid Folgendes umfasst:
(a) die als SEQ ID Nr. 7 dargestellte Aminosäuresequenz oder
(b) mindestens neun bis zwanzig aufeinander folgende Aminosäuren der als SEQ ID Nr. 7 dargestellten Aminosäuresequenz, wobei die neun bis zwanzig aufeinander folgenden Aminosäuren die Klasse I des Haupthistokompatibilitätskomplexes (major histocompatibility complex, MHC) spezifisch binden,
zur Verwendung in einem Verfahren zur Erzeugung einer Immunantwort auf *Mycobacterium tuberculosis* in einem Subjekt, wobei das Verfahren Folgendes umfasst:
Verabreichen einer therapeutisch wirksamen Menge des Polypeptids oder des Polynukleotids an das Subjekt;
wodurch eine Immunantwort auf *Mycobacterium tuberculosis* induziert wird.

5. Polynukleotid zur Verwendung nach Anspruch 4, wobei das Polypeptid aus folgendem besteht:
(a) der als SEQ ID Nr. 7 dargestellten Aminosäuresequenz oder
(b) mindestens neun aufeinander folgenden Aminosäuren der als SEQ ID Nr. 7 dargestellten Aminosäuresequenz, wobei die neun aufeinander folgenden Aminosäuren die MHC-Klasse I spezifisch binden.

6. Polypeptid oder Polynukleotid zur Verwendung nach einem der Ansprüche 1 - 5, wobei das Verfahren weiterhin das Verabreichen einer therapeutisch wirksamen Menge eines Hilfsstoffs umfasst.

7. Polypeptid oder Polynukleotid zur Verwendung nach einem der Ansprüche 1 - 6, wobei die Immunantwort eine schützende Immunantwort ist.

8. Polypeptid oder Polynukleotid zur Verwendung nach einem der Ansprüche 1 - 7, wobei das Subjekt mit Mtb infiziert ist oder bei dem Subjekt ein Risiko einer Infektion mit Mtb besteht oder das Subjekt eine latente Infektion mit Mtb hat.

9. Isoliertes Polypeptid zur Verwendung nach Anspruch 1,
wobei das Polypeptid neun bis zwanzig aufeinander folgende Aminosäuren der als SEQ ID Nr. 7 dargestellten Aminosäuresequenzen umfasst, wobei die neun bis zwanzig aufeinander folgenden Aminosäuren die Klasse I des Haupthistokompatibilitätskomplexes (major histocompatibility complex, MHC) spezifisch binden, wobei das isolierte Polypeptid nicht die als SEQ ID Nr. 7 dargestellte Aminosäuresequenz umfasst.

10. Isoliertes Polypeptid zur Verwendung nach Anspruch 9,
wobei das Polypeptid aus neun bis zwanzig aufeinander folgenden Aminosäuren der als SEQ ID Nr. 7 dargestellten Aminosäuresequenzen besteht, wobei die neun bis zwanzig aufeinander folgenden Aminosäuren die Klasse I des Haupthistokompatibilitätskomplexes (major histocompatibility complex, MHC) spezifisch binden.

11. Isoliertes Polynukleotid zur Verwendung in einem Verfahren zur Erzeugung einer Immunantwort auf *Mycobacterium tuberculosis* in einem Subjekt, wobei das Verfahren Folgendes umfasst:
Verabreichen einer therapeutisch wirksamen Menge des Polynukleotids an das Subjekt,
wobei das Polynukleotid das wie in Anspruch 9 oder 10 definierte Polypeptid codiert.

12. Isoliertes Polynukleotid zur Verwendung nach Anspruch 11, wobei das Polynukleotid mit einem Promotor operativ verknüpft ist.

13. Vektor zur Verwendung in einem Verfahren zur Erzeugung einer Immunantwort auf *Mycobacterium tuberculosis* in einem Subjekt, wobei das Verfahren Folgendes umfasst:
Verabreichen einer therapeutisch wirksamen Menge des Polynukleotids in dem Vektor an das Subjekt,
wobei der Vektor das wie in Anspruch 11 oder 12 definierte Polynukleotid umfasst.

14. Vektor zur Verwendung nach Anspruch 13, wobei der Vektor ein Plasmidvektor oder ein Virusvektor ist.

15. Vektor zur Verwendung nach Anspruch 13, wobei der Plasmidvektor in Hefe exprimiert wird oder wobei es sich bei dem Virusvektor um einen Retrovirus-, Orthopoxvirus-, Avipoxvirus-, Geflügelpockenvirus-, Capripoxvirus-, Suipoxvirus-, Adenovirus-, Herpesvirus-, Alphavirus-, Baculovirus-, Sindbis-Virus-, Vacciniavirus- und Poliovirusvektor handelt.

16. Isolierte Wirtszelle, die den wie in einem der Ansprüche 13 bis 15 definierten Vektor umfasst, wobei die Wirtszelle eine eukaryotische Zelle, eine Hefezelle oder eine *E*.-*coli*-Zelle ist.

17. Pharmazeutische Zusammensetzung zur Verwendung in einem Verfahren zur Erzeugung einer Immunantwort auf *Mycobacterium tuberculosis* in einem Subjekt, wobei das Verfahren Folgendes umfasst:
Verabreichen einer therapeutisch wirksamen Menge einer Zusammensetzung, die das wie in Anspruch 9 oder 10 definierte Polypeptid und einen pharmazeutisch unbedenklichen Träger umfasst oder eine therapeutisch wirksame Menge des wie in Anspruch 11 oder 12 definierten Polynukleotids in einem pharmazeutisch unbedenklichen Träger umfasst, an das Subjekt.

18. Polypeptid oder Polynukleotid, wie in einem der Ansprüche 1 bis 5 definiert, zur Verwendung in einem Verfahren zum Behandeln eines Subjekts, das mit Mycobacterium tuberculosis infiziert ist, wobei das Verfahren das Verabreichen einer therapeutisch wirksamen Menge des Polypeptids oder eines Polynukleotids, das das Polypeptid codiert, an das Subjekt umfasst,
wodurch das Subjekt, das mit *Mycobacterium tuberculosis* infiziert ist, behandelt wird.

19. Polypeptid oder Polynukleotid zur Verwendung nach Anspruch 18, wobei das Subjekt, das mit *Mycobacterium tuberculosis* infiziert ist, kein Tuberkulosesymptom aufweist.

20. Polypeptid oder Polynukleotid zur Verwendung nach Anspruch 19, wobei das Behandeln des Subjekts das Verhindern der Entwicklung von Tuberkulose in dem Subjekt umfasst.

21. Isoliertes Polypeptid oder Polynukleotid, wie in einem der Ansprüche 1 bis 5 definiert, zur Verwendung in einem Verfahren zum Hemmen einer Infektion mit *Mycobacterium tuberculosis* in einem Subjekt, wobei das Verfahren das Verabreichen einer therapeutisch wirksamen Menge des Polypeptids oder des Polynukleotids an das Subjekt umfasst;
wodurch die Infektion mit *Mycobacterium tuberculosis* in dem Subjekt gehemmt wird.

22. Isoliertes Polypeptid oder Polynukleotid zur Verwendung nach Anspruch 21, wobei das Verabreichen der therapeutisch wirksamen Menge des Polypeptids oder des Polynukleotids, das das Polypeptid codiert, an das Subjekt eine Infektion mit *Mycobacterium tuberculosis* verhindert.

23. Pharmazeutische Zusammensetzung zur Verwendung nach Anspruch 17, die weiterhin einen Hilfsstoff umfasst.

## Revendications

1. Polypeptide, ledit polypeptide comprenant :
(a) la séquence d'acides aminés conforme à SEQ ID NO 7 ; ou
(b) au moins neuf à vingt acides aminés consécutifs de la séquence d'acides aminés conforme à SEQ ID NO 7, les neuf à vingt acides aminés consécutifs se liant de manière spécifique à un complexe majeur d'histocompatibilité (CMH) de classe I,
destiné à être utilisé dans un procédé de production d'une réponse immunitaire à *Mycobacterium tuberculosis* chez un sujet, consistant à :
administrer au sujet une quantité pharmaceutiquement efficace du polypeptide ou du polynucléotide ;
induire ainsi une réponse immunitaire à *Mycobacterium tuberculosis.*

2. Polypeptide destiné à être utilisé selon la revendication 1, le polypeptide étant lié par covalence à un support.

3. Polypeptide destiné à être utilisé selon la revendication 1, le polypeptide comprenant :
(a) la séquence d'acides aminés conforme à SEQ ID NO 7 ; ou
(b) au moins neuf acides aminés consécutifs de la séquence d'acides aminés conforme à SEQ ID NO 7, les neuf acides aminés consécutifs se liant de manière spécifique à un CMH de classe I.

4. Polynucléotide codant un polypeptide, le polypeptide comprenant :
(a) la séquence d'acides aminés conforme à SEQ ID NO 7 ; ou
(b) au moins neuf à vingt acides aminés consécutifs de la séquence d'acides aminés conforme à SEQ ID NO 7, les neuf à vingt acides aminés consécutifs se liant de manière spécifique à un complexe majeur d'histocompatibilité (CMH) de classe I,
destiné à être utilisé dans un procédé de production d'une réponse immunitaire à *Mycobacterium tuberculosis* chez un sujet, consistant à :
administrer au sujet une quantité pharmaceutiquement efficace du polypeptide ou du polynucléotide ;
induire ainsi une réponse immunitaire à *Mycobacterium tuberculosis.*

5. Polynucléotide destiné à être utilisé selon la revendication 4, le polypeptide comprenant :
(a) la séquence d'acides aminés conforme à SEQ ID NO 7 ; ou
(b) au moins neuf acides aminés consécutifs de la séquence d'acides aminés conforme à SEQ ID NO 7, les neuf acides aminés consécutifs se liant de manière spécifique à un CMH de classe I.

6. Polypeptide ou polynucléotide destiné à être utilisé selon l'une quelconque des revendications 1 à 5, le procédé consistant en outre à administrer une quantité pharmaceutiquement efficace d'un adjuvant.

7. Polypeptide ou polynucléotide destiné à être utilisé selon l'une quelconque des revendications 1 à 6, dans lequel la réponse immunitaire est une réponse immunitaire de protection.

8. Polypeptide ou polynucléotide destiné à être utilisé selon l'une quelconque des revendications 1 à 7, dans lequel le sujet est infecté par Mtb, ou le sujet présente un risque d'infection par Mtb, ou le sujet est porteur d'une infection latente par Mtb.

9. Polypeptide isolé destiné à être utilisé selon la revendication 1,
le polypeptide comprenant neuf à vingt acides aminés consécutifs de la séquence d'acides aminés conforme à SEQ ID NO 7, les neuf à vingt acides aminés consécutifs se liant de manière spécifique à un complexe majeur d'histocompatibilité (CMH) de classe I, et le polypeptide isolé ne comprenant pas la séquence d'acides aminés conforme à SEQ ID NO 7.

10. Polypeptide isolé destiné à être utilisé selon la revendication 9,
le polypeptide comprenant neuf à vingt acides aminés consécutifs des séquences d'acides aminés conformes à SEQ ID NO 7, les neuf à vingt acides aminés consécutifs se liant de manière spécifique à un complexe majeur d'histocompatibilité (CMH) de classe I.

11. Polynucléotide isolé destiné à être utilisé dans un procédé de production d'une réponse immunitaire à *Mycobacterium tuberculosis* chez un sujet, consistant à :
administrer au sujet une quantité pharmaceutiquement efficace du polynucléotide, ledit polynucléotide codant le polypeptide tel que défini dans la revendication 9 ou 10.

12. Polynucléotide isolé destiné à être utilisé selon la revendication 11, le polynucléotide étant lié de manière fonctionnelle à un promoteur.

13. Vecteur destiné à être utilisé dans un procédé de production d'une réponse immunitaire à *Mycobacterium tuberculosis* chez un sujet, consistant à :
administrer au sujet une quantité pharmaceutiquement efficace du polynucléotide dans le vecteur,
ledit vecteur comprenant le polynucléotide tel que défini dans la revendication 11 ou 12.

14. Vecteur destiné à être utilisé selon la revendication 13, le vecteur étant un vecteur plasmide ou un vecteur viral.

15. Vecteur destiné à être utilisé selon la revendication 13, dans lequel le vecteur plasmide est exprimé chez la levure ou dans lequel le vecteur viral est un vecteur rétroviral, orthopox, avipox, fowlpox, capripox, suipox, adénovirus, herpèsvirus, alphavirus, baculovirus, de virus Sindbis, de virus de la vaccine et de poliovirus.

16. Cellule hôte isolée comprenant le vecteur tel que défini dans l'une quelconque des revendications 13 à 15, la cellule hôte étant une cellule eucaryote, une cellule de levure ou une cellule *E*. *coli.*

17. Composition pharmaceutique destinée à être utilisée dans un procédé de production d'une réponse immunitaire à *Mycobacterium tuberculosis* chez un sujet, consistant à :
administrer au sujet une quantité pharmaceutiquement acceptable d'une composition comprenant le polypeptide tel que défini dans la revendication 9 ou 10 et un support pharmaceutiquement acceptable, ou comprenant une quantité pharmaceutiquement efficace du polynucléotide tel que défini dans la revendication 11 ou 12 sur un support pharmaceutiquement acceptable.

18. Polypeptide ou polynucléotide tel que défini dans l'une quelconque des revendications 1 à 5, destiné à être utilisé dans un procédé de traitement d'un sujet infecté par *Mycobacterium tuberculosis,* consistant à administrer au sujet une quantité pharmaceutiquement efficace du polypeptide ou d'un polynucléotide codant le polypeptide, et à traiter ainsi le sujet infecté par *Mycobacterium tuberculosis.*

19. Polypeptide ou polynucléotide destiné à être utilisé selon la revendication 18, dans lequel le sujet infecté par *Mycobacterium tuberculosis* ne présente pas de symptôme de tuberculose.

20. Polypeptide ou polynucléotide destiné à être utilisé selon la revendication 19, dans lequel le traitement du sujet consiste à prévenir le développement de la tuberculose chez le sujet.

21. Polypeptide ou polynucléotide isolé tel que défini dans l'une quelconque des revendications 1 à 5, destiné à être utilisé dans un procédé d'inhibition d'une infection par *Mycobacterium tuberculosis* chez un sujet, consistant à
administrer au sujet une quantité pharmaceutiquement efficace du polypeptide ou du polynucléotide ;
inhiber ainsi l'infection par *Mycobacterium tuberculosis* chez le sujet.

22. Polypeptide ou polynucléotide isolé destiné à être utilisé selon la revendication 21, dans lequel l'administration au sujet de la quantité pharmaceutiquement efficace du polypeptide ou du polynucléotide codant le polypeptide prévient une infection par *Mycobacterium tuberculosis.*

23. Composition pharmaceutique destinée à être utilisée selon la revendication 17, comprenant en outre un adjuvant.


### SEQUENCE LISTING

<110> Oregon Health and Science University David , Lewinsohn M. Deborah, Lewinsohn A.
<120> METHODS FOR PRODUCING AN IMMUNE RESPONSE TO TUBERCULOSIS
<130> 899-77364-02
<150> US 60/782,364
   <151> 2006-03-14
<160> 38
<170> PatentIn version 3.3
<210> 1
   <211> 97
   <212> PRT
   <213> Mycobacterium tuberculosis
<220>
   <221> MISC_FEATURE
   <222> (2) .. (2)
   <223> Xaa can be A or T
<220>
   <221> MISC_FEATURE
   <222> (58) .. (58)
   <223> Xaa can be T or A
<220>
   <221> MISC_FEATURE
   <222> (59) .. (59)
   <223> Xaa any amino acid or no amino acid
<400> 1
   Met Xaa Ser Arg Phe Met Thr Asp Pro His Ala Met Arg Asp Met Ala
   1               5                   10                  15
   Gly Arg Phe Glu Val His Ala Gln Thr Val Glu Asp Glu Ala Arg Arg
               20                  25                  30
   Met Trp Ala Ser Ala Gln Asn Ile Ser Gly Ala Gly Trp Ser Gly Met
           35                  40                  45
   Ala Glu Ala Thr Ser Leu Asp Thr Met Xaa Xaa Met Asn Gln Ala Phe
       50                  55                  60
   Arg Asn Ile Val Asn Met Leu His Gly Val Arg Asp Gly Leu Val Arg
   65                  70                  75                  80
   Asp Ala Asn Asn Tyr Glu Gln Gln Glu Gln Ala Ser Gln Gln Ile Leu
                   85                  90                  95
   Ser
<210> 2
   <211> 97
   <212> PRT
   <213> Mycobacterium tuberculosis
<400> 2
   Met Ala Ser Arg Phe Met Thr Asp Pro His Ala Met Arg Asp Met Ala
   1               5                   10                  15
   Gly Arg Phe Glu Val His Ala Gln Thr Val Glu Asp Glu Ala Arg Arg
               20                  25                  30
   Met Trp Ala Ser Ala Gln Asn Ile Ser Gly Ala Gly Trp Ser Gly Met
           35                  40                  45
   Ala Glu Ala Thr Ser Leu Asp Thr Met Thr Gln Met Asn Gln Ala Phe
       50                  55                  60
   Arg Asn Ile Val Asn Met Leu His Gly Val Arg Asp Gly Leu Val Arg
   65                  70                  75                  80
   Asp Ala Asn Asn Tyr Glu Gln Gln Glu Gln Ala Ser Gln Gln Ile Leu
                   85                  90                  95
   Ser
<210> 3
   <211> 98
   <212> PRT
   <213> Mycobacterium tuberculosis
<400> 3
   Met Ala Ser Arg Phe Met Thr Asp Pro His Ala Met Arg Asp Met Ala
   1 5 10 15
   Gly Arg Phe Glu Val His Ala Gln Thr Val Glu Asp Glu Ala Arg Arg
   20 25 30
   Met Trp Ala Ser Ala Gln Asn Ile Ser Gly Ala Gly Trp Ser Gly Met
   35 40 45
   Ala Glu Ala Thr Ser Leu Asp Thr Met Ala Gln Met Asn Gln Ala Phe
   50 55 60
   Arg Asn Ile Val Asn Met Leu His Gly Val Arg Asp Gly Leu Val Arg
   65 70 75 80
   Asp Ala Asn Asn Tyr Glu Gln Gln Glu Gln Ala Ser Gln Gln Ile Leu
   85 90 95
   Ser Ser
<210> 4
   <211> 97
   <212> PRT
   <213> Mycobacterium tuberculosis
<400> 4
   Met Ala Ser Arg Phe Met Thr Asp Pro His Ala Met Arg Asp Met Ala
   1 5 10 15
   Gly Arg Phe Glu Val His Ala Gln Thr Val Glu Asp Glu Ala Arg Arg
   20 25 30
   Met Trp Ala Ser Ala Gln Asn Ile Ser Gly Ala Gly Trp Ser Gly Met
   35 40 45
   Ala Glu Ala Thr Ser Leu Asp Thr Met Thr Met Asn Gln Ala Phe Arg
   50 55 60
   Asn Ile Val Asn Met Leu His Gly Val Arg Asp Gly Leu Val Arg Asp
   65 70 75 80
   Ala Asn Asn Tyr Glu Gln Gln Glu Gln Ala Ser Gln Gln Ile Leu Ser
   85 90 95
   Ser
<210> 5
   <211> 98
   <212> PRT
   <213> Mycobacterium tuberculosis
<400> 5
   Met Ala Thr Arg Phe Met Thr Asp Pro His Ala Met Arg Asp Met Ala
   1               5                   10                  15
   Gly Arg Phe Glu Val His Ala Gln Thr Val Glu Asp Glu Ala Arg Arg
               20                  25                  30
   Met Trp Ala Ser Ala Gln Asn Ile Ser Gly Ala Gly Trp Ser Gly Met
           35                  40                  45
   Ala Glu Ala Thr Ser Leu Asp Thr Met Ala Gln Met Asn Gln Ala Phe
       50                  55                  60
   Arg Asn Ile Val Asn Met Leu His Gly Val Arg Asp Gly Leu Val Arg
   65                  70                  75                  80
   Asp Ala Asn Asn Tyr Glu Gln Gln Glu Gln Ala Ser Gln Gln Ile Leu
                   85                  90                  95
   Ser Ser
<210> 6
   <211> 98
   <212> PRT
   <213> Mycobacterium tuberculosis
<400> 6
   Met Thr Ser Arg Phe Met Thr Asp Pro His Ala Met Arg Asp Met Ala
   1               5                   10                  15
   Gly Arg Phe Glu Val His Ala Gln Thr Val Glu Asp Glu Ala Arg Arg
               20                  25                  30
   Met Trp Ala Ser Ala Gln Asn Ile Ser Gly Ala Gly Trp Ser Gly Met
           35                  40                  45
   Ala Glu Ala Thr Ser Leu Asp Thr Met Thr Gln Met Asn Gln Ala Phe
       50                  55                  60
   Arg Asn Ile Val Asn Met Leu His Gly Val Arg Asp Gly Leu Val Arg
   65                  70                  75                  80
   Asp Ala Asn Asn Tyr Glu Gln Gln Glu Gln Ala Ser Gln Gln Ile Leu
                   85                  90                  95
   Ser Ser
<210> 7
   <211> 144
   <212> PRT
   <213> Mycobacterium tuberculosis
<400> 7
   Met Ser Tyr Met Ile Ala Thr Pro Ala Ala Leu Thr Ala Ala Ala Thr
   1               5                   10                  15
   Asp Ile Asp Gly Ile Gly Ser Ala Val Ser Val Ala Asn Ala Ala Ala
               20                  25                  30
   Val Ala Ala Thr Thr Gly Val Leu Ala Ala Gly Gly Asp Glu Val Leu
           35                  40                  45
   Ala Ala Ile Ala Arg Leu Phe Asn Ala Asn Ala Glu Glu Tyr His Ala
   50 55 60
   Leu Ser Ala Gln Val Ala Ala Phe Gln Thr Leu Phe Val Arg Thr Leu
   65 70 75 80
   Thr Gly Gly Cys Gly Val Phe Arg Arg Arg Arg Gly Arg Gln Cys Val
   85 90 95
   Thr Ala Ala Glu His Arg Ala Ala Gly Ala Gly Arg Arg Gln Arg Arg
   100 105 110
   Arg Arg Ser Gly Asp Gly Gln Trp Arg Leu Arg Gln Gln Arg His Phe
   115 120 125
   Gly Cys Gly Gly Gln Pro Glu Phe Arg Gln His Ser Glu His Arg Arg
   130 135 140
<210> 8
   <211> 694
   <212> PRT
   <213> Mycobacterium tuberculosis
<400> 8
   Val Ser Leu Val Ile Ala Thr Pro Gln Leu Leu Ala Thr Ala Ala Leu
   1 5 10 15
   Asp Leu Ala Ser Ile Gly Ser Gln Val Ser Ala Ala Asn Ala Ala Ala
   20 25 30
   Ala Met Pro Thr Thr Glu Val Val Ala Ala Ala Ala Asp Glu Val Ser
   35 40 45
   Ala Ala Ile Ala Gly Leu Phe Gly Ala His Ala Arg Gln Tyr Gln Ala
   50 55 60
   Leu Ser Val Gln Val Ala Ala Phe His Glu Gln Phe Val Gln Ala Leu
   65 70 75 80
   Thr Ala Ala Ala Gly Arg Tyr Ala Ser Thr Glu Ala Ala Val Glu Arg
   85 90 95
   Ser Leu Leu Gly Ala Val Asn Ala Pro Thr Glu Ala Leu Leu Gly Arg
   100 105 110
   Pro Leu Ile Gly Asn Gly Ala Asp Gly Thr Ala Pro Gly Gln Pro Gly
   115 120 125
   Ala Ala Gly Gly Leu Leu Phe Gly Asn Gly Gly Asn Gly Ala Ala Gly
       130                 135                 140
   Gly Phe Gly Gln Thr Gly Gly Ser Gly Gly Ala Ala Gly Leu Ile Gly
   145                 150                 155                 160
   Asn Gly Gly Asn Gly Gly Ala Gly Gly Thr Gly Ala Ala Gly Gly Ala
                   165                 170                 175
   Gly Gly Asn Gly Gly Trp Leu Trp Gly Asn Gly Gly Asn Gly Gly Val
               180                 185                 190
   Gly Gly Thr Ser Val Ala Ala Gly Ile Gly Gly Ala Gly Gly Asn Gly
           195                 200                 205
   Gly Asn Ala Gly Leu Phe Gly His Gly Gly Ala Gly Gly Thr Gly Gly
       210                 215                 220
   Ala Gly Leu Ala Gly Ala Asn Gly Val Asn Pro Thr Pro Gly Pro Ala
   225                 230                 235                 240
   Ala Ser Thr Gly Asp Ser Pro Ala Asp Val Ser Gly Ile Gly Asp Gln
                   245                 250                 255
   Thr Gly Gly Asp Gly Gly Thr Gly Gly His Gly Thr Ala Gly Thr Pro
               260                 265                 270
   Thr Gly Gly Thr Gly Gly Asp Gly Ala Thr Ala Thr Ala Gly Ser Gly
           275                 280                 285
   Lys Ala Thr Gly Gly Ala Gly Gly Asp Gly Gly Thr Ala Ala Ala Gly
       290                 295                 300
   Gly Gly Gly Gly Asn Gly Gly Asp Gly Gly Val Ala Gln Gly Asp Ile
   305                 310                 315                 320
   Ala Ser Ala Phe Gly Gly Asp Gly Gly Asn Gly Ser Asp Gly Val Ala
                   325                 330                 335
   Ala Gly Ser Gly Gly Gly Ser Gly Gly Ala Gly Gly Gly Ala Phe Val
               340                 345                 350
   His Ile Ala Thr Ala Thr Ser Thr Gly Gly Ser Gly Gly Phe Gly Gly
           355                 360                 365
   Asn Gly Ala Ala Ser Ala Ala Ser Gly Ala Asp Gly Gly Ala Gly Gly
       370                 375                 380
   Ala Gly Gly Asn Gly Gly Ala Gly Gly Leu Leu Phe Gly Asp Gly Gly
   385 390 395 400
   Asn Gly Gly Ala Gly Gly Ala Gly Gly Ile Gly Gly Asp Gly Ala Thr
   405 410 415
   Gly Gly Pro Gly Gly Ser Gly Gly Asn Ala Gly Ile Ala Arg Phe Asp
   420 425 430
   Ser Pro Asp Pro Glu Ala Glu Pro Asp Val Val Gly Gly Lys Gly Gly
   435 440 445
   Asp Gly Gly Lys Gly Gly Ser Gly Leu Gly Val Gly Gly Ala Gly Gly
   450 455 460
   Thr Gly Gly Ala Gly Gly Asn Gly Gly Ala Gly Gly Leu Leu Phe Gly
   465 470 475 480
   Asn Gly Gly Asn Gly Gly Asn Ala Gly Ala Gly Gly Asp Gly Gly Ala
   485 490 495
   Gly Val Ala Gly Gly Val Gly Gly Asn Gly Gly Gly Gly Gly Thr Ala
   500 505 510
   Thr Phe His Glu Asp Pro Val Ala Gly Val Trp Ala Val Gly Gly Val
   515 520 525
   Gly Gly Asp Gly Gly Ser Gly Gly Ser Ser Leu Gly Val Gly Gly Val
   530 535 540
   Gly Gly Ala Gly Gly Val Gly Gly Lys Gly Gly Ala Ser Gly Met Leu
   545 550 555 560
   Ile Gly Asn Gly Gly Asn Gly Gly Ser Gly Gly Val Gly Gly Ala Gly
   565 570 575
   Gly Val Gly Gly Ala Gly Gly Asp Gly Gly Asn Gly Gly Ser Gly Gly
   580 585 590
   Asn Ala Ser Thr Phe Gly Asp Glu Asn Ser Ile Gly Gly Ala Gly Gly
   595 600 605
   Thr Gly Gly Asn Gly Gly Asn Gly Ala Asn Gly Gly Asn Gly Gly Ala
   610 615 620
   Gly Gly Ile Ala Gly Gly Ala Gly Gly Ser Gly Gly Phe Leu Ser Gly
   625 630 635 640
   Ala Ala Gly Val Ser Gly Ala Asp Gly Ile Gly Gly Ala Gly Gly Ala
   645 650 655
   Gly Gly Ala Gly Gly Ala Gly Gly Ser Gly Gly Glu Ala Gly Ala Gly
   660 665 670
   Gly Leu Thr Asn Gly Pro Gly Ser Pro Gly Val Ser Gly Thr Glu Gly
   675 680 685
   Met Ala Gly Ala Pro Gly
   690
<210> 9
   <211> 325
   <212> PRT
   <213> Mycobacterium tuberculosis
<400> 9
   Met His Gln Val Asp Pro Asn Leu Thr Arg Arg Lys Gly Arg Leu Ala
   1 5 10 15
   Ala Leu Ala Ile Ala Ala Met Ala Ser Ala Ser Leu Val Thr Val Ala
   20 25 30
   Val Pro Ala Thr Ala Asn Ala Asp Pro Glu Pro Ala Pro Pro Val Pro
   35 40 45
   Thr Thr Ala Ala Ser Pro Pro Ser Thr Ala Ala Ala Pro Pro Ala Pro
   50 55 60
   Ala Thr Pro Val Ala Pro Pro Pro Pro Ala Ala Ala Asn Thr Pro Asn
   65 70 75 80
   Ala Gln Pro Gly Asp Pro Asn Ala Ala Pro Pro Pro Ala Asp Pro Asn
   85 90 95
   Ala Pro Pro Pro Pro Val Ile Ala Pro Asn Ala Pro Gln Pro Val Arg
   100 105 110
   Ile Asp Asn Pro Val Gly Gly Phe Ser Phe Ala Leu Pro Ala Gly Trp
   115 120 125
   Val Glu Ser Asp Ala Ala His Phe Asp Tyr Gly Ser Ala Leu Leu Ser
   130 135 140
   Lys Thr Thr Gly Asp Pro Pro Phe Pro Gly Gln Pro Pro Pro Val Ala
   145 150 155 160
   Asn Asp Thr Arg Ile Val Leu Gly Arg Leu Asp Gln Lys Leu Tyr Ala
   165 170 175
   Ser Ala Glu Ala Thr Asp Ser Lys Ala Ala Ala Arg Leu Gly Ser Asp
   180 185 190
   Met Gly Glu Phe Tyr Met Pro Tyr Pro Gly Thr Arg Ile Asn Gln Glu
   195 200 205
   Thr Val Ser Leu Asp Ala Asn Gly Val Ser Gly Ser Ala Ser Tyr Tyr
   210 215 220
   Glu Val Lys Phe Ser Asp Pro Ser Lys Pro Asn Gly Gln Ile Trp Thr
   225 230 235 240
   Gly Val Ile Gly Ser Pro Ala Ala Asn Ala Pro Asp Ala Gly Pro Pro
   245 250 255
   Gln Arg Trp Phe Val Val Trp Leu Gly Thr Ala Asn Asn Pro Val Asp
   260 265 270
   Lys Gly Ala Ala Lys Ala Leu Ala Glu Ser Ile Arg Pro Leu Val Ala
   275 280 285
   Pro Pro Pro Ala Pro Ala Pro Ala Pro Ala Glu Pro Ala Pro Ala Pro
   290 295 300
   Ala Pro Ala Gly Glu Val Ala Pro Thr Pro Thr Thr Pro Thr Pro Gln
   305 310 315 320
   Arg Thr Leu Pro Ala
   325
<210> 10
   <211> 582
   <212> PRT
   <213> Mycobacterium tuberculosis
<400> 10
   Met Leu Leu Ala Leu Leu Arg Gln His Ile Arg Pro Tyr Arg Arg Leu
   1 5 10 15
   Val Ala Met Leu Met Met Leu Gln Leu Val Ser Thr Leu Ala Ser Leu
   20 25 30
   Tyr Leu Pro Thr Val Asn Ala Ala Ile Val Asp Asp Gly Val Ala Lys
   35 40 45
   Gly Asp Thr Ala Thr Ile Val Arg Leu Gly Ala Val Met Leu Gly Val
   50 55 60
   Thr Gly Leu Gln Val Leu Cys Ala Ile Gly Ala Val Tyr Leu Gly Ser
   65 70 75 80
   Arg Thr Gly Ala Gly Phe Gly Arg Asp Leu Arg Ser Ala Met Phe Glu
                   85                  90                  95
   His Ile Ile Thr Phe Ser Glu Arg Glu Thr Ala Arg Phe Gly Ala Pro
               100                 105                 110
   Thr Leu Leu Thr Arg Ser Thr Asn Asp Val Arg Gln Ile Leu Phe Leu
           115                 120                 125
   Val Gln Met Thr Ala Thr Val Leu Val Thr Ala Pro Ile Met Cys Val
       130                 135                 140
   Gly Gly Ile Ile Met Ala Ile His Gln Glu Ala Ala Leu Thr Trp Leu
   145                 150                 155                 160
   Leu Leu Val Ser Val Pro Ile Leu Ala Val Ala Asn Tyr Trp Ile Ile
                   165                 170                 175
   Ser His Met Leu Pro Leu Phe Arg Arg Met Gln Ser Leu Ile Asp Gly
               180                 185                 190
   Ile Asn Arg Val Met Arg Asp Gln Leu Ser Gly Val Arg Val Val Arg
           195                 200                 205
   Ala Phe Thr Arg Glu Gly Tyr Glu Arg Asp Lys Phe Ala Gln Ala Asn
       210                 215                 220
   Thr Ala Leu Ser Asn Ala Ala Leu Ser Ala Gly Asn Trp Gln Ala Leu
   225                 230                 235                 240
   Met Leu Pro Val Thr Thr Leu Thr Ile Asn Ala Ser Ser Val Ala Leu
                   245                 250                 255
   Ile Trp Phe Gly Gly Leu Arg Ile Asp Ser Gly Gln Met Gln Val Gly
               260                 265                 270
   Ser Leu Ile Ala Phe Leu Ser Tyr Phe Ala Gln Ile Leu Met Ala Val
           275                 280                 285
   Leu Met Ala Thr Met Thr Leu Ala Val Leu Pro Arg Ala Ser Val Cys
       290                 295                 300
   Ala Glu Arg Ile Thr Glu Val Leu Ser Thr Pro Ala Ala Leu Gly Asn
   305                 310                 315                 320
   Pro Asp Asn Pro Lys Phe Pro Thr Asp Gly Val Thr Gly Val Val Arg
                   325                 330                 335
   Leu Ala Gly Ala Thr Phe Thr Tyr Pro Gly Ala Asp Cys Pro Val Leu
   340 345 350
   Gln Asp Ile Ser Leu Thr Ala Arg Pro Gly Thr Thr Thr Ala Ile Val
   355 360 365
   Gly Ser Thr Gly Ser Gly Lys Ser Thr Leu Val Ser Leu Ile Cys Arg
   370 375 380
   Leu Tyr Asp Val Thr Ala Gly Ala Val Leu Val Asp Gly Ile Asp Val
   385 390 395 400
   Arg Glu Tyr His Thr Glu Arg Leu Trp Ser Ala Ile Gly Leu Val Pro
   405 410 415
   Gln Arg Ser Tyr Leu Phe Ser Gly Thr Val Ala Asp Asn Leu Arg Tyr
   420 425 430
   Gly Gly Gly Pro Asp Gln Val Val Thr Glu Gln Glu Met Trp Glu Ala
   435 440 445
   Leu Arg Val Ala Ala Ala Asp Gly Phe Val Gln Thr Asp Gly Leu Gln
   450 455 460
   Thr Arg Val Ala Gln Gly Gly Val Asn Phe Ser Gly Gly Gln Arg Gln
   465 470 475 480
   Arg Leu Ala Ile Ala Arg Ala Val Ile Arg Arg Pro Ala Ile Tyr Val
   485 490 495
   Phe Asp Asp Ala Phe Ser Ala Leu Asp Val His Thr Asp Ala Lys Val
   500 505 510
   His Ala Ser Leu Arg Gln Val Ser Gly Asp Ala Thr Ile Ile Val Val
   515 520 525
   Thr Gln Arg Ile Ser Asn Ala Ala Gln Ala Asp Gln Val Ile Val Val
   530 535 540
   Asp Asn Gly Lys Ile Val Gly Thr Gly Thr His Glu Thr Leu Leu Ala
   545 550 555 560
   Asp Cys Pro Thr Tyr Ala Glu Phe Ala Ala Ser Gln Ser Leu Ser Ala
   565 570 575
   Thr Val Gly Gly Val Gly
   580
<210> 11
   <211> 468
   <212> PRT
   <213> Mycobacterium tuberculosis
<400> 11
   Met Ser Tyr Val Ile Ala Ala Pro Glu Met Leu Ala Thr Thr Ala Ala
   1               5                   10                  15
   Asp Val Asp Gly Ile Gly Ser Ala Ile Arg Ala Ala Ser Ala Ser Ala
               20                  25                  30
   Ala Gly Pro Thr Thr Gly Leu Leu Ala Ala Ala Ala Asp Glu Val Ser
           35                  40                  45
   Ser Ala Ala Ala Ala Leu Phe Ser Glu Tyr Ala Arg Glu Cys Gln Glu
       50                  55                  60
   Val Leu Lys Gln Ala Ala Ala Phe His Gly Glu Phe Thr Arg Ala Leu
   65                  70                  75                  80
   Ala Ala Ala Gly Ala Ala Tyr Ala Gln Ala Glu Ala Ser Asn Thr Ala
                   85                  90                  95
   Ala Met Ser Gly Thr Ala Gly Ser Ser Gly Ala Leu Gly Ser Val Gly
               100                 105                 110
   Met Leu Ser Gly Asn Pro Leu Thr Ala Leu Met Met Gly Gly Thr Gly
           115                 120                 125
   Glu Pro Ile Leu Ser Asp Arg Val Leu Ala Ile Ile Asp Ser Ala Tyr
       130                 135                 140
   Ile Arg Pro Ile Phe Gly Pro Asn Asn Pro Val Ala Gln Tyr Thr Pro
   145                 150                 155                 160
   Glu Gln Trp Trp Pro Phe Ile Gly Asn Leu Ser Leu Asp Gln Ser Ile
                   165                 170                 175
   Ala Gln Gly Val Thr Leu Leu Asn Asn Gly Ile Asn Ala Glu Leu Gln
               180                 185                 190
   Asn Gly His Asp Val Val Val Phe Gly Tyr Ser Gln Ser Ala Ala Val
           195                 200                 205
   Ala Thr Asn Glu Ile Arg Ala Leu Met Ala Leu Pro Pro Gly Gln Ala
       210                 215                 220
   Pro Asp Pro Ser Arg Leu Ala Phe Thr Leu Ile Gly Asn Ile Asn Asn
   225                 230                 235                 240
   Pro Asn Gly Gly Val Leu Glu Arg Tyr Val Gly Leu Tyr Leu Pro Phe
   245 250 255
   Leu Asp Met Ser Phe Asn Gly Ala Thr Pro Pro Asp Ser Pro Tyr Gln
   260 265 270
   Thr Tyr Met Tyr Thr Gly Gln Tyr Asp Gly Tyr Ala His Asn Pro Gln
   275 280 285
   Tyr Pro Leu Asn Ile Leu Ser Asp Leu Asn Ala Phe Met Gly Ile Arg
   290 295 300
   Trp Val His Asn Ala Tyr Pro Phe Thr Ala Ala Glu Val Ala Asn Ala
   305 310 315 320
   Val Pro Leu Pro Thr Ser Pro Gly Tyr Thr Gly Asn Thr His Tyr Tyr
   325 330 335
   Met Phe Leu Thr Gln Asp Leu Pro Leu Leu Gln Pro Ile Arg Ala Ile
   340 345 350
   Pro Phe Val Gly Thr Pro Ile Ala Glu Leu Ile Gln Pro Asp Leu Arg
   355 360 365
   Val Leu Val Asp Leu Gly Tyr Gly Tyr Gly Tyr Ala Asp Val Pro Thr
   370 375 380
   Pro Ala Ser Leu Phe Ala Pro Ile Asn Pro Ile Ala Val Ala Ser Ala
   385 390 395 400
   Leu Ala Thr Gly Thr Val Gln Gly Pro Gln Ala Ala Leu Val Ser Ile
   405 410 415
   Gly Leu Leu Pro Gln Ser Ala Leu Pro Asn Thr Tyr Pro Tyr Leu Pro
   420 425 430
   Ser Ala Asn Pro Gly Leu Met Phe Asn Phe Gly Gln Ser Ser Val Thr
   435 440 445
   Glu Leu Ser Val Leu Ser Gly Ala Leu Gly Ser Val Ala Arg Leu Ile
   450 455 460
   Pro Pro Ile Ala
   465
<210> 12
   <211> 3716
   <212> PRT
   <213> Mycobacterium tuberculosis
<400> 12
   Met Glu Phe Pro Val Leu Pro Pro Glu Ile Asn Ser Val Leu Met Tyr
   1               5                   10                  15
   Ser Gly Ala Gly Ser Ser Pro Leu Leu Ala Ala Ala Ala Ala Trp Asp
               20                  25                  30
   Gly Leu Ala Glu Glu Leu Gly Ser Ala Ala Val Ser Phe Gly Gln Val
           35                  40                  45
   Thr Ser Gly Leu Thr Ala Gly Val Trp Gln Gly Ala Ala Ala Ala Ala
       50                  55                  60
   Met Ala Ala Ala Ala Ala Pro Tyr Ala Gly Trp Leu Gly Ser Val Ala
   65                  70                  75                  80
   Ala Ala Ala Glu Ala Val Ala Gly Gln Ala Arg Val Val Val Gly Val
                   85                  90                  95
   Phe Glu Ala Ala Leu Ala Ala Thr Val Asp Pro Ala Leu Val Ala Ala
               100                 105                 110
   Asn Arg Ala Arg Leu Val Ala Leu Ala Val Ser Asn Leu Leu Gly Gln
           115                 120                 125
   Asn Thr Pro Ala Ile Ala Ala Ala Glu Ala Glu Tyr Glu Leu Met Trp
       130                 135                 140
   Ala Ala Asp Val Ala Ala Met Ala Gly Tyr His Ser Gly Ala Ser Ala
   145                 150                 155                 160
   Ala Ala Ala Ala Leu Pro Ala Phe Ser Pro Pro Ala Gln Ala Leu Gly
                   165                 170                 175
   Gly Gly Val Gly Ala Phe Leu Thr Ala Leu Phe Ala Ser Pro Ala Lys
               180                 185                 190
   Ala Leu Ser Leu Asn Ala Gly Leu Gly Asn Val Gly Asn Tyr Asn Val
           195                 200                 205
   Gly Leu Gly Asn Val Gly Val Phe Asn Leu Gly Ala Gly Asn Val Gly
       210                 215                 220
   Gly Gln Asn Leu Gly Phe Gly Asn Ala Gly Gly Thr Asn Val Gly Phe
   225                 230                 235                 240
   Gly Asn Leu Gly Asn Gly Asn Val Gly Phe Gly Asn Ser Gly Leu Gly
                   245                 250                 255
   Ala Gly Leu Ala Gly Leu Gly Asn Ile Gly Leu Gly Asn Ala Gly Ser
               260                 265                 270
   Ser Asn Tyr Gly Phe Ala Asn Leu Gly Val Gly Asn Ile Gly Phe Gly
           275                 280                 285
   Asn Thr Gly Thr Asn Asn Val Gly Val Gly Leu Thr Gly Asn His Leu
       290                 295                 300
   Thr Gly Ile Gly Gly Leu Asn Ser Gly Thr Gly Asn Ile Gly Leu Phe
   305                 310                 315                 320
   Asn Ser Gly Thr Gly Asn Val Gly Phe Phe Asn Ser Gly Thr Gly Asn
                   325                 330                 335
   Phe Gly Val Phe Asn Ser Gly Asn Tyr Asn Thr Gly Val Gly Asn Ala
               340                 345                 350
   Gly Thr Ala Ser Thr Gly Leu Phe Asn Ala Gly Asn Phe Asn Thr Gly
           355                 360                 365
   Val Val Asn Val Gly Ser Tyr Asn Thr Gly Ser Phe Asn Ala Gly Asp
       370                 375                 380
   Thr Asn Thr Gly Gly Phe Asn Pro Gly Gly Val Asn Thr Gly Trp Leu
   385                 390                 395                 400
   Asn Thr Gly Asn Thr Asn Thr Gly Ile Ala Asn Ser Gly Asn Val Asn
                   405                 410                 415
   Thr Gly Ala Phe Ile Ser Gly Asn Phe Asn Asn Gly Val Leu Trp Val
               420                 425                 430
   Gly Asp Tyr Gln Gly Leu Phe Gly Val Ser Ala Gly Ser Ser Ile Pro
           435                 440                 445
   Ala Ile Pro Ile Gly Leu Val Leu Asn Gly Asp Ile Gly Pro Ile Thr
       450                 455                 460
   Ile Gln Pro Ile Pro Ile Leu Pro Thr Ile Pro Leu Ser Ile His Gln
   465                 470                 475                 480
   Thr Val Asn Leu Gly Pro Leu Val Val Pro Asp Ile Val Ile Pro Ala
                   485                 490                 495
   Phe Gly Gly Gly Ile Gly Ile Pro Ile Asn Ile Gly Pro Leu Thr Ile
               500                 505                 510
   Thr Pro Ile Thr Leu Phe Ala Gln Gln Thr Phe Val Asn Gln Leu Pro
           515                 520                 525
   Phe Pro Thr Phe Ser Leu Gly Lys Ile Thr Ile Pro Gln Ile Gln Thr
       530                 535                 540
   Phe Asp Ser Asn Gly Gln Leu Val Ser Phe Ile Gly Pro Ile Val Ile
   545                 550                 555                 560
   Asp Thr Thr Ile Pro Gly Pro Thr Asn Pro Gln Ile Asp Leu Thr Ile
                   565                 570                 575
   Arg Trp Asp Thr Pro Pro Ile Thr Leu Phe Pro Asn Gly Ile Ser Ala
               580                 585                 590
   Pro Asp Asn Pro Leu Gly Leu Leu Val Ser Val Ser Ile Ser Asn Pro
           595                 600                 605
   Gly Phe Thr Ile Pro Gly Phe Ser Val Pro Ala Gln Pro Leu Pro Leu
       610                 615                 620
   Ser Ile Asp Ile Glu Gly Gln Ile Asp Gly Phe Ser Thr Pro Pro Ile
   625                 630                 635                 640
   Thr Ile Asp Arg Ile Pro Leu Thr Val Gly Gly Gly Val Thr Ile Gly
                   645                 650                 655
   Pro Ile Thr Ile Gln Gly Leu His Ile Pro Ala Ala Pro Gly Val Gly
               660                 665                 670
   Asn Thr Thr Thr Ala Pro Ser Ser Gly Phe Phe Asn Ser Gly Ala Gly
           675                 680                 685
   Gly Val Ser Gly Phe Gly Asn Val Gly Ala Gly Ser Ser Gly Trp Trp
       690                 695                 700
   Asn Gln Ala Pro Ser Ala Leu Leu Gly Ala Gly Ser Gly Val Gly Asn
   705                 710                 715                 720
   Val Gly Thr Leu Gly Ser Gly Val Leu Asn Leu Gly Ser Gly Ile Ser
                   725                 730                 735
   Gly Phe Tyr Asn Thr Ser Val Leu Pro Phe Gly Thr Pro Ala Ala Val
               740                 745                 750
   Ser Gly Ile Gly Asn Leu Gly Gln Gln Leu Ser Gly Val Ser Ala Ala
           755                 760                 765
   Gly Thr Thr Leu Arg Ser Met Leu Ala Gly Asn Leu Gly Leu Ala Asn
   770 775 780
   Val Gly Asn Phe Asn Thr Gly Phe Gly Asn Val Gly Asp Val Asn Leu
   785 790 795 800
   Gly Ala Ala Asn Ile Gly Gly His Asn Leu Gly Leu Gly Asn Val Gly
   805 810 815
   Asp Gly Asn Leu Gly Leu Gly Asn Ile Gly His Gly Asn Leu Gly Phe
   820 825 830
   Ala Asn Leu Gly Leu Thr Ala Gly Ala Ala Gly Val Gly Asn Val Gly
   835 840 845
   Phe Gly Asn Ala Gly Ile Asn Asn Tyr Gly Leu Ala Asn Met Gly Val
   850 855 860
   Gly Asn Ile Gly Phe Ala Asn Thr Gly Thr Gly Asn Ile Gly Ile Gly
   865 870 875 880
   Leu Val Gly Asp His Arg Thr Gly Ile Gly Gly Leu Asn Ser Gly Ile
   885 890 895
   Gly Asn Ile Gly Leu Phe Asn Ser Gly Thr Gly Asn Val Gly Phe Phe
   900 905 910
   Asn Ser Gly Thr Gly Asn Phe Gly Ile Gly Asn Ser Gly Arg Phe Asn
   915 920 925
   Thr Gly Ile Gly Asn Ser Gly Thr Ala Ser Thr Gly Leu Phe Asn Ala
   930 935 940
   Gly Ser Phe Ser Thr Gly Ile Ala Asn Thr Gly Asp Tyr Asn Thr Gly
   945 950 955 960
   Ser Phe Asn Ala Gly Asp Thr Asn Thr Gly Gly Phe Asn Pro Gly Gly
   965 970 975
   Ile Asn Thr Gly Trp Phe Asn Thr Gly His Ala Asn Thr Gly Leu Ala
   980 985 990
   Asn Ala Gly Thr Phe Gly Thr Gly Ala Phe Met Thr Gly Asp Tyr Ser
   995 1000 1005
   Asn Gly Leu Leu Trp Arg Gly Gly Tyr Glu Gly Leu Val Gly Val
   1010 1015 1020
   Arg Val Gly Pro Thr Ile Ser Gln Phe Pro Val Thr Val His Ala
   1025 1030 1035
   Ile Gly Gly Val Gly Pro Leu His Val Ala Pro Val Pro Val Pro
   1040 1045 1050
   Ala Val His Val Glu Ile Thr Asp Ala Thr Val Gly Leu Gly Pro
   1055 1060 1065
   Phe Thr Val Pro Pro Ile Ser Ile Pro Ser Leu Pro Ile Ala Ser
   1070 1075 1080
   Ile Thr Gly Ser Val Asp Leu Ala Ala Asn Thr Ile Ser Pro Ile
   1085 1090 1095
   Arg Ala Leu Asp Pro Leu Ala Gly Ser Ile Gly Leu Phe Leu Glu
   1100 1105 1110
   Pro Phe Arg Leu Ser Asp Pro Phe Ile Thr Ile Asp Ala Phe Gln
   1115 1120 1125
   Val Val Ala Gly Val Leu Phe Leu Glu Asn Ile Ile Val Pro Gly
   1130 1135 1140
   Leu Thr Val Ser Gly Gln Ile Leu Val Thr Pro Thr Pro Ile Pro
   1145 1150 1155
   Leu Thr Leu Asn Leu Asp Thr Thr Pro Trp Thr Leu Phe Pro Asn
   1160 1165 1170
   Gly Phe Thr Ile Pro Ala Gln Thr Pro Val Thr Val Gly Met Glu
   1175 1180 1185
   Val Ala Asn Asp Gly Phe Thr Phe Phe Pro Gly Gly Leu Thr Phe
   1190 1195 1200
   Pro Arg Ala Ser Ala Gly Val Thr Gly Leu Ser Val Gly Leu Asp
   1205 1210 1215
   Ala Phe Thr Leu Leu Pro Asp Gly Phe Thr Leu Asp Thr Val Pro
   1220 1225 1230
   Ala Thr Phe Asp Gly Thr Ile Leu Ile Gly Asp Ile Pro Ile Pro
   1235 1240 1245
   Ile Ile Asp Val Pro Ala Val Pro Gly Phe Gly Asn Thr Thr Thr
   1250 1255 1260
   Ala Pro Ser Ser Gly Phe Phe Asn Thr Gly Gly Gly Gly Gly Ser
   1265 1270 1275
   Gly Phe Ala Asn Val Gly Ala Gly Thr Ser Gly Trp Trp Asn Gln
   1280 1285 1290
   Gly His Asp Val Leu Ala Gly Ala Gly Ser Gly Val Ala Asn Ala
   1295 1300 1305
   Gly Thr Leu Ser Ser Gly Val Leu Asn Val Gly Ser Gly Ile Ser
   1310 1315 1320
   Gly Trp Tyr Asn Thr Ser Thr Leu Gly Ala Gly Thr Pro Ala Val
   1325 1330 1335
   Val Ser Gly Ile Gly Asn Leu Gly Gln Gln Leu Ser Gly Phe Leu
   1340 1345 1350
   Ala Asn Gly Thr Val Leu Asn Arg Ser Pro Ile Val Asn Ile Gly
   1355 1360 1365
   Trp Ala Asp Val Gly Ala Phe Asn Thr Gly Leu Gly Asn Val Gly
   1370 1375 1380
   Asp Leu Asn Trp Gly Ala Ala Asn Ile Gly Ala Gln Asn Leu Gly
   1385 1390 1395
   Leu Gly Asn Leu Gly Ser Gly Asn Val Gly Phe Gly Asn Ile Gly
   1400 1405 1410
   Ala Gly Asn Val Gly Phe Ala Asn Ser Gly Pro Ala Val Gly Leu
   1415 1420 1425
   Ala Gly Leu Gly Asn Val Gly Leu Ser Asn Ala Gly Ser Asn Asn
   1430 1435 1440
   Trp Gly Leu Ala Asn Leu Gly Val Gly Asn Ile Gly Leu Ala Asn
   1445 1450 1455
   Thr Gly Thr Gly Asn Ile Gly Ile Gly Leu Val Gly Asp Tyr Gln
   1460 1465 1470
   Thr Gly Ile Gly Gly Leu Asn Ser Gly Ser Gly Asn Ile Gly Leu
   1475 1480 1485
   Phe Asn Ser Gly Thr Gly Asn Val Gly Phe Phe Asn Thr Gly Thr
   1490 1495 1500
   Gly Asn Phe Gly Leu Phe Asn Ser Gly Ser Phe Asn Thr Gly Ile
   1505 1510 1515
   Gly Asn Ser Gly Thr Gly Ser Thr Gly Leu Phe Asn Ala Gly Asn
   1520 1525 1530
   Phe Asn Thr Gly Ile Ala Asn Pro Gly Ser Tyr Asn Thr Gly Ser
   1535 1540 1545
   Phe Asn Val Gly Asp Thr Asn Thr Gly Gly Phe Asn Pro Gly Asp
   1550 1555 1560
   Ile Asn Thr Gly Trp Phe Asn Thr Gly Ile Met Asn Thr Gly Thr
   1565 1570 1575
   Arg Asn Thr Gly Ala Leu Met Ser Gly Thr Asp Ser Asn Gly Met
   1580 1585 1590
   Leu Trp Arg Gly Asp His Glu Gly Leu Phe Gly Leu Ser Tyr Gly
   1595 1600 1605
   Ile Thr Ile Pro Gln Phe Pro Ile Arg Ile Thr Thr Thr Gly Gly
   1610 1615 1620
   Ile Gly Pro Ile Val Ile Pro Asp Thr Thr Ile Leu Pro Pro Leu
   1625 1630 1635
   His Leu Gln Ile Thr Gly Asp Ala Asp Tyr Ser Phe Thr Val Pro
   1640 1645 1650
   Asp Ile Pro Ile Pro Ala Ile His Ile Gly Ile Asn Gly Val Val
   1655 1660 1665
   Thr Val Gly Phe Thr Ala Pro Glu Ala Thr Leu Leu Ser Ala Leu
   1670 1675 1680
   Lys Asn Asn Gly Ser Phe Ile Ser Phe Gly Pro Ile Thr Leu Ser
   1685 1690 1695
   Asn Ile Asp Ile Pro Pro Met Asp Phe Thr Leu Gly Leu Pro Val
   1700 1705 1710
   Leu Gly Pro Ile Thr Gly Gln Leu Gly Pro Ile His Leu Glu Pro
   1715 1720 1725
   Ile Val Val Ala Gly Ile Gly Val Pro Leu Glu Ile Glu Pro Ile
   1730 1735 1740
   Pro Leu Asp Ala Ile Ser Leu Ser Glu Ser Ile Pro Ile Arg Ile
   1745 1750 1755
   Pro Val Asp Ile Pro Ala Ser Val Ile Asp Gly Ile Ser Met Ser
   1760 1765 1770
   Glu Val Val Pro Ile Asp Ala Ser Val Asp Ile Pro Ala Val Thr
   1775 1780 1785
   Ile Thr Gly Thr Thr Ile Ser Ala Ile Pro Leu Gly Phe Asp Ile
   1790 1795 1800
   Arg Thr Ser Ala Gly Pro Leu Asn Ile Pro Ile Ile Asp Ile Pro
   1805 1810 1815
   Ala Ala Pro Gly Phe Gly Asn Ser Thr Gln Met Pro Ser Ser Gly
   1820 1825 1830
   Phe Phe Asn Thr Gly Ala Gly Gly Gly Ser Gly Ile Gly Asn Leu
   1835 1840 1845
   Gly Ala Gly Val Ser Gly Leu Leu Asn Gln Ala Gly Ala Gly Ser
   1850 1855 1860
   Leu Val Gly Thr Leu Ser Gly Leu Gly Asn Ala Gly Thr Leu Ala
   1865 1870 1875
   Ser Gly Val Leu Asn Ser Gly Thr Ala Ile Ser Gly Leu Phe Asn
   1880 1885 1890
   Val Ser Thr Leu Asp Ala Thr Thr Pro Ala Val Ile Ser Gly Phe
   1895 1900 1905
   Ser Asn Leu Gly Asp His Met Ser Gly Val Ser Ile Asp Gly Leu
   1910 1915 1920
   Ile Ala Ile Leu Thr Phe Pro Pro Ala Glu Ser Val Phe Asp Gln
   1925 1930 1935
   Ile Ile Asp Ala Ala Ile Ala Glu Leu Gln His Leu Asp Ile Gly
   1940 1945 1950
   Asn Ala Leu Ala Leu Gly Asn Val Gly Gly Val Asn Leu Gly Leu
   1955 1960 1965
   Ala Asn Val Gly Glu Phe Asn Leu Gly Ala Gly Asn Val Gly Asn
   1970 1975 1980
   Ile Asn Val Gly Ala Gly Asn Leu Gly Gly Ser Asn Leu Gly Leu
   1985 1990 1995
   Gly Asn  Val Gly Thr Gly Asn  Leu Gly Phe Gly Asn  Ile Gly Ala
       2000                 2005                 2010
   Gly Asn  Phe Gly Phe Gly Asn  Ala Gly Leu Thr Ala  Gly Ala Gly
       2015                 2020                 2025
   Gly Leu  Gly Asn Val Gly Leu  Gly Asn Ala Gly Ser  Gly Ser Trp
       2030                 2035                 2040
   Gly Leu  Ala Asn Val Gly Val  Gly Asn Ile Gly Leu  Ala Asn Thr
       2045                 2050                 2055
   Gly Thr  Gly Asn Ile Gly Ile  Gly Leu Thr Gly Asp  Tyr Arg Thr
       2060                 2065                 2070
   Gly Ile  Gly Gly Leu Asn Ser  Gly Thr Gly Asn Leu  Gly Leu Phe
       2075                 2080                 2085
   Asn Ser  Gly Thr Gly Asn Ile  Gly Phe Phe Asn Thr  Gly Thr Gly
       2090                 2095                 2100
   Asn Phe  Gly Leu Phe Asn Ser  Gly Ser Tyr Ser Thr  Gly Val Gly
       2105                 2110                 2115
   Asn Ala  Gly Thr Ala Ser Thr  Gly Leu Phe Asn Ala  Gly Asn Phe
       2120                 2125                 2130
   Asn Thr  Gly Leu Ala Asn Ala  Gly Ser Tyr Asn Thr  Gly Ser Leu
       2135                 2140                 2145
   Asn Val  Gly Ser Phe Asn Thr  Gly Gly Val Asn Pro  Gly Thr Val
       2150                 2155                 2160
   Asn Thr  Gly Trp Phe Asn Thr  Gly His Thr Asn Thr  Gly Leu Phe
       2165                 2170                 2175
   Asn Thr  Gly Asn Val Asn Thr  Gly Ala Phe Asn Ser  Gly Ser Phe
       2180                 2185                 2190
   Asn Asn  Gly Ala Leu Trp Thr  Gly Asp Tyr His Gly  Leu Val Gly
       2195                 2200                 2205
   Phe Ser  Phe Ser Ile Asp Ile  Ala Gly Ser Thr Leu  Leu Asp Leu
       2210                 2215                 2220
   Asn Glu  Thr Leu Asn Leu Gly  Pro Ile His Ile Glu  Gln Ile Asp
       2225                 2230                 2235
   Ile Pro  Gly Met Ser Leu Phe  Asp Val His Glu Ile  Val Glu Ile
   2240 2245 2250
   Gly Pro Phe Thr Ile Pro Gln Val Asp Val Pro Ala Ile Pro Leu
   2255 2260 2265
   Glu Ile His Glu Ser Ile His Met Asp Pro Ile Val Leu Val Pro
   2270 2275 2280
   Ala Thr Thr Ile Pro Ala Gln Thr Arg Thr Ile Pro Leu Asp Ile
   2285 2290 2295
   Pro Ala Ser Pro Gly Ser Thr Met Thr Leu Pro Leu Ile Ser Met
   2300 2305 2310
   Arg Phe Glu Gly Glu Asp Trp Ile Leu Gly Ser Thr Ala Ala Ile
   2315 2320 2325
   Pro Asn Phe Gly Asp Pro Phe Pro Ala Pro Thr Gln Gly Ile Thr
   2330 2335 2340
   Ile His Thr Gly Pro Gly Pro Gly Thr Thr Gly Glu Leu Lys Ile
   2345 2350 2355
   Ser Ile Pro Gly Phe Glu Ile Pro Gln Ile Ala Thr Thr Arg Phe
   2360 2365 2370
   Leu Leu Asp Val Asn Ile Ser Gly Gly Leu Pro Ala Phe Thr Leu
   2375 2380 2385
   Phe Ala Gly Gly Leu Thr Ile Pro Thr Asn Ala Ile Pro Leu Thr
   2390 2395 2400
   Ile Asp Ala Ser Gly Ala Leu Asp Pro Ile Thr Ile Phe Pro Gly
   2405 2410 2415
   Gly Tyr Thr Ile Asp Pro Leu Pro Leu His Leu Ala Leu Asn Leu
   2420 2425 2430
   Thr Val Pro Asp Ser Ser Ile Pro Ile Ile Asp Val Pro Pro Thr
   2435 2440 2445
   Pro Gly Phe Gly Asn Thr Thr Ala Thr Pro Ser Ser Gly Phe Phe
   2450 2455 2460
   Asn Ser Gly Ala Gly Gly Val Ser Gly Phe Gly Asn Val Gly Ser
   2465 2470 2475
   Asn Leu Ser Gly Trp Trp Asn Gln Ala Ala Ser Ala Leu Ala Gly
   2480 2485 2490
   Ser Gly Ser Gly Val Leu Asn Val Gly Thr Leu Gly Ser Gly Val
   2495 2500 2505
   Leu Asn Val Gly Ser Gly Val Ser Gly Ile Tyr Asn Thr Ser Val
   2510 2515 2520
   Leu Pro Leu Gly Thr Pro Ala Val Leu Ser Gly Leu Gly Asn Val
   2525 2530 2535
   Gly His Gln Leu Ser Gly Val Ser Ala Ala Gly Thr Ala Leu Asn
   2540 2545 2550
   Gln Ile Pro Ile Leu Asn Ile Gly Leu Ala Asp Val Gly Asn Phe
   2555 2560 2565
   Asn Val Gly Phe Gly Asn Val Gly Asp Val Asn Leu Gly Ala Ala
   2570 2575 2580
   Asn Leu Gly Ala Gln Asn Leu Gly Leu Gly Asn Val Gly Thr Gly
   2585 2590 2595
   Asn Leu Gly Phe Ala Asn Val Gly His Gly Asn Ile Gly Phe Gly
   2600 2605 2610
   Asn Ser Gly Leu Thr Ala Gly Ala Ala Gly Leu Gly Asn Thr Gly
   2615 2620 2625
   Phe Gly Asn Ala Gly Ser Ala Asn Tyr Gly Phe Ala Asn Gln Gly
   2630 2635 2640
   Val Arg Asn Ile Gly Leu Ala Asn Thr Gly Thr Gly Asn Ile Gly
   2645 2650 2655
   Ile Gly Leu Val Gly Asp Asn Leu Thr Gly Ile Gly Gly Leu Asn
   2660 2665 2670
   Ser Gly Ala Gly Asn Ile Gly Leu Phe Asn Ser Gly Thr Gly Asn
   2675 2680 2685
   Ile Gly Phe Phe Asn Ser Gly Thr Gly Asn Phe Gly Ile Gly Asn
   2690 2695 2700
   Ser Gly Ser Phe Asn Thr Gly Ile Gly Asn Ser Gly Thr Gly Ser
   2705 2710 2715
   Thr Gly Leu Phe Asn Ala Gly Ser Phe Asn Thr Gly Val Ala Asn
   2720 2725 2730
   Ala Gly Ser Tyr Asn Thr Gly Ser Phe Asn Ala Gly Asp Thr Asn
       2735                2740                2745
   Thr Gly Gly Phe Asn Pro Gly Thr Ile Asn Thr Gly Trp Phe Asn
       2750                2755                2760
   Thr Gly His Thr Asn Thr Gly Ile Ala Asn Ser Gly Asn Val Gly
       2765                2770                2775
   Thr Gly Ala Phe Met Ser Gly Asn Phe Ser Asn Gly Leu Leu Trp
       2780                2785                2790
   Arg Gly Asp His Glu Gly Leu Phe Ser Leu Phe Tyr Ser Leu Asp
       2795                2800                2805
   Val Pro Arg Ile Thr Ile Val Asp Ala His Leu Asp Gly Gly Phe
       2810                2815                2820
   Gly Pro Val Val Leu Pro Pro Ile Pro Val Pro Ala Val Asn Ala
       2825                2830                2835
   His Leu Thr Gly Asn Val Ala Met Gly Ala Phe Thr Ile Pro Gln
       2840                2845                2850
   Ile Asp Ile Pro Ala Leu Thr Pro Asn Ile Thr Gly Ser Ala Ala
       2855                2860                2865
   Phe Arg Ile Val Val Gly Ser Val Arg Ile Pro Pro Val Ser Val
       2870                2875                2880
   Ile Val Glu Gln Ile Ile Asn Ala Ser Val Gly Ala Glu Met Arg
       2885                2890                2895
   Ile Asp Pro Phe Glu Met Trp Thr Gln Gly Thr Asn Gly Leu Gly
       2900                2905                2910
   Ile Thr Phe Tyr Ser Phe Gly Ser Ala Asp Gly Ser Pro Tyr Ala
       2915                2920                2925
   Thr Gly Pro Leu Val Phe Gly Ala Gly Thr Ser Asp Gly Ser His
       2930                2935                2940
   Leu Thr Ile Ser Ala Ser Ser Gly Ala Phe Thr Thr Pro Gln Leu
       2945                2950                2955
   Glu Thr Gly Pro Ile Thr Leu Gly Phe Gln Val Pro Gly Ser Val
       2960                2965                2970
   Asn Ala Ile Thr Leu Phe Pro Gly Gly Leu Thr Phe Pro Ala Thr
   2975 2980 2985
   Ser Leu Leu Asn Leu Asp Val Thr Ala Gly Ala Gly Gly Val Asp
   2990 2995 3000
   Ile Pro Ala Ile Thr Trp Pro Glu Ile Ala Ala Ser Ala Asp Gly
   3005 3010 3015
   Ser Val Tyr Val Leu Ala Ser Ser Ile Pro Leu Ile Asn Ile Pro
   3020 3025 3030
   Pro Thr Pro Gly Ile Gly Asn Ser Thr Ile Thr Pro Ser Ser Gly
   3035 3040 3045
   Phe Phe Asn Ala Gly Ala Gly Gly Gly Ser Gly Phe Gly Asn Phe
   3050 3055 3060
   Gly Ala Gly Thr Ser Gly Trp Trp Asn Gln Ala His Thr Ala Leu
   3065 3070 3075
   Ala Gly Ala Gly Ser Gly Phe Ala Asn Val Gly Thr Leu His Ser
   3080 3085 3090
   Gly Val Leu Asn Leu Gly Ser Gly Val Ser Gly Ile Tyr Asn Thr
   3095 3100 3105
   Ser Thr Leu Gly Val Gly Thr Pro Ala Leu Val Ser Gly Leu Gly
   3110 3115 3120
   Asn Val Gly His Gln Leu Ser Gly Leu Leu Ser Gly Gly Ser Ala
   3125 3130 3135
   Val Asn Pro Val Thr Val Leu Asn Ile Gly Leu Ala Asn Val Gly
   3140 3145 3150
   Ser His Asn Ala Gly Phe Gly Asn Val Gly Glu Val Asn Leu Gly
   3155 3160 3165
   Ala Ala Asn Leu Gly Ala His Asn Leu Gly Phe Gly Asn Ile Gly
   3170 3175 3180
   Ala Gly Asn Leu Gly Phe Gly Asn Ile Gly His Gly Asn Val Gly
   3185 3190 3195
   Val Gly Asn Ser Gly Leu Thr Ala Gly Val Pro Gly Leu Gly Asn
   3200 3205 3210
   Val Gly Leu Gly Asn Ala Gly Gly Asn Asn Trp Gly Leu Ala Asn
   3215 3220 3225
   Val Gly Val Gly Asn Ile Gly Leu Ala Asn Thr Gly Thr Gly Asn
   3230 3235 3240
   Ile Gly Ile Gly Leu Thr Gly Asp Tyr Gln Thr Gly Ile Gly Gly
   3245 3250 3255
   Leu Asn Ser Gly Ala Gly Asn Leu Gly Leu Phe Asn Ser Gly Ala
   3260 3265 3270
   Gly Asn Val Gly Phe Phe Asn Thr Gly Thr Gly Asn Phe Gly Leu
   3275 3280 3285
   Phe Asn Ser Gly Ser Phe Asn Thr Gly Val Gly Asn Ser Gly Thr
   3290 3295 3300
   Gly Ser Thr Gly Leu Phe Asn Ala Gly Ser Phe Asn Thr Gly Val
   3305 3310 3315
   Ala Asn Ala Gly Ser Tyr Asn Thr Gly Ser Phe Asn Val Gly Asp
   3320 3325 3330
   Thr Asn Thr Gly Gly Phe Asn Pro Gly Ser Ile Asn Thr Gly Trp
   3335 3340 3345
   Leu Asn Ala Gly Asn Ala Asn Thr Gly Val Ala Asn Ala Gly Asn
   3350 3355 3360
   Val Asn Thr Gly Ala Phe Val Thr Gly Asn Phe Ser Asn Gly Ile
   3365 3370 3375
   Leu Trp Arg Gly Asp Tyr Gln Gly Leu Ala Gly Phe Ala Val Gly
   3380 3385 3390
   Tyr Thr Leu Pro Leu Phe Pro Ala Val Gly Ala Asp Val Ser Gly
   3395 3400 3405
   Gly Ile Gly Pro Ile Thr Val Leu Pro Pro Ile His Ile Pro Pro
   3410 3415 3420
   Ile Pro Val Gly Phe Ala Ala Val Gly Gly Ile Gly Pro Ile Ala
   3425 3430 3435
   Ile Pro Asp Ile Ser Val Pro Ser Ile His Leu Gly Leu Asp Pro
   3440 3445 3450
   Ala Val His Val Gly Ser Ile Thr Val Asn Pro Ile Thr Val Arg
   3455 3460 3465
   Thr Pro Pro Val Leu Val Ser Tyr Ser Gln Gly Ala Val Thr Ser
   3470 3475 3480
   Thr Ser Gly Pro Thr Ser Glu Ile Trp Val Lys Pro Ser Phe Phe
   3485 3490 3495
   Pro Gly Ile Arg Ile Ala Pro Ser Ser Gly Gly Gly Ala Thr Ser
   3500 3505 3510
   Thr Gln Gly Ala Tyr Phe Val Gly Pro Ile Ser Ile Pro Ser Gly
   3515 3520 3525
   Thr Val Thr Phe Pro Gly Phe Thr Ile Pro Leu Asp Pro Ile Asp
   3530 3535 3540
   Ile Gly Leu Pro Val Ser Leu Thr Ile Pro Gly Phe Thr Ile Pro
   3545 3550 3555
   Gly Gly Thr Leu Ile Pro Thr Leu Pro Leu Gly Leu Ala Leu Ser
   3560 3565 3570
   Asn Gly Ile Pro Pro Val Asp Ile Pro Ala Ile Val Leu Asp Arg
   3575 3580 3585
   Ile Leu Leu Asp Leu His Ala Asp Thr Thr Ile Gly Pro Ile Asn
   3590 3595 3600
   Val Pro Ile Ala Gly Phe Gly Gly Ala Pro Gly Phe Gly Asn Ser
   3605 3610 3615
   Thr Thr Leu Pro Ser Ser Gly Phe Phe Asn Thr Gly Ala Gly Gly
   3620 3625 3630
   Gly Ser Gly Phe Ser Asn Thr Gly Ala Gly Met Ser Gly Leu Leu
   3635 3640 3645
   Asn Ala Met Ser Asp Pro Leu Leu Gly Ser Ala Ser Gly Phe Ala
   3650 3655 3660
   Asn Phe Gly Thr Gln Leu Ser Gly Ile Leu Asn Arg Gly Ala Gly
   3665 3670 3675
   Ile Ser Gly Val Tyr Asn Thr Gly Ala Leu Gly Val Val Thr Ala
   3680 3685 3690
   Ala Val Val Ser Gly Phe Gly Asn Val Gly Gln Gln Leu Ser Gly
   3695 3700 3705
   Leu Leu Phe Thr Gly Val Gly Pro
   3710 3715
<210> 13
   <211> 11
   <212> PRT
   <213> Mycobacterium tuberculosis
<400> 13
   Gln Thr Val Glu Asp Glu Ala Arg Arg Met Trp
   1 5 10
<210> 14
   <211> 9
   <212> PRT
   <213> Mycobacterium tuberculosis
<400> 14
   Val Ser Ala Ala Ile Ala Gly Leu Phe
   1 5
<210> 15
   <211> 297
   <212> DNA
   <213> Mycobacterium tuberculosis
<400> 15
   atggcctcgc gttttatgac ggatccgcac gcgatgcggg acatggcggg ccgttttgag 60
   gtgcacgccc agacggtgga ggacgaggct cgccggatgt gggcgtccgc gcaaaacatc 120
   tcgggcgcgg gctggagtgg catggccgag gcgacctcgc tagacaccat gacccagatg 180
   aatcaggcgt ttcgcaacat cgtgaacatg ctgcacgggg tgcgtgacgg gctggttcgc 240
   gacgccaaca actacgaaca gcaagagcag gcctcccagc agatcctcag cagctga 297
<210> 16
   <211> 297
   <212> DNA
   <213> Mycobacterium tuberculosis
<400> 16
   atggcctcac gttttatgac ggatccgcac gcgatgcggg acatggcggg ccgttttgag 60
   gtgcacgccc agacggtgga ggacgaggct cgccggatgt gggcgtccgc gcaaaacatt 120
   tccggtgcgg gctggagtgg catggccgag gcgacctcgc tagacaccat ggcccagatg 180
   aatcaggcgt ttcgcaacat cgtgaacatg ctgcacgggg tgcgtgacgg gctggttcgc 240
   gacgccaaca actacgagca gcaagagcag gcctcccagc agatcctcag cagctaa 297
<210> 17
   <211> 297
   <212> DNA
   <213> Mycobacterium tuberculosis
<400> 17
   atggcctcac gttttatgac ggatccgcat gcgatgcggg acatggcggg ccgttttgag      60
   gtgcacgccc agacggtgga ggacgaggct cgccggatgt gggcgtccgc gcaaaacatt     120
   tccggtgcgg gctggagtgg catggccgag gcgacctcgc tagacaccat gacctagatg     180
   aatcaggcgt ttcgcaacat cgtgaacatg ctgcacgggg tgcgtgacgg gctggttcgc     240
   gacgccaaca actacgaaca gcaagagcag gcctcccagc agatcctgag cagctag        297
<210> 18
   <211> 297
   <212> DNA
   <213> Mycobacterium tuberculosis
<400> 18
   atggcaacac gttttatgac ggatccgcac gcgatgcggg acatggcggg ccgttttgag      60
   gtgcacgccc agacggtgga ggacgaggct cgccggatgt gggcgtccgc gcaaaacatc     120
   tcgggcgcgg gctggagtgg catggccgag gcgacctcgc tagacaccat ggcccagatg     180
   aatcaggcgt ttcgcaacat cgtgaacatg ctgcacgggg tgcgtgacgg gctggttcgc     240
   gacgccaaca actacgagca gcaagagcag gcctcccagc agatcctcag cagctaa        297
<210> 19
   <211> 297
   <212> DNA
   <213> Mycobacterium tuberculosis
<400> 19
   atgacctcgc gttttatgac ggatccgcac gcgatgcggg acatggcggg ccgttttgag      60
   gtgcacgccc agacggtgga ggacgaggct cgccggatgt gggcgtccgc gcaaaacatt     120
   tccggcgcgg gctggagtgg catggccgag gcgacctcgc tagacaccat gacccagatg     180
   aatcaggcgt ttcgcaacat cgtgaacatg ctgcacgggg tgcgtgacgg gctggttcgc     240
   gacgccaaca actacgaaca gcaagagcag gcctcccagc agatcctcag cagctga        297
<210> 20
   <211> 435
   <212> DNA
   <213> Mycobacterium tuberculosis
<400> 20
   atgtcataca tgattgccac accagcggcg ttgacggcgg cggcaacgga tatcgacggg      60
   attggctcgg cggttagcgt tgcgaacgcc gcggcggtcg ccgcgacaac cggagtgctg     120
   gccgccggtg gcgatgaagt gttggcggcc atcgctaggc tgttcaacgc aaacgccgag     180
   gaatatcacg ccctcagcgc gcaggtggcg gcgtttcaaa ccctgtttgt gcgcaccttg     240
   actggggggt gcggagtctt tcgccggcgc cgaggccgcc aatgcgtcac agctgcagag      300
   catcgcgcgg caggtgcggg gcgccgtcaa cgccgtcgcc ggtcaggtga cgggcaatgg     360
   cggctccggc aacagcggca cttcggctgc ggcggccaac ccgaattccg acaacacagc     420
   gagcatcgcc gatag                                                      435
<210> 21
   <211> 2085
   <212> DNA
   <213> Mycobacterium tuberculosis
<400> 21
   gtgtcgttgg tgatcgcgac gccgcagctg ctggcaactg cggctttgga tttagcgagt 60
   attggttcgc aggtgagcgc ggctaatgcg gccgcggcga tgccgacgac ggaagtggtg 120
   gctgcggctg ccgatgaagt gtcggcggcg attgcggggt tgttcggggc ccatgctcgg 180
   cagtatcagg cgctcagcgt acaggtggca gcgtttcacg agcagtttgt gcaggcgttg 240
   actgcggccg cgggtcggta tgccagcact gaggccgctg ttgagcggag tctgctgggt 300
   gcggtgaatg cgcccaccga ggcgcttttg gggcgcccgt tgatcggaaa cggcgccgac 360
   gggacggcac ccgggcagcc tggcgcggcc ggcgggttgc tgtttggcaa cggtggcaac 420
   ggcgcggctg gcgggttcgg tcaaaccggc ggcagcggag gcgcggccgg gttgatcggc 480
   aacggcggca acggcggggc cggtggtacc ggcgcggccg gcggtgccgg tgggaacggg 540
   gggtggttgt ggggcaacgg cggcaacggc ggtgtcggcg gcaccagcgt ggccgcaggc 600
   atcgggggtg cgggcggtaa cggcggcaac gccgggctgt tcggccatgg cggcgccggt 660
   ggtaccggcg gcgccggcct cgccggggca aacggggtca atcccacgcc cggccccgcg 720
   gccagcaccg gggacagccc ggcagatgtg tccggcatcg gtgatcaaac cggcggcgac 780
   ggcggcacgg gcggccatgg cactgccggc acgccgaccg gtggcaccgg cggcgacggt 840
   gccaccgcga cggcaggctc gggcaaggcc accggcggtg ccggtggtga cggcggtacc 900
   gccgctgccg gtggcggcgg cggcaacggc ggcgacggcg gagtcgcgca gggcgacatt 960
   gcgagcgcct ttggcggtga tggtggcaac gggtccgacg gtgtagccgc cggcagtggg 1020
   ggtggtagcg gcggcgccgg aggcggcgct ttcgtacaca tcgccactgc cacctctacc 1080
   ggtggtagcg gcggtttcgg tggtaacggg gctgccagtg ccgcctccgg cgccgacggt 1140
   ggcgcagggg gagctggcgg caatggtggc gccggcgggt tgctattcgg tgatggcggc 1200
   aacggtggcg ccggtggcgc gggtggtatc ggtggtgacg gcgccacggg ggggcccggg 1260
   ggaagcggcg gcaacgctgg catcgcgagg tttgacagcc cagaccccga ggcagaaccc 1320
   gatgtggtcg gcggcaaggg tggtgatggc ggcaagggcg gcagcggcct tggcgtcggc 1380
   ggcgccggcg ggaccggcgg cgcgggcggc aacggcggcg ccggcgggtt gttgttcggc 1440
   aacggcggca acggcggcaa cgccggggcc ggcggggatg gcggcgccgg cgttgccggt 1500
   ggggttggcg gtaacggcgg cggtggtggc accgcgacgt ttcacgaaga cccggtcgct 1560
   ggtgtctggg cggtcggtgg cgtaggtggt gatggtggct ccggcggcag ctcgcttggt 1620
   gtcggcgggg tgggcggagc cggtggcgtg ggtggcaagg gtggcgccag cggcatgttg 1680
   atcggcaacg gcggcaacgg tggcagcggc ggagtcggtg gggccggtgg agtcggcggg 1740
   gctggcggtg acggcggcaa cggcggctcc ggtggcaacg ccagtacttt tggcgatgag 1800
   aactccatcg gcggggccgg cgggacgggc ggcaacgggg gcaacggcgc aaacggcggt 1860
   aacggtggcg ctggcggtat tgccggcggt gcgggtgggt ccggagggtt cctcagcggt 1920
   gccgcaggag tcagcggcgc tgacggtatc ggtggcgcgg gcggcgcagg cggtgccggt 1980
   ggcgcgggcg gtagcggcgg tgaggcaggc gcggggggcc tcaccaacgg ccccgggtcc 2040
   cctggcgttt ccggcaccga aggcatggcc ggcgcgcccg gctag 2085
<210> 22
   <211> 978
   <212> DNA
   <213> Mycobacterium tuberculosis
<400> 22
   atgcatcagg tggaccccaa cttgacacgt cgcaagggac gattggcggc actggctatc 60
   gcggcgatgg ccagcgccag cctggtgacc gttgcggtgc ccgcgaccgc caacgccgat 120
   ccggagccag cgcccccggt acccacaacg gccgcctcgc cgccgtcgac cgctgcagcg 180
   ccacccgcac cggcgacacc tgttgccccc ccaccaccgg ccgccgccaa cacgccgaat 240
   gcccagccgg gcgatcccaa cgcagcacct ccgccggccg acccgaacgc accgccgcca 300
   cctgtcattg ccccaaacgc accccaacct gtccggatcg acaacccggt tggaggattc 360
   agcttcgcgc tgcctgctgg ctgggtggag tctgacgccg cccacttcga ctacggttca 420
   gcactcctca gcaaaaccac cggggacccg ccatttcccg gacagccgcc gccggtggcc 480
   aatgacaccc gtatcgtgct cggccggcta gaccaaaagc tttacgccag cgccgaagcc 540
   accgactcca aggccgcggc ccggttgggc tcggacatgg gtgagttcta tatgccctac 600
   ccgggcaccc ggatcaacca ggaaaccgtc tcgctcgacg ccaacggggt gtctggaagc 660
   gcgtcgtatt acgaagtcaa gttcagcgat ccgagtaagc cgaacggcca gatctggacg 720
   ggcgtaatcg gctcgcccgc ggcgaacgca ccggacgccg ggccccctca gcgctggttt 780
   gtggtatggc tcgggaccgc caacaacccg gtggacaagg gcgcggccaa ggcgctggcc 840
   gaatcgatcc ggcctttggt cgccccgccg ccggcgccgg caccggctcc tgcagagccc 900
   gctccggcgc cggcgccggc cggggaagtc gctcctaccc cgacgacacc gacaccgcag 960
   cggaccttac cggcctga 978
<210> 23
   <211> 1749
   <212> DNA
   <213> Mycobacterium tuberculosis
<400> 23
   atgctcctgg ccctgctgcg ccagcacatc cgaccgtacc gccggctggt cgcgatgctg 60
   atgatgctgc agctggtcag caccctggct tcgctatacc tcccgacggt caacgccgca 120
   atcgtcgacg acggcgtcgc caagggcgac accgccacca tcgtacggct gggtgcggtg 180
   atgcttgggg tgaccggatt gcaggtgctg tgcgcgatcg gggcggtcta tctgggctcc 240
   cggaccgggg cgggtttcgg ccgtgacctg cgctcggcaa tgttcgaaca catcatcacc 300
   ttctcggaac gcgagaccgc ccgattcggc gctccgacgt tgttgacccg cagcaccaac 360
   gacgtccggc agatcctgtt cctggtccag atgaccgcca ccgtgctggt caccgcaccg 420
   atcatgtgcg tcggcggaat catcatggcc atccaccagg aggccgcgct gacatggctg 480
   ctgctggtca gcgttccgat tctggccgta gcaaactact ggatcatctc ccacatgctg 540
   ccgctcttcc gccgcatgca gagcctgatc gacggcatca accgggtgat gcgcgatcag 600
   ctgtccgggg tgcgagtggt ccgcgccttc acccgcgaag gctatgaacg cgacaagttc 660
   gcgcaggcca atacggcgct gtcgaatgcc gcactgagcg ccggcaactg gcaagcactg 720
   atgctgccgg tgaccacgct gaccatcaac gcatccagcg tcgcactgat ctggttcggt 780
   gggctacgca tcgacagcgg ccagatgcag gtcggctccc tgatcgcctt cctgtcctac 840
   ttcgcccaga tcctgatggc ggtgttgatg gcgaccatga cgctggccgt gctgccacga 900
   gcgtcggtct gcgccgaacg catcaccgag gtgctttcca cgcccgccgc actcggtaac 960
   cccgacaatc ccaagttccc gacggacggg gtcacgggcg tagtgcgctt ggctggcgca 1020
   acctttacct atcctggcgc cgactgcccg gtgctgcagg acatttcgtt gactgcgcgg 1080
   cccggtacca ccaccgcgat cgtcggcagt accggttcgg gcaagtcgac actggtgtcg 1140
   ttgatctgcc ggctctacga cgtcaccgct ggcgcggtct tggttgacgg tatcgacgtc 1200
   cgcgagtacc acaccgagcg gctctggtca gcgatcgggc tggtgcccca gcgcagctac 1260
   ctcttctccg gaaccgtcgc ggacaacctg cgctacggcg ggggcccaga ccaggtagtc 1320
   accgagcagg agatgtggga ggcgctgcgg gtcgccgcgg ccgacggctt tgtacaaaca 1380
   gacgggctgc agacgcgtgt cgcccaaggt ggtgtcaact tctccggcgg gcagcgccaa 1440
   cggctggcga tagcccgagc ggtcatccga cgtccggcca tctatgtgtt cgacgacgcg 1500
   ttctccgcac ttgacgtgca caccgacgcc aaagtccacg catcgctgcg acaggtatct 1560
   ggtgatgcaa ccatcattgt tgttacacaa cggatttcga atgccgctca ggccgaccag 1620
   gtcatcgttg tcgataacgg taagatcgtc ggcacgggca cccacgaaac gctgctggcc 1680
   gattgcccca cctatgccga attcgccgcc tcacaatcgc tgagcgccac ggtcgggggt 1740
   gtagggtga 1749
<210> 24
   <211> 1407
   <212> DNA
   <213> Mycobacterium tuberculosis
<400> 24
   atgtcctacg tcatcgcggc cccggagatg ttggcaacga cggccgcgga cgtggacggg     60
   atcggttcgg cgatacgagc ggccagcgcg tccgctgcgg gtccaacgac cggactgctg    120
   gccgcggccg ccgatgaggt gtcgtcggcc gctgcagcgc tgttcagcga atacgcgcgc    180
   gaatgtcaag aggtcctaaa gcaggctgcg gcgttccatg gcgagttcac ccgggcgctg    240
   gctgccgccg gggccgccta tgcccaggct gaagccagca acaccgctgc tatgtcgggc    300
   accgccgggt ccagcggcgc cctcggttct gtcgggatgc tgtcaggcaa cccgctaacc    360
   gcgttgatga tgggcggcac cggggaaccg atccttagtg accgcgtctt ggcgatcatt    420
   gacagcgcat acattcggcc cattttcggg cccaacaacc cggtcgccca gtacacgccc    480
   gagcagtggt ggccgtttat cgggaacctg tcactggacc aatccatcgc ccagggtgtc    540
   acgctgctga acaacggcat caacgcggaa ctacaaaatg ggcatgacgt cgtcgttttc    600
   ggctactcgc aaagcgccgc ggtagcgacc aatgaaatac gcgctcttat ggcgttacca    660
   ccgggccaag ccccagatcc aagccggctg gctttcacgt tgatcggtaa tatcaataac    720
   cccaacggcg gcgtcctcga gcgttacgtg ggcctttacc tcccgttctt ggatatgtcg    780
   ttcaacggtg cgactccacc ggattccccc taccagacct acatgtacac cggccaatac    840
   gacggctacg cccacaaccc gcagtacccg ctcaatatct tgtcggacct caacgccttc    900
   atgggcatca gatgggtgca caacgcgtac cccttcaccg cggccgaggt tgccaatgcc    960
   gtgccgttgc ccacgtctcc gggctacacc ggcaacaccc attactacat gtttctgacc   1020
   caggacctgc cgctgttgca gccgattcgc gccatcccct tcgtagggac cccaatagcc   1080
   gagctgattc agcccgacct acgggtgcta gtcgacttgg gctatggcta cggctacgcc   1140
   gacgtaccca ccccggccag cctgttcgcg ccaatcaacc cgatcgccgt ggcctcggcc   1200
   ctggcgaccg ggaccgtgca aggcccccaa gccgccctag taagcatcgg attgttaccg   1260
   cagtccgcgc tacccaatac gtatccgtat cttccgtcgg cgaatccggg cctgatgttc   1320
   aacttcggtc aatccagtgt gacggagttg tcggtgctca gtggcgccct cgggtccgta   1380
   gcgagattga ttccaccgat cgcgtga                                         1407
<210> 25
   <211> 11151
   <212> DNA
   <213> Mycobacterium tuberculosis
<400> 25
   atggagtttc cggtgttgcc accggaaatc aactccgtgc tgatgtattc gggtgcgggg    60
   tcgagcccgt tgctggcggc ggccgcggcg tgggatgggc tggctgagga gttggggtcg   120
   gcggcggtgt cgtttgggca ggtgacgtcg ggcctgacgg cgggggtgtg gcagggtgcg   180
   gcggcggcgg cgatggcggc cgcggcggcg ccgtatgcgg ggtggttggg ttcggtggcg   240
   gccgcggccg aggcggtggc cgggcaggcg cgggtggtgg tgggggtctt tgaggcggcg   300
   ttggcggcga cggtggatcc ggcgctggtg gcggccaacc gggcgcggct ggtggcgttg 360
   gcggtgtcga atctgttggg gcagaacacg ccggcgatcg cggccgccga ggccgagtac 420
   gagctgatgt gggccgccga tgtggcggcg atggccggct accattccgg cgcgtcggct 480
   gctgccgcgg cgttgccggc gttcagccca ccggcgcagg cgctgggggg aggtgtcggc 540
   gcgttcctta ccgccctgtt cgccagccct gcgaaggcgc tgagcctgaa tgcgggtttg 600
   ggcaatgtcg gcaattacaa cgtcgggttg ggcaatgtcg gggtgttcaa cctgggcgcg 660
   ggcaatgtgg gtgggcagaa tctgggtttc gggaatgccg gtggcaccaa tgtcgggttc 720
   ggcaacctcg gtaacgggaa tgtcgggttc ggcaactccg gtctgggggc gggcctggcc 780
   ggcttgggca atatcgggtt gggcaatgcg ggcagcagca actatggttt cgcaaacctg 840
   ggtgtgggca acatcggttt cggcaacacc ggcaccaaca acgtcggcgt cgggctcacc 900
   ggcaaccacc tgacgggtat cgggggcctg aattcgggca ccgggaatat cgggttgttc 960
   aactccggca ccgggaatgt ggggttcttc aattcgggga ccgggaactt cggggtgttc 1020
   aactcgggta attacaacac cggtgtcggt aatgcgggga cggccagcac ggggttgttc 1080
   aatgccggca atttcaacac cggcgtggtg aacgtgggca gttacaacac cggcagtttc 1140
   aacgccggcg acaccaacac cggtggcttc aaccccggcg gtgtgaacac cggctggctg 1200
   aacaccggca acaccaacac cggcatcgcc aactcgggca acgtcaacac cggcgcgttc 1260
   atctcgggca acttcaacaa cggcgtgctg tgggtgggtg actaccaggg cctgttcggc 1320
   gtctccgccg gctcgtcgat ccccgcaatt cccatcggcc tggtgctcaa cggcgacatc 1380
   ggcccgatca ccatccagcc catcccgatc ctgcccacca tcccgctcag cattcaccaa 1440
   accgtcaact tgggcccgct ggtggttccc gacatcgtga tccccgcctt cggcggcggt 1500
   atcggcatac ccatcaacat cggcccgctg accatcacac ccatcaccct gtttgcccaa 1560
   cagacatttg tcaaccaatt gccctttccc accttcagtt tagggaaaat cacaattcca 1620
   caaatccaaa cctttgattc taacggtcag cttgtcagct ttatcggccc tatcgttatc 1680
   gacaccacca ttcccggacc caccaatcca cagattgatt taacgatcag atgggatacc 1740
   cctccgatca cgctgttccc gaatggcatc agtgctcccg ataatccttt ggggttgctg 1800
   gtgagtgtgt cgatcagtaa cccgggcttt accatcccgg gatttagtgt tcccgcgcag 1860
   ccgttgccgt tgtcgatcga tatcgagggc cagatcgacg ggttcagcac cccgccgatc 1920
   acgatcgatc gcatccccct gaccgtgggg ggcggggtca cgatcggccc catcacgatc 1980
   cagggccttc atatcccggc ggcgccggga gtggggaaca ccaccacggc cccgtcgtcg 2040
   ggattcttca actccggtgc gggtggggtg tcgggtttcg gcaacgtcgg cgcgggcagc 2100
   tcgggctggt ggaaccaggc gccgagcgcg ctgttggggg ccggttcggg tgttggcaac 2160
   gtgggcaccc tgggctcggg tgtgctcaac ctgggctcag ggatctcggg gttctacaac 2220
   accagcgtgt tgcctttcgg gacaccggcg gcggtgtcgg gcatcggcaa cctgggccag 2280
   cagctgtcgg gggtgtcggc ggcgggaacc acgctgcgct cgatgctcgc cggcaacctc 2340
   gggttggcca atgtgggcaa cttcaacacc gggttcggaa atgtcgggga cgtcaacctg 2400
   ggtgcggcca acatcggtgg gcacaacctg ggcctgggca atgtcgggga cggcaacctg 2460
   gggttgggca acatcggcca tggcaacctg gggtttgcca acttgggcct gaccgccggc 2520
   gcggcggggg tgggcaatgt tggttttggc aatgccggca tcaacaacta tggcttggcg 2580
   aacatgggtg tgggcaatat tgggtttgcc aacaccggca cgggcaacat cgggatcggg 2640
   ctggtcgggg accatcggac cgggatcggg ggcttgaact ccggcatcgg caatatcggg 2700
   ttgttcaact ccggcaccgg caacgtcggg ttcttcaatt ccgggaccgg caacttcggc 2760
   atcgggaact ccggccgctt caacaccggg atcggtaata gcggaacggc cagcaccggg 2820
   ctcttcaatg ccggcagctt cagcaccggc atcgccaaca ctggtgacta caacacgggc 2880
   agcttcaacg ccggcgacac caacaccggt ggcttcaacc cgggcggcat caacaccggc 2940
   tggttcaaca ccgggcatgc caacaccggg ttggccaacg cgggcacctt cggcaccggc 3000
   gccttcatga cgggcgacta cagcaacggc ctgttgtggc ggggcggcta cgagggcctg 3060
   gtcggcgtcc gcgtcgggcc cacgatctcc caattcccgg tcaccgtgca cgcgatcggc 3120
   ggggtgggcc cgctgcatgt ggcgcccgtc ccggtacccg ccgtgcacgt cgagatcacc 3180
   gacgccaccg tcggcctggg tccgttcacc gtcccaccga tcagcattcc ctcacttccc 3240
   atcgccagca tcaccggaag cgtggacctg gccgcaaaca ccatctcgcc gattcgcgct 3300
   cttgacccgc tcgccggttc gatagggctt tttctcgagc cgttccgcct cagtgaccca 3360
   tttatcacca ttgatgcgtt ccaagttgtt gccggtgtct tgttcctaga gaacatcatt 3420
   gtgcccggcc tcacggttag cggtcagata ttggtcaccc cgacaccaat tcccctaacc 3480
   ctcaacttgg acaccacccc gtggacgctt ttcccgaatg gtttcaccat tcccgcgcaa 3540
   acccccgtga cggtgggtat ggaggtcgcc aacgacgggt tcaccttctt cccgggtggg 3600
   ctgacctttc cgcgggcctc cgccggggtc accggactgt ccgtggggct ggacgcgttc 3660
   acgctgttgc ccgacgggtt caccctcgac accgtgccgg cgaccttcga cggcaccatc 3720
   ctcatcggcg atatcccgat cccgatcatc gatgtgccgg cggtgccggg gttcggcaac 3780
   accaccacgg ccccatcgtc ggggttcttc aacaccggcg gcggcggtgg atcggggttc 3840
   gccaacgtcg gcgcgggcac gtcgggctgg tggaaccagg ggcacgacgt gttagcaggg 3900
   gcgggctcgg gagttgccaa tgccggcacg ctgagctcgg gcgtgctgaa cgtcggctcg 3960
   gggatctccg ggtggtacaa caccagcacc ctgggagcgg gcaccccggc ggtggtctcg 4020
   ggcatcggca acctcggcca gcagctgtcg gggttcttgg caaatgggac cgtgctcaac 4080
   cggagcccca ttgtcaatat cgggtgggcc gatgtgggcg cgttcaacac cgggttgggc 4140
   aatgtggggg acctcaactg gggtgcggcc aacatcggcg cgcagaacct gggcctgggc 4200
   aatctcggca gcgggaacgt cgggttcggc aacatcggtg ccggcaacgt cgggttcgcc 4260
   aactcgggtc cggcggtggg cctggccggc ctgggcaacg tggggttgag caatgccggc 4320
   agcaacaact gggggctggc caacctgggt gtgggcaaca tcgggttggc caacaccggc 4380
   acgggcaaca tcgggatcgg gctggtcggc gactaccaga ccggcatcgg cggcctcaac 4440
   tcgggtagtg gcaatatcgg attgttcaat tccggcaccg gcaatgtcgg gttcttcaac 4500
   accggcaccg gcaacttcgg actgttcaac tccggtagtt tcaacaccgg catcggtaat 4560
   agcggaaccg gcagtactgg gctcttcaat gccggcaatt tcaacaccgg catcgccaac 4620
   cccgggtcgt acaacacggg cagcttcaat gtcggtgata ccaacaccgg tggtttcaac 4680
   ccgggcgaca tcaacaccgg ctggttcaac accggcatta tgaatacggg cacccgcaac 4740
   accggcgccc tcatgtcggg gaccgacagc aacggcatgc tgtggcgcgg cgaccacgag 4800
   ggcctgttcg gcctgtccta tggcatcacg atcccgcaat tcccgatccg catcaccacg 4860
   actggcggta tcggccccat cgtcatcccg gacaccacga tccttccgcc gctgcacctg 4920
   cagatcaccg gcgacgcgga ctacagcttc accgtgcccg acatccccat ccccgccatc 4980
   cacatcggca tcaatggcgt cgtcaccgtc ggcttcaccg ccccggaagc caccctgctg 5040
   tccgccctga agaataacgg tagcttcatc agcttcggcc ccatcacgct ctcgaatatc 5100
   gatattccgc ccatggattt cacgttaggc ctgcccgttc ttggtcctat cacgggccaa 5160
   ctcggaccaa ttcatcttga gccaatcgtg gtggccggga tcggtgtgcc cctggagatc 5220
   gagcccatcc ccctggatgc gatttcgttg agtgagtcga ttcctatccg catacctgtt 5280
   gatattccgg cctcggtcat cgatgggatt tcaatgtcgg aagtggtgcc gatcgatgcg 5340
   tccgtggaca tcccggcggt cacgatcaca ggcaccacca tttccgcgat cccgctgggc 5400
   ttcgacattc gcaccagtgc cggacccctc aacatcccga tcatcgacat cccggcggcg 5460
   ccgggcttcg ggaactcgac ccagatgccg tcgtcggggt tcttcaacac cggtgccggc 5520
   ggcggatcgg gcatcggcaa cttgggtgcg ggcgtgtcgg gcctgctcaa ccaggccggc 5580
   gcggggtcac tggtggggac actctcgggg ctgggcaatg ccggcaccct ggcctcgggt 5640
   gtgctgaact ccggcaccgc catctccggg ctgttcaacg tgagcacgct ggacgccacc 5700
   accccggcgg tgatctcggg gttcagcaac ctcggcgacc atatgtcggg ggtgtccatc 5760
   gatggcctga tcgcgatcct caccttccca cctgccgagt ccgtgttcga tcagatcatc 5820
   gacgcggcca tcgccgagct gcagcacctc gacatcggca acgctttggc cttgggcaat 5880
   gtcggcgggg tgaacctcgg tttggctaac gtcggtgagt tcaacctggg tgcgggcaac 5940
   gtcggcaaca tcaacgtcgg cgccggcaac ctcggcggca gcaacttggg gttgggcaac 6000
   gtcgggaccg gcaacctcgg gttcggcaac atcggtgccg gcaatttcgg attcggcaac 6060
   gcgggcctga ccgcgggcgc ggggggcctg ggcaatgtgg ggttgggtaa cgccggcagc 6120
   ggcagctggg ggttggccaa cgtgggtgtg ggcaatatcg ggttggccaa caccggcacc 6180
   ggcaacatcg ggatcgggct gaccggggac tatcggaccg ggatcggcgg cctgaactcg 6240
   ggcaccggga acctcgggtt gttcaactcg ggcaccggca acatcgggtt cttcaacacc 6300
   gggaccggga acttcgggct gttcaactcg ggcagttaca gcaccggtgt ggggaatgcg 6360
   ggcacggcca gcaccgggtt gttcaacgcg gggaacttca acaccggtct ggccaatgcc 6420
   ggctcctaca acaccggcag cctcaacgtg ggcagcttca acaccggcgg cgtcaacccg 6480
   ggcaccgtca acaccggctg gttcaacacc ggccacacca acaccggcct gttcaacacc 6540
   ggcaacgtca acaccggcgc gttcaactcc ggcagcttca acaacggggc gctgtggacc 6600
   ggtgactacc acgggctggt cggcttctcc ttcagcatcg acatcgccgg cagcaccctg 6660
   ctggacctca acgaaaccct caacctgggc cccatccaca tcgagcagat cgacatcccc 6720
   ggcatgtcgc tgttcgacgt ccacgaaatc gtcgagatcg gacccttcac catcccgcag 6780
   gtcgatgttc ccgcgatacc gctagagatc cacgaatcga tccacatgga tcccatcgtc 6840
   ctggtgcccg ccaccacaat tcccgcacag acgagaacca ttccgctgga catccccgcc 6900
   tcacccgggt caaccatgac gcttccgctc atcagcatgc gcttcgaagg cgaggactgg 6960
   atcctcgggt cgaccgcggc gattcccaat ttcggagacc ccttcccggc gcccacccag 7020
   ggcatcacca ttcacaccgg ccctggcccc ggaacgaccg gcgagctcaa gatatctatt 7080
   ccgggtttcg agattccgca aatcgctacc acgagattcc tgttggacgt gaacatcagc 7140
   ggtggtctgc cggccttcac cttgttcgcg ggtggcctga cgatccccac gaacgccatc 7200
   ccgttaacga tcgatgcgtc cggcgcgctg gatccgatca cgattttccc gggtgggtac 7260
   acgatcgacc cgctgccgct gcacctggcg ctgaatctca ccgtgcccga cagcagcatc 7320
   ccgatcatcg atgtcccgcc gacgccaggg ttcggcaaca ccacggcgac cccgtcgtcg 7380
   gggttcttca actccggcgc cggtggggtg tcggggttcg gaaacgtcgg gtcgaacctg 7440
   tcgggctggt ggaaccaggc ggcgagcgcg ctggcggggt cgggatcggg ggtgttgaat 7500
   gtcggcacgc tgggctcggg tgtgctcaac gtcggctcgg gtgtctcggg gatctacaac 7560
   accagcgtgt tgccgctcgg gacgccggcg gtgctgtcgg gcctcggcaa cgtcggccat 7620
   cagctgtcgg gcgtgtctgc ggccgggacc gcgttgaacc agatccccat cctcaacatc 7680
   gggttggcgg atgtgggcaa cttcaacgtc gggttcggca acgtcgggga cgttaacctg 7740
   ggcgcggcca acctcggtgc gcaaaacctg gggctgggca acgtcggcac cggcaacctc 7800
   ggcttcgcca acgtcggcca cggcaatatc ggtttcggca attcgggtct gaccgccggc 7860
   gcggccggcc tgggcaacac ggggttcggc aatgccggca gcgccaacta tggtttcgcc 7920
   aaccagggcg tgcgcaacat cgggttggcc aacaccggca ccggcaacat cgggatcggg 7980
   ctggtggggg acaacctcac cggcatcggg ggcctgaact ccggtgccgg caatatcggc 8040
   ttgttcaact ccggcaccgg caacatcggg ttcttcaact ccgggaccgg caacttcggc 8100
   atcggtaact cgggcagctt caacaccggc atcggcaata gcggaacggg cagcactggg 8160
   ctcttcaatg ccggcagctt caacaccggc gtggccaacg ccggcagcta caacaccggc 8220
   agcttcaatg ccggcgacac caacaccggg gggttcaacc cgggcaccat caacaccggc 8280
   tggttcaaca ccggccacac caataccggc atcgccaact cgggcaacgt cggcaccggc 8340
   gcgttcatgt cgggcaactt cagcaacggc ctgttgtggc ggggtgatca cgagggcctg 8400
   ttcagcctgt tctacagcct cgacgtgccc cggatcacca tcgtggacgc ccacctcgac 8460
   ggcggcttcg gacccgtggt cctcccgccc atcccggtgc cggccgttaa tgcgcacctg 8520
   accggaaacg tcgcgatggg cgcattcacc attccgcaga tcgacatccc cgcactcacc 8580
   ccaaacatca ccggaagcgc cgccttccgc atcgttgtgg ggtccgtgcg cattccgccg 8640
   gtgagtgtca ttgtggagca aataatcaac gcctcggttg gggcggagat gaggatagat 8700
   cccttcgaaa tgtggactca aggcactaat ggccttggta taaccttcta ttcattcgga 8760
   tcggccgacg gttcgcccta cgccaccggc ccactcgttt tcggcgccgg cacgagcgac 8820
   ggaagccatc tcaccatttc cgcgtccagc ggggcgttta ccactccgca gctcgaaact 8880
   ggcccgatca cgttgggctt ccaggtgccc ggcagcgtca acgcgatcac cctcttcccc 8940
   ggtggtttga cgttcccggc gacctcgctg ctgaacctgg acgtgaccgc cggcgccggc 9000
   ggcgtggaca tcccggccat cacctggccc gagatcgcgg cgagcgccga cggctcggtg 9060
   tatgtcctcg ccagcagcat cccgctgatc aacatcccgc ccaccccggg cattgggaac 9120
   agcaccatca ccccgtcgtc gggcttcttc aacgccggcg cgggcggggg atcgggcttc 9180
   ggcaacttcg gcgcgggcac ctcgggctgg tggaaccagg cgcacaccgc gctggcgggg 9240
   gcgggctcgg gttttgccaa cgttggcacg ctgcattccg gtgtgctcaa cctgggctcg 9300
   ggtgtctcgg ggatctacaa caccagcacg ctgggggtgg ggaccccggc gctggtctca 9360
   ggcctgggca acgtcggcca ccaactgtcg gggctgcttt ccggcgggtc cgcggtgaac 9420
   ccggtgaccg ttctgaatat cgggttggcc aacgtcggca gccacaacgc cggtttcggc 9480
   aatgtcgggg aggtcaacct gggcgcggcc aacctcggcg cgcacaacct gggcttcgga 9540
   aatatcggcg ccggcaacct ggggttcggc aatattggcc acggcaatgt cggagtcggc 9600
   aactcgggtc tgaccgcggg cgtgccgggc ctgggcaatg tggggttggg caatgccggc 9660
   ggcaacaact gggggttggc caacgtgggc gtgggcaata tcgggttggc caacaccggc 9720
   accggcaaca ttgggatcgg gctgaccggc gactaccaga ccggcatcgg cggcctaaat 9780
   tccggtgccg gcaacctggg gttgttcaac tccggcgccg gcaacgtcgg gttcttcaac 9840
   accgggaccg gcaacttcgg gttgttcaac tccggcagct tcaacaccgg cgtcggcaat 9900
   agcggaacgg gcagcactgg gctcttcaat gccggcagtt tcaacaccgg tgtggccaac 9960
   gccggcagct acaacacggg cagcttcaat gtcggtgaca ccaacaccgg gggcttcaac 10020
   ccgggcagca tcaacaccgg ctggctcaac gccggcaacg ccaacaccgg ggtggccaac  10080
   gcgggcaatg tcaacaccgg cgccttcgtc accggcaact tcagcaacgg catcctgtgg  10140
   cgcggcgact accagggcct ggccggcttc gccgtgggct acaccctccc gctgttcccc  10200
   gcggtgggcg ccgacgtcag cggcgggatc ggcccgatta ccgtgctgcc gcccatccac  10260
   atcccgccca ttccggtcgg cttcgccgcg gtcggtggca tcggcccgat cgccatcccg  10320
   gacatctctg ttccatccat tcacttgggc ctcgaccccg ccgtccatgt cggctccatc  10380
   accgtcaacc ccattaccgt caggaccccg cccgtgctcg tcagttactc ccaaggagcc  10440
   gtcaccagca cgtccggacc aacctcagag atttgggtca agcccagctt cttccccgga  10500
   atccggatcg cgccctctag cggcgggggt gcaacgtcca cgcaaggggc atactttgtg  10560
   gggcccatct ccatcccctc cggcacggtg accttcccgg gattcaccat ccccctcgac  10620
   ccgatcgaca tcggcctgcc ggtgtcgctg accatcccgg ggttcaccat cccgggcggc  10680
   accctgatcc ccaccctccc gctgggcctc gcgttgtcca atggcatccc gcccgtcgac  10740
   atcccggcca tcgttctcga ccggatcttg ctggacctgc acgccgacac cactatcggc  10800
   ccgatcaacg tcccgatcgc cgggttcggc ggggcgccgg gtttcgggaa ctcgaccacg  10860
   ctgccgtcgt cgggcttctt caacaccgga gctggcggcg gttcgggctt tagcaacacc  10920
   ggcgcgggca tgtcgggatt gctcaacgcg atgtcggatc cgctgctcgg gtcggcgtcg  10980
   ggcttcgcca acttcggcac ccagctctcc ggcatcctca accgcggcgc cggcatctcg  11040
   ggcgtgtaca acaccggcgc gctgggtgtt gtcaccgcgg ccgtcgtctc gggtttcggc  11100
   aacgtcggcc agcaactgtc gggcttgctc ttcaccggcg tcgggcccta a           11151
<210> 26
   <211> 10
   <212> PRT
   <213> Mycobacterium tuberculosis
<400> 26
   Ala Glu Met Lys Thr Asp Ala Ala Thr Leu
   1               5                   10
<210> 27
   <211> 10
   <212> PRT
   <213> Mycobacterium tuberculosis
<400> 27
   Arg Ala Asp Glu Glu Gln Gln Gln Ala Leu
   1               5                   10
<210> 28
   <211> 10
   <212> PRT
   <213> Mycobacterium tuberculosis
<400> 28
   Thr Ala Ala Gln Ala Ala Val Val Arg Phe
   1               5                   10
<210> 29
   <211> 8
   <212> PRT
   <213> Mycobacterium tuberculosis
<400> 29
   Ala Glu Met Lys Thr Asp Ala Ala
   1               5
<210> 30
   <211> 11
   <212> PRT
   <213> Mycobacterium tuberculosis
<400> 30
   Ala Glu Met Lys Thr Asp Ala Ala Thr Leu Ala
   1               5                   10
<210> 31
   <211> 9
   <212> PRT
   <213> Mycobacterium tuberculosis
<400> 31
   Asn Ile Arg Gln Ala Gly Val Gln Tyr
   1               5
<210> 32
   <211> 9
   <212> PRT
   <213> Mycobacterium tuberculosis
<400> 32
   Glu Met Lys Thr Asp Ala Ala Thr Leu
   1               5
<210> 33
   <211> 10
   <212> PRT
   <213> Mycobacterium tuberculosis
<400> 33
   Ala Glu Met Lys Thr Asp Ala Ala Thr Leu
   1               5                   10
<210> 34
   <211> 9
   <212> PRT
   <213> Mycobacterium tuberculosis
<400> 34
   Leu Leu Asp Ala His Ile Pro Gln Leu
   1 5
<210> 35
   <211> 9
   <212> PRT
   <213> Mycobacterium tuberculosis
<400> 35
   Ala Ala His Ala Arg Phe Val Ala Ala
   1 5
<210> 36
   <211> 11
   <212> PRT
   <213> Mycobacterium tuberculosis
<400> 36
   Ala Val Ile Asn Thr Thr Cys Asn Tyr Gly Gln
   1 5 10
<210> 37
   <211> 9
   <212> PRT
   <213> Mycobacterium tuberculosis
<400> 37
   Ala Ser Pro Val Ala Gln Ser Tyr Leu
   1 5
<210> 38
   <211> 10
   <212> PRT
   <213> Mycobacterium tuberculosis
<400> 38
   Glu Leu Pro Gln Trp Leu Ser Ala Asn Arg
   1 5 10